# EUROPÄISCHE PATENTSCHRIFT

(11) **EP 0 646 596 B1**
(45) Veröffentlichungstag und Bekanntmachung des Hinweises auf die Patenterteilung: **26.05.1999**
(21) Anmeldenummer: 94113573.3
(22) Anmeldetag: 31.08.1994
(51) Int. Cl.: C07K 5/00, A61K 38/10

(54) **Nukleinsäure-bindende Oligomere mit C-Verzweigung für Therapie und Diagnostik**
Nucleic acid binding oligomers having C-branches for therapy and diagnosis
Oligomères liant les acides nucléiques avec branchement en C et leur usage en thérapie et diagnostic

(30) Priorität: 13.09.1993 DE 4331011
(43) Veröffentlichungstag der Anmeldung: 05.04.1995
(73) Patentinhaber: BAYER AG, 51368 Leverkusen (DE)
(72) Erfinder: Löbberding, Dr. Antonius, D-42113 Wuppertal (DE); Mielke, Dr. Burkhard, D-51375 Leverkusen (DE); Schwemler, Dr. Christoph, D-42799 Leichlingen (DE); Schwenner, Dr. Eckhard, D-42113 Wuppertal (DE); Stropp, Dr. Udo, D-42781 Haan (DE); Springer, Dr. Wolfgang, D-42113 Wuppertal (DE); Kretschmer, Dr. Axel, D-51429 Bergisch Gladbach (DE); Pötter, Dr. Thorsten, D-51061 Köln (DE)

(56) Entgegenhaltungen:
- EP-A- 0 300 796
- CHEMICAL ABSTRACTS, Band 113, Nr. 21, 19. November 1990, Columbus, Ohio, USA P. WESTERMANN et al. "Preparation of oligodoxy- ribonucleotide-based affinity carriers" Seite 776, Nr. 191 856t; & DD-A-273 444
- CHEMICAL ABSTRACTS, Band 94, Nr. 5, 2. Februar 1981, Columbus, Ohio, USA S.A. STREL'TSOV et al. "Specific interaction between oligovaline and nucleic acids Seite 185, Nr. 26 354j; & Biofizika 11980, 25(5), 929-41

## Beschreibung

Das gezielte Abschalten von Genexpression durch komplementäre Nukleinsäuren, sogenannte Antisense-Oligonukleotide, stellt einen neuen Therapieansatz dar. Mögliche Anwendungen reichen von der Behandlung viraler Infektionen bis zur Therapie von Krebs (S. Agrawal, Tibtech 10, 152 (1992); W . James, Antiviral Chemistry & Chemotherapie 2, 191 (1991); B. Calabretta, Cancer Research 51, 4505 (1991)). Die Kontrolle der Genexpression erfolgt auf der Ebene von DNA und RNA und gelingt bereits mit unmodifizierten Oligonukleotiden (C. Helene, Anti-Cancer Drug Design 6, 569 (1991); E. Uhlmann, A. Peyman, Chemical Reviews 90, 543 (1990)). Diese sind jedoch aufgrund mangelnder enzymatischer Stabilität und zu geringer Aufnahme in zelluläre Systeme für therapeutische Anwendungen nicht geeignet. Therapeutische Anwendungen erfordern chemisch modifizierte Antisense-Oligonukleotide.

Oligonukleotide mit modifiziertem Internukleotidphosphat oder einer phosphatfreien Internukleotidverknüpfung wurden in vielen Arbeiten systematisch untersucht; ihre Synthese erwies sich jedoch als sehr aufwendig und beobachtete therapeutische Effekte als nicht ausreichend (E. Uhlmann, A. Peyman, Chemical Reviews 90, 543 (1990)).

Eine Alternative zur Modifikation oder Substitution der Phosphatgruppe in Nukleinsäuren ist der komplette Austausch von Ribose und Phosphat durch ein anderes Rückgrat. Dieses Konzept wurde erstmals von Pitha et al. realisiert, der Ribosephosphat durch Poly-N-Vinyl-Derivate ersetzte, was zu sogenannter "Plastik-DNA" führt (J. Pitha, P.O.P. Ts'O, J. Org. Chem. 33 1341 (1968); J. Pitha, . Adv. Polym. Sci. 50, 1 (1983)). Es erlaubt jedoch nicht den gezielten Aufbau definierter Sequenzen.

Die Synthese definierter Sequenzen gelingt, wenn anstelle von Zuckerphosphat beispielsweise ein Polyamid-Rückgrat verwendet wird, das in Analogie zur konventionellen Peptidsynthese (M. Bodanszky, Principles of Peptide Synthesis, Springer, Berlin, 1984) schrittweise aufgebaut wird. Dieses Konzept wurde von verschiedenen Arbeitsgruppen bearbeitet (B.V. Tyaglov, V.I. Permogorov, N.A. Chernykh, Yu. A. Semiletov, K. Konde, Yu.P. Shvachkin, Zh. Obshch. Khim. 57, 1393 (1987); J.E. Summerton et al. WO 86/05518; R.S. Varma et al. WO 92/18518; O. Buchardt et al. WO 92/20702; H. Wang, D.D. Weller, Tetrahedron Letters 32, 7385 (1991); P. Garner, J.U. Yoo, Tetrahedron Letters 34, 1275 (1993); S.-B. Huang, J.S. Nelson, D.D. Weller, J. Org. Chem. 56, 6007 (1991); H. De Koning, U.K. Pandit, Rec. Trav. Chim. 91 1069 (1971); A.B. Cheikh, L.E. Orgel, J. Mol. Evol., 30, 315 (1990)).

Polyamid-Nukleinsäuren eignen sich ebenfalls für diagnostische und molekular-biologische Anwendungen (Buchardt et al. WO 92/20703).

Wir haben neue Nukleinsäuren-bindende Oligomere mit einer C-Verzweigung synthetisiert und für diese wurde eine überraschend gute Bindung an DNA und RNA gefunden. Die Substanzen eignen sich zur Kontrolle von Genexpression und zeigen antivirale Eigenschaften. Weiterhin lassen sich derartige Substanzen in Diagnostik und Molekularbiologie zur Isolierung, Identifizierung und Quantifizierung von Nukleinsäuren verwenden.

Die Erfindung betrifft Verbindungen der allgemeinen Formel (I) 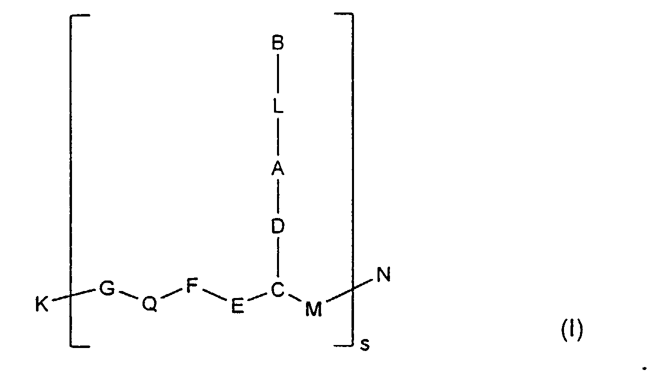 in der
- A: für -CO-, -CHR-, -CRR'- steht,
- B: unabhängig voneinander ausgewählt wird aus einer Gruppe, bestehend aus: (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin oder durch chemische Modifikation von diesen abgeleitete Derivate sowie halogenierten Vorstufen dieser Nukleobasen, sowie geschützte Derivate der Nukleobasen mit den in der Nukleotid- oder Peptidchemie üblichen Schutzgruppen,
- C: für -CH-, -CR- steht, wobei C einheitlich S oder R konfiguriert sein kann,
- D: für -NH-, -CH₂-, -CHR-, -CRR'- steht,
- E: für -NH-, -NR-, -CHR-, -CRR'-, -O-, -S- steht,

A und E miteinander über eine Alkylkette [(-CH₂)ₙ-, mit n = 1, 2] verbunden sein können,
- F: für -CH₂-, -CO-, -SO₂-, -SO-, -CS- steht,
- Q: für (-CR¹R²)ₘ- mit m = 0, 1, 2 und R¹, R² unabhängig voneinander ausgewählt aus einer Gruppe bestehend aus Resten von natürlichen oder unnatürlichen Aminosäuren wie z.B. aus:
Glycin, Alanin, Valin, Leucin, Isoleucin, Serin, Threonin, Cystein, Methionin, Phenylalanin, Tyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, 4-Nitrophenylalanin, 3-Nitrophenylalanin, 2-Nitrophenylalanin, 2-, 3- oder 4-Aminophenylalanin, 3,4-Dichlorphenylalanin, 4-Jodphenylalanin, 4-Methoxyphenylalanin, 1-Triazolylalanin, 2-Pyridylalanin, 3-Pyridylalanin, 4-Pyridylalanin, 1-Naphthylalanin oder 2-Naphthylalanin, gegebenenfalls mit Schutzgruppen, stereochemisch einheitlich in der L-Form oder der D-Form steht,

G und Q miteinander über eine Alkylkette [(-CH₂)ₙ- mit n = 1, 2] verbunden sein können
- G: für -NH-, -NR-, -O-, -S- steht,
- M: für -CH₂-, -CO-, -SO₂-, -SO-, -CS- steht,
- L: für (CH₂)ₚ mit p = 0, 1, 2, -CHR-, -CRR'- steht,
- K: für Carrier-System, Reporter-Ligand, H, löslichkeitsvermittelnde Gruppe steht,
- N: für Carrier-System, Reporter-Ligand, OH, löslichkeitsvermittelnde Gruppe steht und
- s: für einen Wert von 1 bis 30 steht.

Allgemeine Definition der Reste R und R':
R und R' können unabhängig voneinander aus folgender Gruppe ausgewählt werden: H, OH, Alkyl (wobei Alkyl = Methyl, Ethyl, n-Propyl, iso-Propyl, n-Butyl, iso-Butyl oder tert.-Butyl sein kann), Aralkyl (wobei Aralkyl = Benzyl, α-Naphthylmethyl oder β-Naphthylmethyl sein kann) oder Aryl (wobei Aryl = Phenyl, 2-Pyridyl oder 4-Pyridyl gegebenenfalls substituiert mit Methyl, Halogen oder NO₂ sein kann).

Bevorzugt sind Verbindungen der allgemeinen Formel (I)
in der
- A: für -CO-, -CHR-, -CRR'- steht,
- B: unabhängig voneinander ausgewählt wird aus einer Gruppe, bestehend aus: (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin sowie halogenierten Vorstufen dieser Nukleobasen, sowie geschützte Derivate der Nukleobasen mit den in der Nukleotid- oder Peptidchemie üblichen Schutzgruppen,
- C: für -CH-, -CR- steht, wobei C einheitlich S oder R konfiguriert sein kann,
- D: für -NH-, -CH₂-, -CHR-, -CRR'- steht,
- E: für -NR-, -NH-, -CHR-, -CRR'-, -O- steht,

A und E miteinander über eine Alkylkette [-(CH₂)ₙ-, mit n = 1, 2] verbunden sein können,
- F: für -CH₂-, -CO-, -SO₂-, -SO-, -CS- steht,
- Q: für (-CR¹R²)ₘ- mit m = 0, 1, 2 und R¹, R² unabhängig voneinander ausgewählt aus einer Gruppe bestehend aus Resten von natürlichen oder unnatürlichen Aminosäuren wie z.B. aus:
Glycin, Alanin, Valin, Leucin, Isoleucin, Serin, Threonin, Cystein, Methionin, Phenylalanin, Tyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, 4-Nitrophenylalanin, 3-Nitrophenylalanin, 2-Nitrophenylalanin, 1-Naphthylalanin oder 2-Naphthylalanin, gegebenenfalls mit Schutzgruppen, stereochemisch einheitlich in der L-Form oder der D-Form steht,

G und Q miteinander über eine Alkylkette [(-CH₂)ₙ mit n = 1, 2] verbunden sein können
- G: für -NH-, -NR-, -O- steht,
- M: für -CH₂-, -CO-, -SO₂-, -CS- steht,
- L: für (CH₂)ₚ mit p = 0, 1, 2, -CHR-, -CRR'- steht,
- K: für Carrier-System, Reporter-Ligand, H, löslichkeitsvermittelnde Gruppe steht,
- N: für Carrier-System, Reporter-Ligand, OH, löslichkeitsvermittelnde Gruppe steht,
- s: für einen Wert von 1 bis 30 steht,

Allgemeine Definition der Reste R und R':
R und R' können unabhängig voneinander aus folgender Gruppe ausgewählt werden: H, OH, Alkyl (wobei Alkyl = Methyl, Ethyl, n-Propyl oder n-Butyl, sein kann) Benzyl oder Aryl (wobei Aryl = Phenyl, gegebenenfalls substituiert mit Methyl, Halogen oder NO₂ sein kann).

Besonders bevorzugt sind Verbindungen der allgemeinen Formel (I)
in der
- A: für -CO-, -CHR-, -CRR'- steht,
- B: unabhängig voneinander ausgewählt wird aus einer Gruppe, bestehend aus: (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin sowie halogenierten Vorstufen dieser Nukleobasen. sowie geschützte Derivate der Nukleobasen mit den in der Nukleotid- oder Peptidchemie üblichen Schutzgruppen,
- C: für -CH-, -CR- steht, wobei C einheitlich S oder R konfiguriert sein kann,
- D: für -NH-, -CH₂-, -CHR-, -CRR'- steht,
- E: für -NR-, -NH-, -CHR- steht,

A und E miteinander über eine Alkylkette [-(CH₂)ₙ-, mit n = 1, 2] verbunden sein können,
- F: für -CH₂-, -CO-, -CS- steht,
- Q: für (-CR¹R²)ₘ mit m = 0, 1, 2 und R¹, R² unabhängig voneinander ausgewählt aus einer Gruppe bestehend aus Resten von natürlichen oder unnatürlichen Aminosäuren wie z.B. aus:
Glycin, Alanin, Valin,Leucin, Isoleucin, Serin, Threonin, Cystein, Methionin, Phenylalanin, Thyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, gegebenenfalls mit Schutzgruppen, stereochemisch einheitlich in der L-Form oder der D-Form steht,

G und Q miteinander über eine Alkylkette [(-CH₂)ₙ mit n = 1, 2] verbunden sein können
- G: für -NH-, -NR-, -O- steht,
- M: für -CH₂-, -CO-, -CS- steht,
- L: für (CH₂)ₚ mit p = 0, 1, 2, -CHR-, -CRR'- steht,
- K: für Carrier-System, Reporter-Ligand, H, löslichkeitsvermittelnde Gruppe steht,
- N: für Carrier-System, Reporter-Ligand, OH, löslichkeitsvermittelnde Gruppe steht,
- s: für einen Wert von 1 bis 30 steht,

Allgemeine Definition der Reste R und R':
R und R' können unabhängig voneinander aus folgender Gruppe ausgewählt werden: H, OH, Methyl oder Benzyl.

### Monomere- und Dipeptid-Bausteine für Peptid-Nukleinsäuren

Die Erfindung betrifft weiterhin Verbindungen der allgemeinen Formel (II) 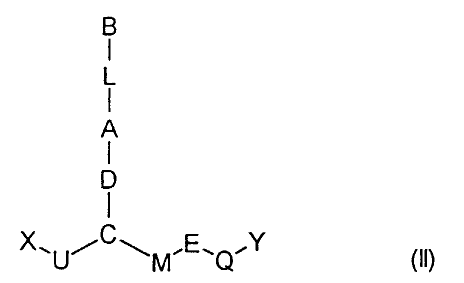 in der
- A: für -CO-, -CHR-, -CRR'- steht,
- B: unabhängig voneinander gewählt wird aus einer Gruppe, bestehend aus: (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin oder durch chemische Modifikation von diesen abgeleiteten Derivaten, sowie halogenierten Vorstufen dieser Nukleobasen, sowie geschützte Derivate dieser Nukleobasen mit den in der Nukleotid- oder Peptidchemie üblichen Schutzgruppen,
- C: für -CH-, -CR- steht, wobei C einheitlich S oder R konfiguriert sein kann,
- D: für -NH-, -CH₂-, -CHR-, -CRR'- steht,
- E: für -NR-, -NR-, CHR-, -CRR'-, -O-, -S- steht,
- Q: für (-CR¹R²-)ₘ mit m = 0, 1, 2 und R¹, R² unabhängig voneinander ausgewählt aus einer Gruppe bestehend aus Resten von natürlichen oder unnatürlichen Aminosäuren wie z.B. aus: Glycin, Alanin, Valin, Leucin, Isoleucin, Serin, Threonin, Cystein, Methionin, Phenylalanin, Tyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, 4-Nitrophenyl alanin, 3-Nitrophenylalanin, 2-Nitrophenylalanin, 2-, 3- oder 4-Aminophenylalanin, 3,4-Dichlorphenylalanin, 4-Jodphenylalanin, 4-Methoxyphenylalanin, 1-Triazolylalanin, 2-Pyridylalanin, 3-Pyridylalanin, 4-Pyridylalanin, 1-Naphthylalanin oder 2-Naphthylalanin, gegebenenfalls mit Schutzgruppen, stereochemisch einheitlich in der L-Form oder der D-Form steht,

E und Q miteinander über eine Alkylkette [(-CH₂)ₙ- mit n = 1, 2] verbunden sein können
- M: für -CH₂-, -CO-, -SO₂-, -SO-, -CS- steht,
- L: für (CH₂)ₚ mit p = 0, 1, 2, -CHR-, -CRR'- steht,
- U: für -NH-, -NR-,
- X: für eine beliebige aus der Peptidchemie bekannte Schutzgruppe, H oder eine beliebige natürliche oder unnatürliche Aminosäure in geschützter oder ungeschützter Form und
- Y: für COOH, CSOH, CH₂OH, COOR" mit R" = beliebige Schutzgruppe aus der Peptidchemie, Carrier, Reporter-Ligand, löslichkeitsvermittelnde Gruppe steht.

Allgemeine Definition der Reste R und R':
R und R' können unabhängig voneinander aus folgender Gruppe ausgewählt werden: H, OH, Alkyl (wobei Alkyl = Methyl, Ethyl, n-Propyl, iso-Propyl, n-Butyl, iso-Butyl oder tert.-Butyl sein kann), Aralkyl (wobei Aralkyl = Benzyl, α-Naphthylmethyl oder β-Naphthylmethyl sein kann) oder Aryl (wobei Aryl = Phenyl, 2-Pyridyl oder 4-Pyridyl gegebenenfalls substituiert mit Methyl, Halogen oder NO₂ sein kann).

Bevorzugt sind Verbindungen der allgemeinen Formel (II)
in der
- A: für -CO-, -CHR-, -CRR'- steht,
- B: unabhängig voneinander gewählt wird aus einer Gruppe, bestehend aus: (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin sowie halogenierten Vorstufen dieser Nukleobasen, sowie geschützte Derivate dieser Nukleobasen mit den in der Nukleotid- oder Peptidchemie üblichen Schutzgruppen,
- C: für -CH-, -CR- steht, wobei C einheitlich S oder R konfiguriert sein kann,
- D: für -NH-, -CH₂-, -CHR-, -CRR'- steht,
- E: für -NR-, -NH-, -CHR-, -CRR'-, -O- steht,
- Q: für (-CR¹R²-)ₘ mit m = 0, 1, 2 und R¹, R² unabhängig voneinander ausgewählt aus einer Gruppe bestehend aus Resten von natürlichen oder unnatürlichen Aminosäuren wie z.B. aus: Glycin, Alanin, Valin, Leucin, Isoleucin, Serin, Threonin, Cystein, Methionin, Phenylalanin, Thyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, 4-Nitrophenylalanin, 3-Nitrophenylalanin, 2-Nitrophenylalanin, 1-Naphthylalanin oder 2-Naphthylalanin, gegebenenfalls mit Schutzgruppen, stereochemisch einheitlich in der L-Form oder der D-Form steht,

E und Q miteinander über eine Alkylkette [(-CH₂-)ₙ mit n = 1, 2] verbunden sein können
- M: für -CH₂-, -CO-, -SO₂-, -SO-, -CS- steht,
- L: für (CH₂)ₚ mit p = 0, 1, 2, -CHR-, -CRR'- steht,
- U: für -NH-, -NR-,
- X: für eine beliebige aus der Peptidchemie bekannte Schutzgruppe, H oder eine beliebige natürliche oder unnatürliche Aminosäure in geschützter oder ungeschützter Form und
- Y: für COOH, CSOH, CH₂OH, COOR" mit R" = beliebige Schutzgruppe aus der Peptidchemie, Carrier, Reporter-Ligand, löslichkeitsvermittelnde Gruppe steht.

Allgemeine Definition der Reste R und R':
R und R' können unabhängig voneinander aus folgender Gruppe ausgewählt werden: H, OH, Alkyl (wobei Alkyl = Methyl, Ethyl, n-Propyl oder n-Butyl, sein kann), Benzyl oder Aryl (wobei Aryl = Phenyl, gegebenenfalls substituiert mit Methyl, Halogen oder NO₂ sein kann).

Besonders bevorzugt sind Verbindungen der allgemeinen Formel (II)
in der
- A: für -CO-, -CHR-, -CRR'- steht,
- B: unabhängig voneinander gewählt wird aus einer Gruppe, bestehend aus: (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin sowie halogenierten Vorstufen dieser Nukleobasen sowie geschützte Derivate dieser Nukleobasen mit den in der Nukleotid- oder Peptidchemie üblichen Schutzgruppen,
- C: für -CH-, -CR- steht, wobei C einheitlich S oder R konfiguriert sein kann,
- D: für -NH-, -CH₂-, -CHR-, -CRR'- steht,
- E: für -NR-, -NH-, -CHR-, -O- steht,
- Q: für (-CR¹R²-)ₘ mit m = 0, 1, 2 und R¹, R² unabhängig voneinander ausgewählt aus einer Gruppe bestehend aus Resten von natürlichen oder unnatürlichen Aminosäuren wie z.B. aus: Glycin, Alanin, Valin, Leucin, Isoleucin, Serin, Threonin, Cystein, Methionin, Phenylalanin, Thyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, gegebenenfalls mit Schutzgruppen, stereochemisch einheitlich in der L-Form oder der D-Form steht,

E und Q miteinander über eine Alkylkette [(-CH₂-)ₙ mit n = 1, 2] verbunden sein können
- M: für -CH₂-, -CO-, -CS- steht,
- L: für (CH₂)ₚ mit p = 0, 1, 2, -CHR-, -CRR'- steht,
- U: für -NH-, -NR-,
- X: für eine beliebige aus der Peptidchemie bekannte Schutzgruppe, H oder eine beliebige natürliche oder unnatürliche Aminosäure in geschützter oder ungeschützter Form und
- Y: für COOH, CSOH, CH₂OH, COOR" mit R" = beliebige Schutzgruppe aus der Peptidchemie, Carrier, Reporter-Ligand, löslichkeitsvermittelnde Gruppe steht.

Allgemeine Definition der Reste R und R':
R und R' können unabhängig voneinander aus folgender Gruppe ausgewählt werden: H, OH, Methyl oder Benzyl.

Die Erfindung betrifft auch Verbindungen der allgemeinen Formel (III) 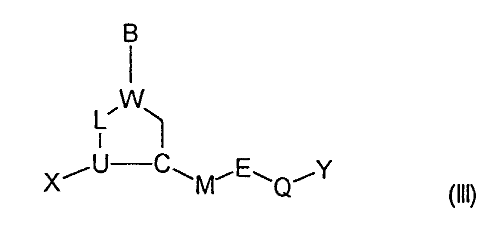 in der
- B: unabhängig voneinander gewählt wird aus einer Gruppe, bestehend aus: (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin oder durch chemische Modifikation von diesen abgeleiteten Derivaten, sowie halogenierten Vorstufen dieser Nukleobasen sowie geschützte Derivate dieser Nukleobasen mit den in der Nukleotid- oder Peptidchemie üblichen Schutzgruppen,
- C: für -CH-, -CR- steht, wobei C einheitlich S oder R konfiguriert sein kann,
- E: für -NR-, -CHR-, -CRR'-, -O-, -S- steht,
- Q: für (-CR¹R²-)ₘ mit m = 0, 1, 2 und R¹, R² unabhängig voneinander ausgewählt aus einer Gruppe bestehend aus Resten von natürlichen oder unnatürlichen Aminosäuren wie z.B. aus: Glycin, Alanin, Valin, Leucin, Isoleucin, Serin, Threonin, Cystein, Methionin, Phenylalanin, Thyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, 4-Nitrophenylalanin, 3-Nitrophenylalanin, 2-Nitrophenylalanin, 2-, 3- oder 4-Aminophenylalanin, 3,4-Dichlorphenylalanin, 4-Jodphenylalanin, 4-Methoxyphenylalanin, 1-Triazolylalanin, 2-Pyridylalanin, 3-Pyridylalanin, 4-Pyridylalanin, 1-Naphthylalanin oder 2-Naphthylalanin, gegebenenfalls mit Schutzgruppen, stereochemisch einheitlich in der L-Form oder der D-Form steht,

E und Q miteinander über eine Alkylkette [(-CH₂-)ₙ mit n = 1, 2] verbunden sein können
- M: für -CH₂-, -CO-, -SO₂-, -SO-, -CS- steht,
- L: für -CH₂- steht,
- U: für -NH-, -NR- steht,
- X: für eine beliebige aus der Peptidchemie bekannte Schutzgruppe, H oder eine beliebige natürliche oder unnatürliche Aminosäure in geschützter oder ungeschützter Form steht,
- Y: für COOH, CSOH, CH₂OH, COOR" mit R" = beliebige Schutzgruppe aus der Peptidchemie, Carrier, Reporter-Ligand, löslichkeitsvermittelnde Gruppe steht,
- W: für ein chirales C-Atom steht, welches einheitlich S oder R konfiguriert sein kann.

Allgemeine Definition der Reste R und R':
R und R' können unabhängig voneinander aus folgender Gruppe ausgewählt werden: H, OH, Alkyl (wobei Alkyl = Methyl, Ethyl, n-Propyl, iso-Propyl, n-Butyl, iso-Butyl oder tert.-Butyl sein kann), Aralkyl (wobei Aralkyl = Benzyl, α-Naphthylmethyl oder β-Naphthylmethyl sein kann) oder Aryl (wobei Aryl = Phenyl, 2-Pyridyl oder 4-Pyridyl gegebenenfalls substituiert mit Methyl, Halogen oder NO₂ sein kann).

Bevorzugt sind Verbindungen der allgemeinen Formel (III)
in der
- B: unabhängig voneinander gewählt wird aus einer Gruppe, bestehend aus: -H, -OH, (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin sowie halogenierten Vorstufen dieser Nukleobasen, sowie geschützte Derivate dieser Nukleobasen mit den in der Nukleotid- oder Peptidchemie üblichen Schutzgruppen,
- C: für -CH-, -CR- steht, wobei C einheitlich S oder R konfiguriert sein kann,
- E: für -NR-, -NH-, -CHR-, -CRR'-, -O- steht,
- Q: für (-CR¹R²-)ₘ mit m = 0, 1, 2 und R¹, R² unabhängig voneinander ausgewählt aus einer Gruppe bestehend aus Resten von natürlichen oder unnatürlichen Aminosäuren wie z.B. aus: Glycin. Alanin, Valin, Leucin, Isoleucin, Serin, Threonin, Cystein, Methionin, Phenylalanin, Thyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, 4-Nitrophenylalanin, 3-Nitrophenylalanin, 2-Nitrophenylalanin, 1-Naphthylalanin oder 2-Naphthylalanin, gegebenenfalls mit Schutzgruppen, stereochemisch einheitlich in der L-Form oder der D-Form steht,

E und Q miteinander über eine Alkylkette [(-CH₂-)ₙ mit n = 0, 1, 2] verbunden sein können
- M: für -CH₂-, -CO-, -SO₂-, -SO-, -CS- steht,
- L: für -CH₂- steht,
- U: für -NH-, -NR-,
- X: für eine beliebige aus der Peptidchemie bekannte Schutzgruppe, H oder eine beliebige natürliche oder unnatürliche Aminosäure in geschützter oder ungeschützter Form steht,
- Y: für COOH, CSOH, CH₂OH, COOR" mit R" = beliebige Schutzgruppe aus der Peptidchemie, Carrier, Reporter-Ligand, löslichkeitsvermittelnde Gruppe steht,
- W: für ein chirales C-Atom steht, welches einheitlich S oder R konfiguriert sein kann.

Allgemeine Definition der Reste R und R':
R und R' können unabhängig voneinander aus folgender Gruppe ausgewählt werden: H, OH, Alkyl (wobei Alkyl = Methyl, Ethyl, n-Propyl oder n-Butyl, sein kann), Benzyl oder Aryl (wobei Aryl = Phenyl, gegebenenfalls substituiert mit Methyl, Halogen oder NO₂ sein kann).

Besonders bevorzugt sind Verbindungen der allgemeinen Formel (III)
in der
- B: unabhängig voneinander gewählt wird aus einer Gruppe, bestehend aus: -H, -OH, (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin sowie halogenierten Vorstufen dieser Nukleobasen sowie geschützte Derivate dieser Nukleobasen mit den in der Nukleotid- oder Peptidchemie üblichen Schutzgruppen,
- C: für -CH-, -CR- steht, wobei C einheitlich S oder R konfiguriert sein kann,
- E: für -NR-, -NH-, -CHR-, -O- steht,
- Q: für (-CR¹R²-)ₘ mit m = 0, 1, 2 und R¹, R² unabhängig voneinander ausgewählt aus einer Gruppe bestehend aus Resten von natürlichen oder unnatürlichen Aminosäuren wie z.B. aus: Glycin, Alanin, Valin, Leucin, Isoleucin, Serin, Threonin, Cystein, Methionin, Phenylalanin, Thyrosin, Histidin, Tryptophan, Lysin, Ornithin, Asparaginsäure, Glutaminsäure, Asparagin, Glutamin, Arginin, Prolin, Hydroxyprolin. Sarcosin, Aminoisobuttersäure, Dehydroaminosäuren wie z.B. Dehydroalanin, Dehydro-α-aminobuttersäure, andere unnatürliche Aminosäuren wie Phenylglycin, gegebenenfalls mit Schutzgruppen, stereochemisch einheitlich in der L-Form oder der D-Form steht,

E und Q miteinander über eine Alkylkette [(-CH₂-)ₙ mit n = 1, 2] verbunden sein können
- M: für -CH₂-, -CO-, -CS- steht,
- L: für -CH₂- steht,
- U: für -NH-, -NR-,
- X: für eine beliebige aus der Peptidchemie bekannte Schutzgruppe, H oder eine beliebige natürliche oder unnatürliche Aminosäure in geschützter oder ungeschützter Form steht,
- Y: für COOH, CSOH, CH₂OH, COOR" mit R" = beliebige Schutzgruppe aus der Peptidchemie, Carrier, Reporter-Ligand, löslichkeitsvermittelnde Gruppe steht,
- W: für ein chirales C-Atom steht, welches einheitlich S oder R konfiguriert sein kann.

Allgemeine Definition der Reste R und R':
R und R' können unabhängig voneinander aus folgender Gruppe ausgewählt werden: H, OH, Methyl oder Benzyl.

Mit Carrier-System oder Reporter-Ligand ist gemeint ein zellspezifisches Bindungs- und Erkennungs-Agens, das spezifisch an die Zelloberfläche bindet und das die Internalisierung der der Erfindung zugrunde liegenden Nukleinsäure-bindenden Oligomeren bewirkt. Die Internalisierung kann auf verschiedenem Wege, z.B. durch Endocytose oder aktive Transportmechanismen erfolgen.

Die Struktur der Zelloberfläche kann ein Protein, Polypeptid, Kohlenhydrat, Lipid oder eine Kombination daraus sein. Typischerweise wird die Zellaufnahme durch Oberflächenrezeptoren bewirkt. Deshalb kann das Bindungs- und Erkennungs-Agens ein natürlicher oder synthetischer Ligand eines Rezeptors sein.

Der Ligand kann ein Protein, Polypeptid, Kohlenhydrat, Lipid oder eine Kombination von diesen sein, ausgestattet mit funktionellen Gruppen, die so angeordnet sind, daß sie durch die Zelloberflächen-Struktur erkannt werden können. Es kann sich auch um eine Komponente oder die Gesamtheit eines biologischen Organismus handeln, z.B. eines Virus, einer Zelle oder um artifizielle Transportsysteme, wie z.B. Liposomen. Es kann sich weiterhin um einen Antikörper oder ein Analogon eines Antikörpers handeln.

Für die Adressierung an unterschiedliche Zellen müssen verschiedene Liganden eingesetzt werden.

Als Liganden für die Adressierung an Makrophagen kommen bevorzugt Kohlenhydrate, wie z.B. Mannose, Polykationen, wie z.B. Polylysine, Polyarginine, Polyornithine, basische Proteine, wie z.B. Avidin, sowie Glycopeptide. Peptide oder Lipopeptide in Frage (G.Y. Chu et al., WO 9304701).

Mit löslichkeitsvermittelnden Gruppen sind funktionelle Gruppen gemeint die die Löslichkeit in Wasser vermitteln. Dabei kann es sich z.B. um Ester oder Amide von Aminosäuren, Hydroxycarbonsäuren, Aminosulfonsäuren, Hydroxysulfonsäuren oder Diaminen handeln. Bevorzugt sind Amide von Diaminocarbonsäure, wie Ornithin, Lysin oder 2,4-Diaminobuttersäure.

### Peptid-Nukleinsäuren mit α,ω-Diaminocarbonsäure-Backbone

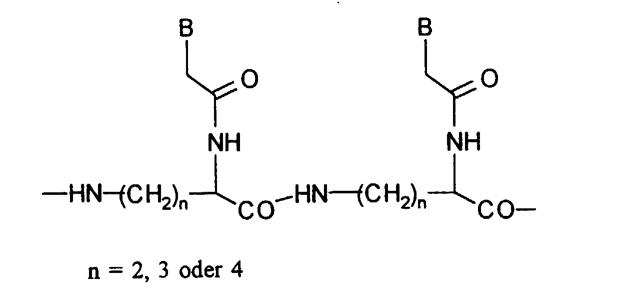

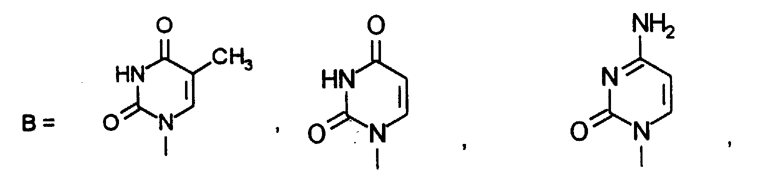

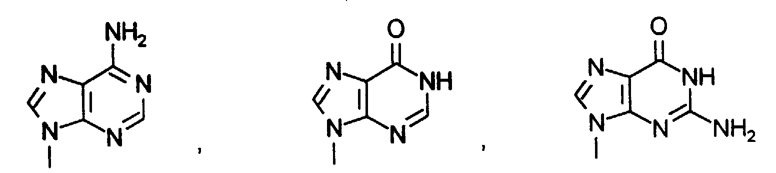

usw.

Bei den Verbindungen dieses Typs ist das Zuckerphosphat-Rückgrat der natürlichen Nukleinsäuren durch ein über die Seitenketten verknüpftes Polymeres aus α,ω-Diaminocarbonsäuren, wie z.B. Lysin, Ornithin oder 2,4-Diaminobuttersäure ersetzt. Die Nukleobasen sind über Acyl-Spacer an die α-Aminogruppen gebunden. Es resultieren relativ flexible Oligomere.

### Peptid-Nukleinsäuren mit 2-Aminobutyrylglycin-Backbone

Bei den Verbindungen dieses Typs ist das Ribosephosphat- bzw. Desoxyribosephosphat-Backbone von RNA bzw. DNA durch ein Peptidbackbone aus 2-Aminobutyrylglycin-Dipeptiden ersetzt. Das resultierende Oligomere zeichnet sich durch seine hohe Flexibilität aus. Durch den Kettenaufbau mit chiralen α-Aminosäuren wird zudem die Variationsbreite (Änderung der Chiralität, Verwendung anderer Spacer anstelle von Glycin) bei der Oligomerensynthese enorm erhöht.

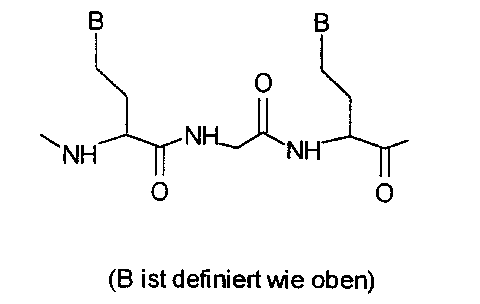

### Peptid-Nukleinsäuren mit Pyrrolidin-2-carboxyglycin-Backbone

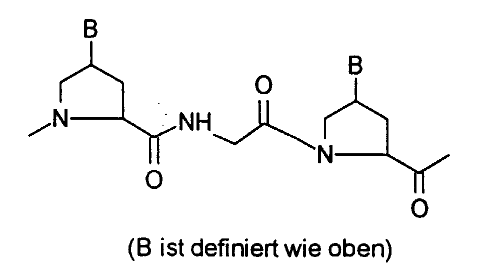

Bei diesem Strukturtyp ist das Zuckerphosphat-Backbone der natürlichen Nukleinsäuren durch ein Peptidbackbone aus Pyrrolidin-2-carboxylglycin-Dipeptiden ersetzt. Durch den Einsatz von Pyrrolidin-2-carbonsäure im Backbone erhält man rigide Strukturen. Durch Variation der Chiralitätszentren an der Pyrrolidin-2-carbonsäure erhält man Oligomere mit unterschiedlicher Konformation. Außerdem erlaubt auch dieser Ansatz das Ersetzen des Glycinspacers durch andere Spacer wie z.B. andere Aminosäuren oder Hydroxycarbonsäuren..

### Biologische Eigenschaften bzw. Wirkungen dieser Verbindungen

### Stabilität gegenüber Proteasen und Nukleasen

Neben der Kettenlänge, der Sequenz und der Zellpermeabilität spielt die Protease- und Nukleaseresistenz eine wichtige Rolle für die biologische Wirkung von Nukleinsäure-bindenden Oligomeren.

Die synthetisierten Oligomeren wurden deshalb in Bezug auf ihre Protease- und Nukleasestabilität mit natürlichen Oligonukleotiddiestern verglichen.

Die Nukleinsäure bindenden Oligomere wurden hierzu mit unspezifischen und spezifischen Proteasen wie z.B. Pronase E, Proteinase K, Trypsin, Endoprotease Lys-C, V8 Protease, Protease IX, Protease XXI, und Nukleasen wie z.B. S1 Nuklease, Bal31 Nuklease, Phosphodiesterase sowie Zellextrakte, Organextrakte, Blutserum und Blutextrakte, die verschiedene Nukleasen und Proteasen enthalten, behandelt. Durch Polyacrylamidgelelektrophorese und UV-Shadowing auf DC-Platten mit UV-Indikator sowie durch Silberanfärbung der Polyacrylamidgele wurden die Oligomeren auf Degradation überprüft.

Natürliche Oligonukleotid-Diester weisen eine nur geringe Nukleasestabilität auf. Sie werden innerhalb von 30 Minuten bis zu 1 Stunde vollständig degradiert.

Nukleinsäuren-bindende Oligomere mit C-Verzweigung sind dagegen vollständig resistent gegenüber Nukleasen und Proteasen und eignen sich deshalb besonders gut für den Einsatz als Inhibitoren mit Antisense Wirkung.

### DNA-Einzelstrangbindung durch Gel-Shift-Analysen

Die hier beschriebenen Nukleinsäure-bindenden Oligomeren wurden in Gel-Shift-Analysen untersucht. Bei diesen Band-Shift-Experimenten wurde das geänderte Laufverhalten von radioaktiv-markierten Oligonukleotiden nach Hybridisierung mit den hier beschriebenen Oligomeren in einer Polyacrylamidgelelektrophorese gemessen. Die hybridisierten Oligonukleotide wandern in der Elektrophorese langsamer, weil sich durch die Hybridbildung erstens das Molekulargewicht vergrößert und zweitens sich die Ladung pro Masseeinheit im Hybridkomplex verringert. Im Vergleich zu einem nicht hybridisierten Oligonukleotid kommt es im Gel zu einem verzögerten Laufverhalten (Gel-Retardation).

### Strangverdrängung in doppelsträngiger Plasmid-DNA

Nukleinsäure-bindende Oligomere sind biologisch aktiv, indem sie sequenzselektiv die Bindung durch Strangverdrängung an doppelsträngige DNA (dsDNA) zeigen. Diese Wirkung von Nukleinsäure-bindenden Oligomeren läßt sich sequenzabhängig und konzentrationsabhängig in in-vitro Tests nachweisen.

### Inhibition der Genexpression (In-vitro-Translationstest)

Nukleinsäure-bindende Oligomere, die in Gel-Shift- und Strangverdrängungs-Experimenten aufgefallen sind, wurden auf ihre Fähigkeit getestet, die Proteinsynthese spezifischer Gene zu inhibieren. Vorraussetzung dafür ist, daß in dem betreffenden Gen die entsprechende Sequenz des Nukleinsäure-bindenden Oligomeren in paralleler oder antiparalleler Basensequenz enthalten ist. Es hatte sich bei den in vitro Translationen gezeigt, daß die hier beschriebenen Nukleinsäure-bindenden Oligomeren sehr potente, sequenzspezifische Inhibitoren für Genexpressionen sind. Dabei waren kürzere Sequenzen und geringere Konzentrationen als bei sequenzgleichen Oligonukleotiden ausreichend für bessere Hemmungen.

Die Zielsequenz kann aus dem Promotor eines krankheitsauslösenden Gens stammen. Insbesondere Enhancer- oder Transkriptionsfaktoren und DNA- oder RNA-Polymerase bindende Zielsequenzen aus Genen von Viren, Bakterien, Pilzen, Endoparasiten, Oncogenen oder Genen, die an der Ausprägung von Entzündungserkrankungen, Autoimmunerkrankungen, oder Herzkreislauferkankungen wie Bluthochdruck oder Arteriosklerose beteiligt sind, sind hier als mögliche Zielsequenzen für die therapeutische Anwendung von Nukleinsäuren-bindenden Oligomeren zu nennen.

Die entsprechenden pharmazeutischen Zubereitungen enthalten neben Oligomeren mit C-Verzweigung die für parenterale Zubereitungen üblichen Hilfsstoffe wie z.B. Puffer und/oder Stabilisatoren oder Liposomenformulierungen. Ferner ist eine topische Anwendung denkbar. Die hierfür einsetzbaren Zubereitungen sind z.B. Salben, Cremes, Lösungen oder Pflaster, die neben dem Wirkstoff die für diese Anwendung geeigneten pharmazeutischen Hilfsstoffe enthalten.

Therapeutisch wirksame Nukleinsäuren bindende Oligomere, wie sie hier beschrieben sind, können nicht nur wie oben erwähnt die Genexpression durch die sequenz-selektive Bindung an mRNA hemmen, sondern natürlich auch aufgrund ihrer Eigenschaft doppelsträngige DNA zu verdrängen, die Promotor- und Enhancersequenzen von zu hemmenden Genen sequenz-selektiv inaktivieren.

Nukleinsäuren-bindende Oligomere enthalten für diese Anwendung der Gen-Inaktivierung nicht nur Nukleobasensequenzen von (-)-Strang DNA sondern durchaus auch die (+)-Strang DNA Sequenz der zu hemmenden Ziel-DNA.

### 5. Synthese von monomeren Bausteinen

### 5.1 Monomere für Peptid-Nukleinsäuren mit α,ω-Diaminocarbonsäure-Backbone

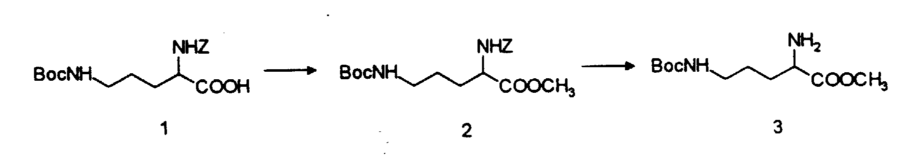

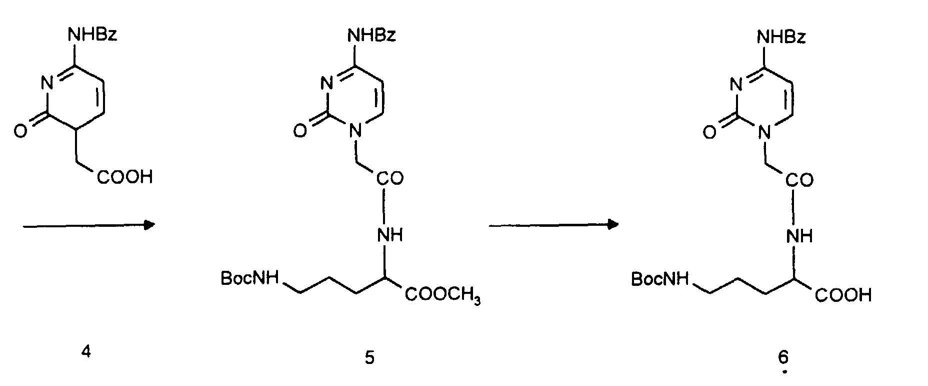

Die Synthese der Monomeren für Peptid-Nukleinsäuren mit α,ω-Dianünocarbonsäure-Backbone sei am Beispiel des Cytosin-Ornithin-Derivates 6 erläutert:
α-N-(Benzyloxycarbonyl)-δ-N-(tert.-butoxycarbonyl)-L-ornithin 1 wird mit Methyliodid und Cäsiumcarbonat in den Methylester 2 überführt. Die α-N-Benzyloxycarbonyl-Schutzgruppe wird hydrogenolytisch entfernt. Das Derivat 3 mit freier α-Aminogruppe wird in Gegenwart eines Kondensationsmittels, z.B. N,N'-Dicyclohexylcarbodiimid, mit N⁴-Benzoyl-1-carboxymethyl-cytosin 4 zur Reaktion gebracht. Dabei resultiert 5. Alternativ ist die Verknüpfung auch unter Verwendung von Aktivestern, z.B. Pentafluorphenylestern der 1-Carboxymethylnukleobasen möglich.

Der Methylester in 5 wird in Gegenwart einer Base verseift. Die Benzoat-Schutzgruppe wird nicht angegriffen.

Die Peptid-Nukleinsäure 6 ist für den Einsatz in der Festphasen-Peptidsynthese unter "Boc-Bedingungen" geeignet.

Derivate anderer Nukleobasen und anderer α,ω-Diaminocarbonsäuren, wie z.B. Lysin und 2,4-Diaminobuttersäure sind analog zugänglich. Außerdem können anstelle der L-Aminosäurederivate auch die D-Aminosäurederivate eingesetzt werden.

### 5.2 Monomere für Peptid-Nukleinsäuren mit 2-Aminobutyryl-glycin-Backbone

Als Beispiel für die Monomerensynthese wird der Thyminbaustein angeführt: 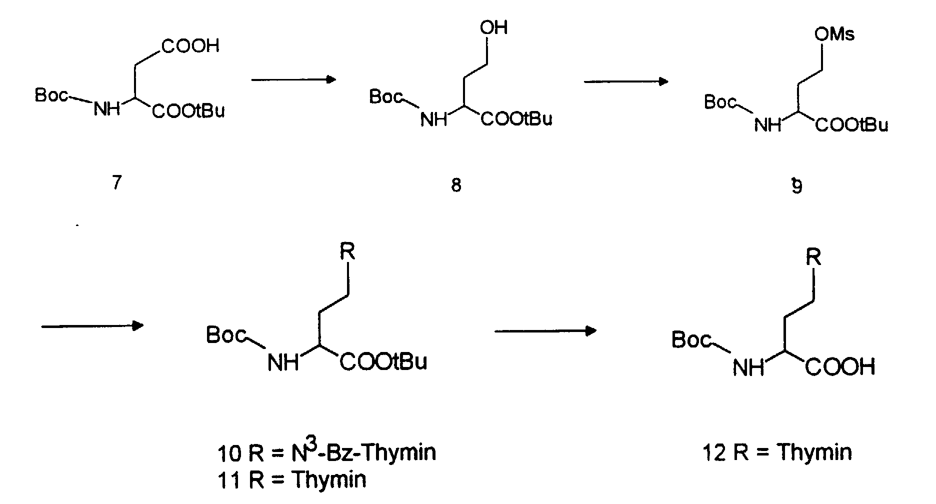 N-tert.-Butyloxycarbonyl-L-Asparaginsäure wird in einem aprotischen dipolaren Lösungsmittel mit einem komplexen Hydrid zu N-tert.-Butyloxycarbonyl-L-Homoserin-tert.-butylester 8 reduziert (außer der L-Asparaginsäure läßt sich die Synthese auch mit D-Asparaginsäure durchführen). Die Hydroxylgruppe wird in eine Abgangsgruppe überführt (z.B. zu 9) und gegen eine heterocyclische Nukleobase (z.B. 10) substituiert. Anschließend werden die Schutzgruppen abgespalten und die α-Aminofunktion geschützt (z.B. 12). Das 2-Aminobutyrylglycin-Backbone erhält man bei der Oligomerisierung dadurch, daß alternierend der 2-Aminobutyryl-Baustein und ein Glycin-Baustein verwendet werden.

Andere Backbone-Typen erhält man ganz einfach durch Verwendung anderer Spacer anstelle des Glycins bei der Oligomerisierung.

### 5.3 Monomere für Peptid-Nukleinsäuren mit Pyrrolidin-2-carboxyl-glycin-Backbone

Als Beispiel für die Monomerensynthese wird der Thyminbaustein aufgeführt:
Ein N- und C-terminal geschützter Hydroxyprolin-Baustein (z.B. 13 oder 14) wird in einem aprotischen Lösungsmittel in einer Mitsunobu Reaktion mit einer Nukleobase umgesetzt, wobei z.B. 15 oder 16 erhalten wird. Anschließend wird der Ester gespalten und bei 15 oder 16 zusätzlich die Nukleobase entschützt, so daß man 17 erhält. Ausgehend vom L-trans-Hydroxyprolinderivat 14 erhält man dabei das L-cis-Pyrrolidin-2-carbonsäureprodukt 17. Um in die Reihe der L-trans-Pyrrolidin-2-carbonsäureprodukte zu gelangen benötigt man L-cis-Hydroxyprolinderivate, welche man durch Chiralitätsumkehr in der 4-Position aus den. L-trans-Hydroxyprolinderivaten erhält (z.B. auf dem Weg von 14 in einer Mitsunobu Reaktion über 18 nach 19). Der weitere Syntheseweg zu 21 erfolgt dann analog zu den Umsetzungen von 14 nach 17. Außer den L-cis- und den L-trans-Produkten lassen sich auch die D-cis und die D-trans Produkte herstellen.

Andere Backbonetypen erhält man durch Verwendung anderer Spacer anstelle des Glycins bei der Oligomerisierung.

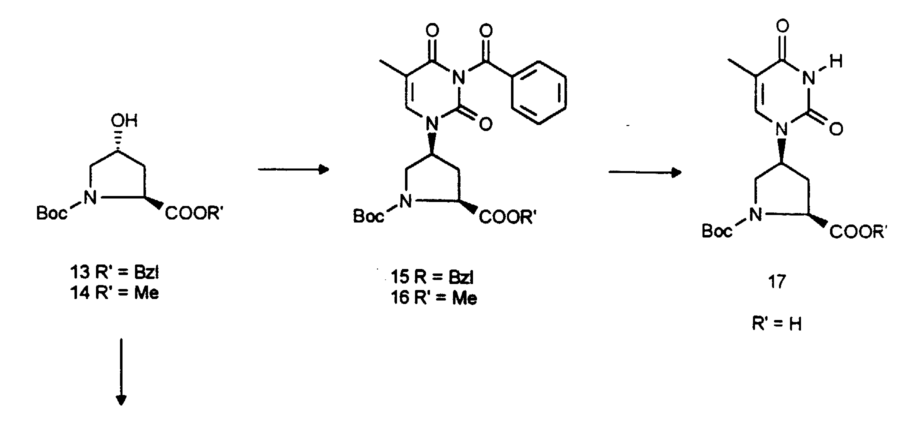

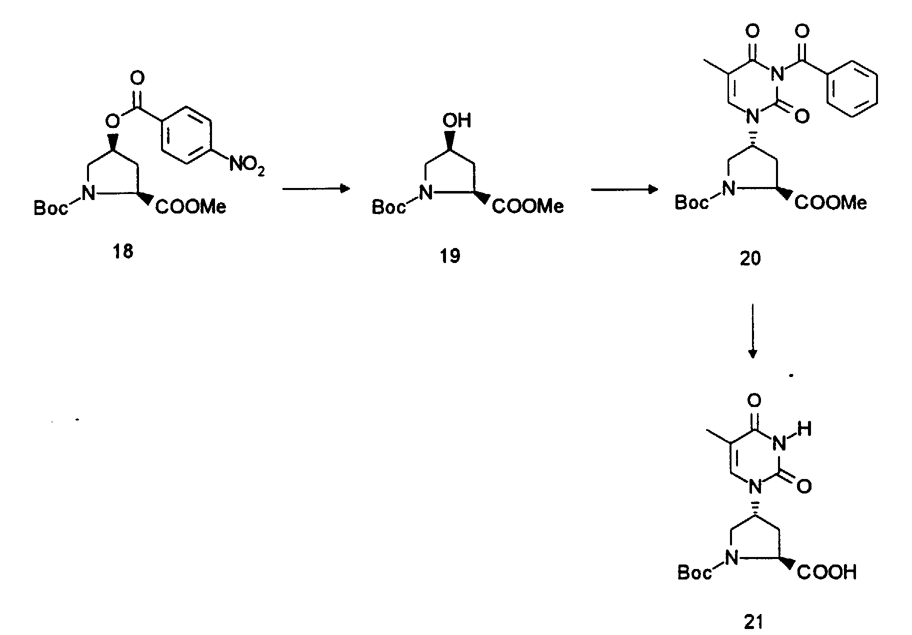

### Dipeptidbausteine für Peptid-Nukleinsäuren mit 2-Aminobutyryl-glycin-Backbone

Zur Synthese der Dipeptide wird z.B. N-Boc-4-(thymin-1-yl)-2-L-aminobuttersäure 12 mit Glycinmethylester in Gegenwart von EDCI*HCl und HOBt*H₂O zu 22 gekuppelt. Anschließend wird der Ester verseift, was zur Verbindung 23 führt.

Außer Glycin lassen sich auf vergleichbarem Weg auch andere natürliche oder unnatürliche Aminosäuren ankuppeln. Die erhaltenen Dipeptide dienen dann zur Oligomerisierung.

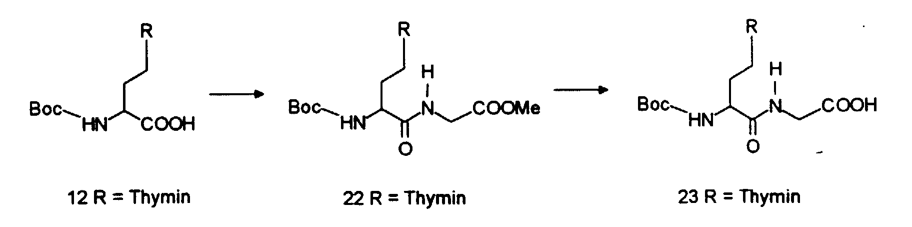

### Dipeptidbausteine für Peptid-Nukleinsäuren mit Pyrrolidin-2-carboxyl-glycin-Backbone

Zur Synthese der Dipeptide wird z.B. 2S,4S-N-Boc-4-(thymin-1-yl)pyrrolidin-2-carbonsäure 17 mit Glycinmethylester in Gegenwart von EDCI*HCl und HOBt*H₂O zu 24 gekuppelt. Anschließend wird der Ester verseift, was zur Verbindung 25 führt.

Außer Glycin lassen sich auf vergleichbarem Weg auch andere natürliche oder unnatürliche Aminosäuren ankuppeln. Die erhaltenen Dipeptide dienen dann zur Oligomerisierung.

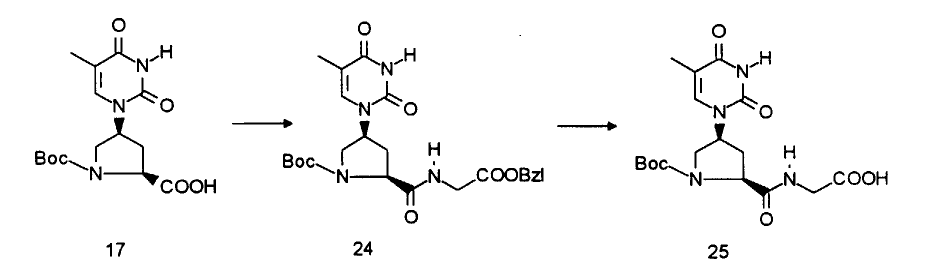

### Oligomeren-Synthesen

Die Verknüpfung der Bausteine zu Oligomeren erfolgt durch Festphasen-peptidsynthese. Als polymere Träger wurden PAM-, MBHA- oder HMP-Resins der Firma Applied Biosystems eingesetzt. Die Bausteine werden in Analogie zur konventionellen Peptidsynthese entweder nach dem Fmoc- oder Boc-Verfahren verknüpft. Die Aktivierung der Bausteine erfolgt in N-Methyl-2-pyrrolidon durch Umsetzung mit Hydroxybenzotriazol/Dicyclohexylcarbodiimide oder Pentafluorphenol/Dicyclohexylcarbodiimid. Die Sequenzen werden im Anschluß durch Behandlung mit HF oder Trifluormethansulfonsäure (Boc-Methode, PAM- oder MBHA-Resin) oder durch Trifluoressigsäure (Fmoc-Methode, HMP-Resin) abgespalten. Die Reaktionsprodukte werden durch präparative HPLC an RP 8 mit einem ansteigenden Gradienten von Trifluoressigsäure in Acetonitril/Wasser isoliert. Nach dieser Methode wurden Sequenzen mit Kettenlängen bis 15 Bausteinen synthetisiert.

Zur Oligomerisierung wurden vorzugsweise nachfolgend aufgeführte α,ω-Diaminocarbonsäure-Bausteine eingesetzt. Alternativ zur Boc-Schutzgruppe kann ebenfalls die Fmoc-Schutzgruppe eingesetzt werden.

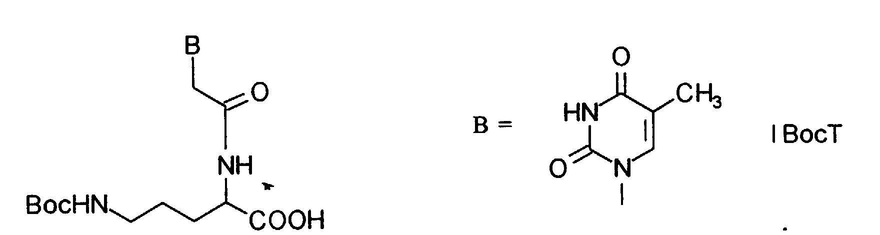

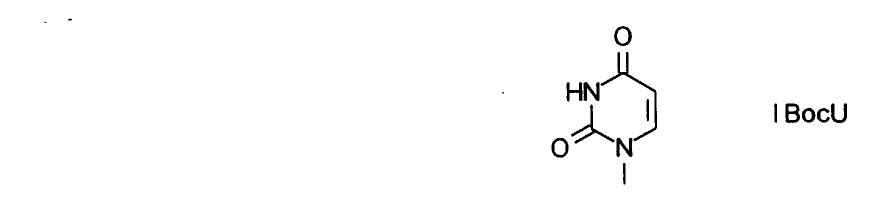

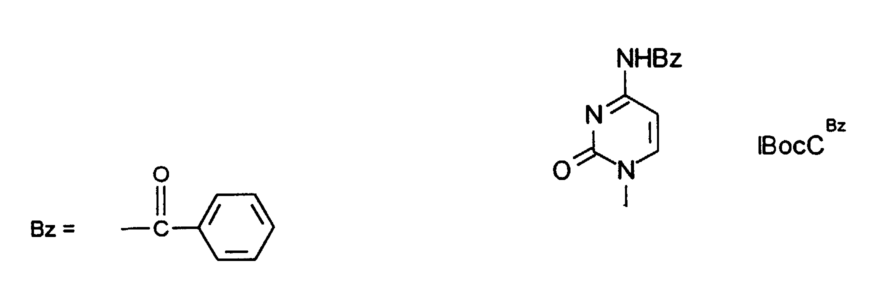

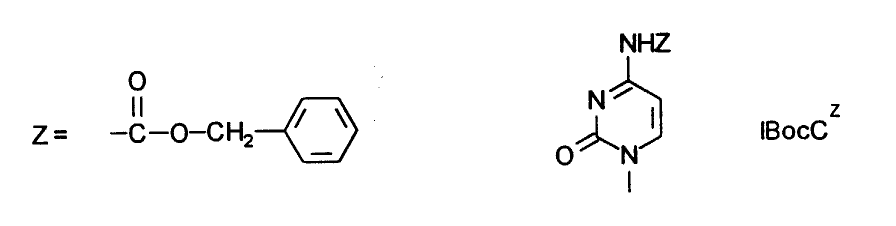

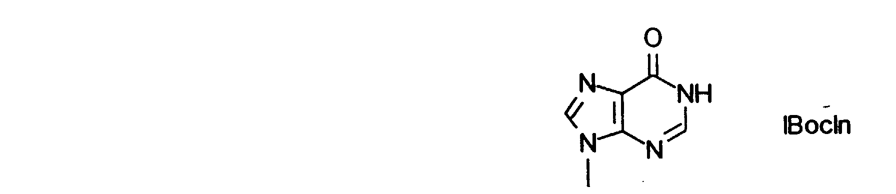

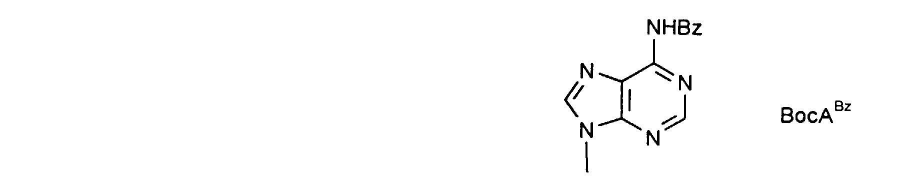

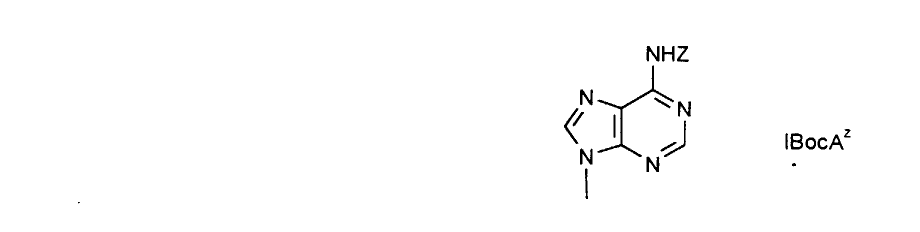

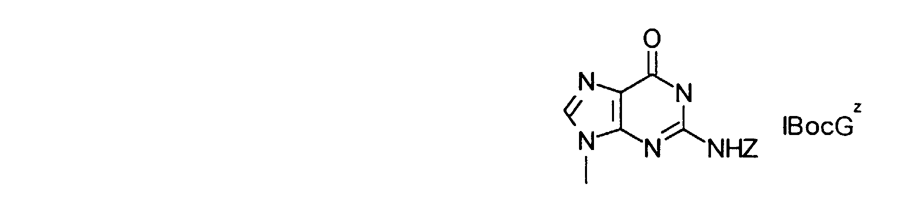

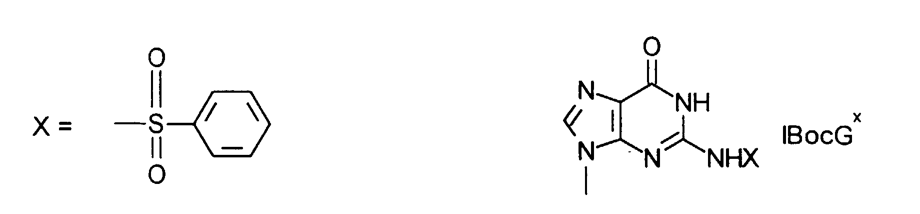

Dies ergibt folgende Syntheseäquivalente:

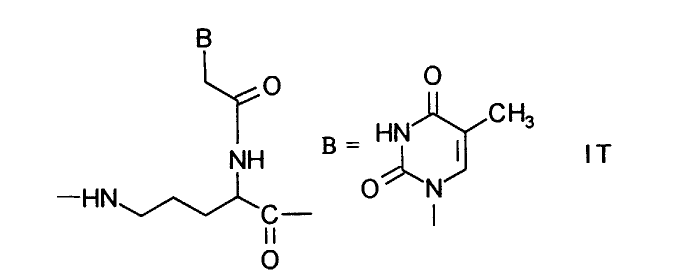

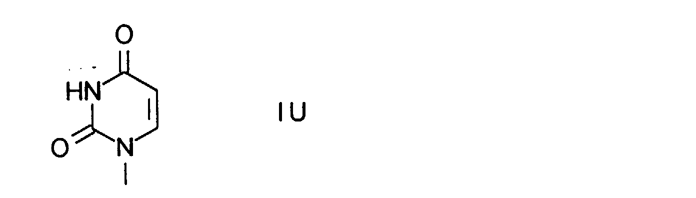

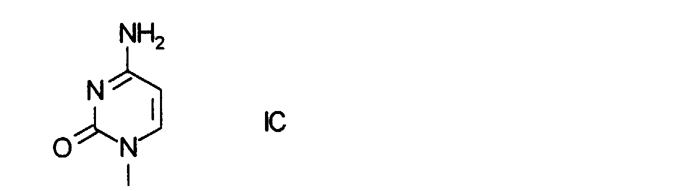

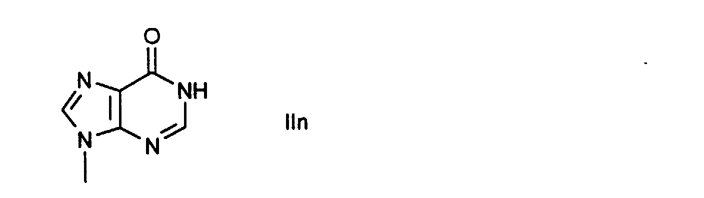

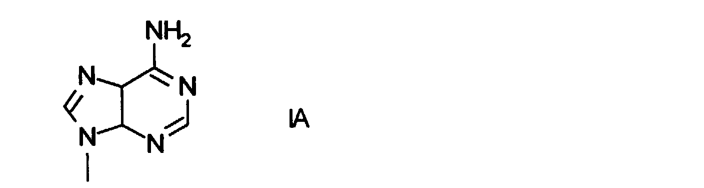

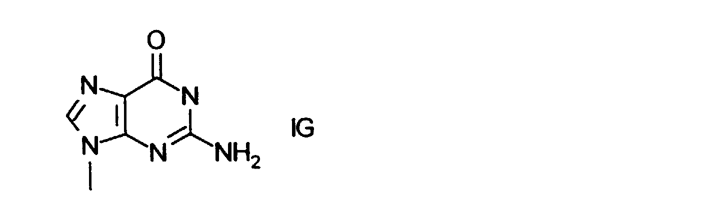

Zur Oligomerisierung wurden vorzugsweise nachfolgend aufgeführte 2-Aminobutyryl-Bausteine eingesetzt. Alternativ zur Boc-Schutzgruppe kann ebenfalls die Fmoc-Schutzgruppe eingesetzt werden:

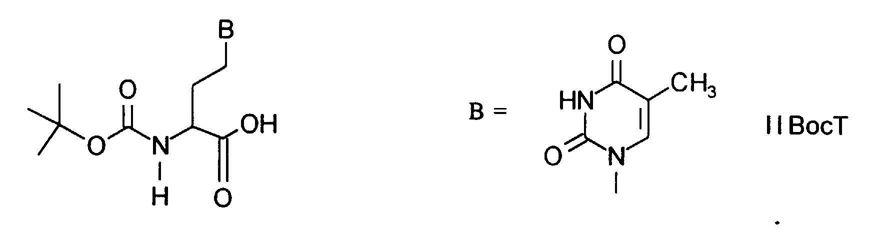

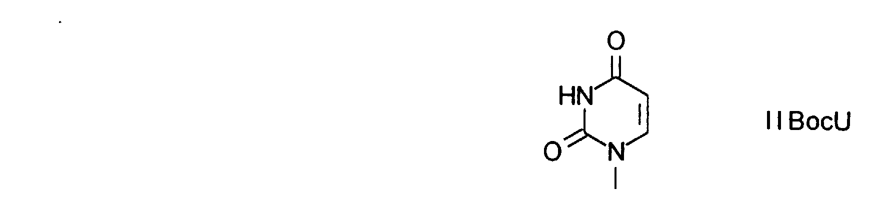

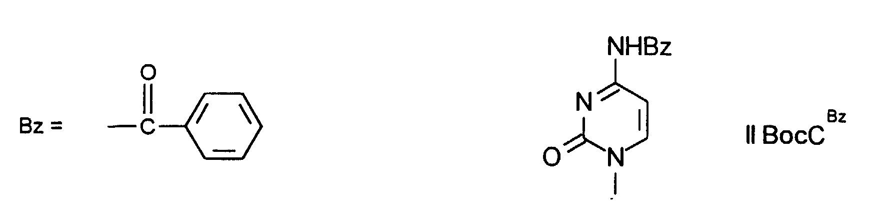

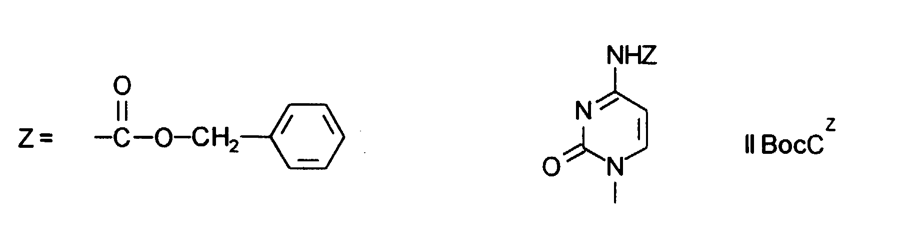

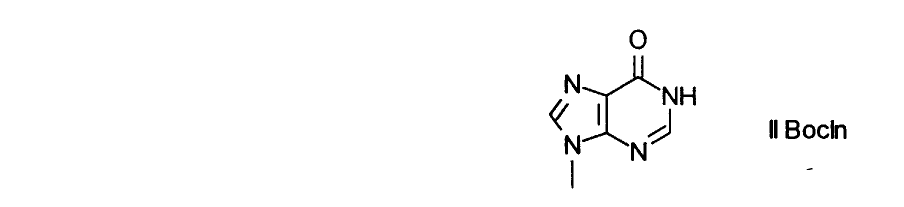

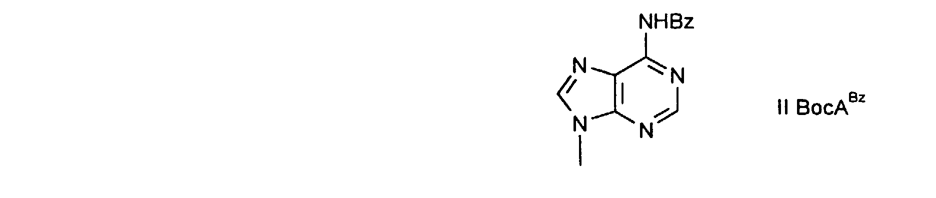

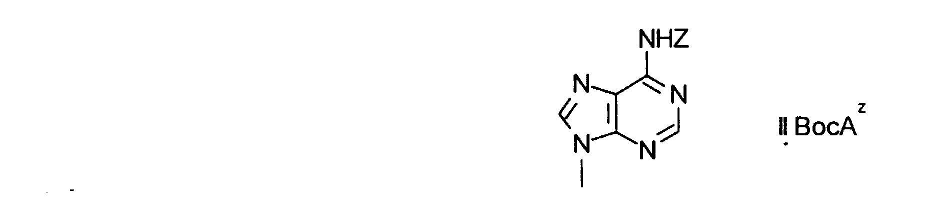

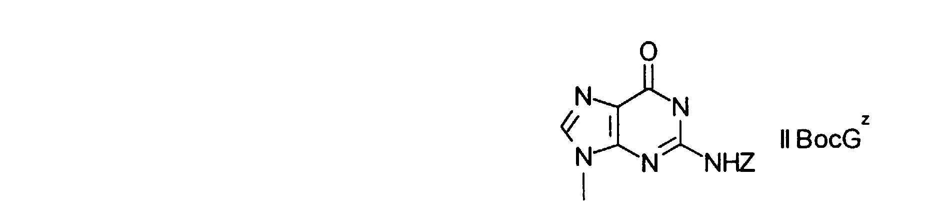

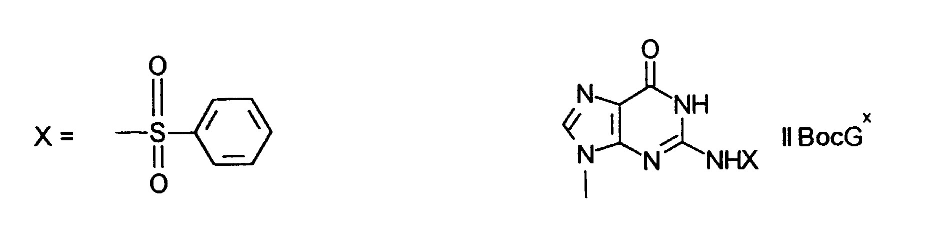

Dies ergibt folgende Syntheseäquivalente:

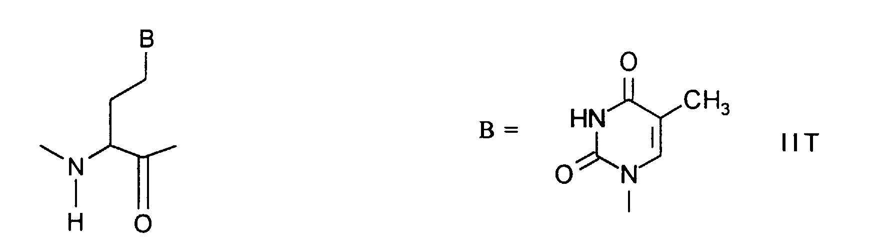

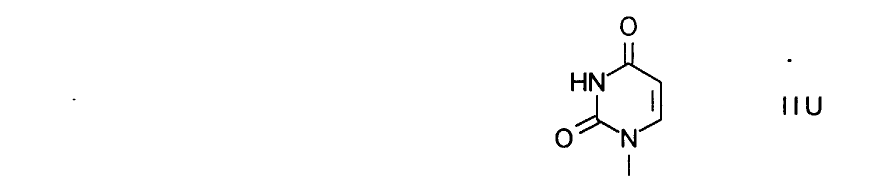

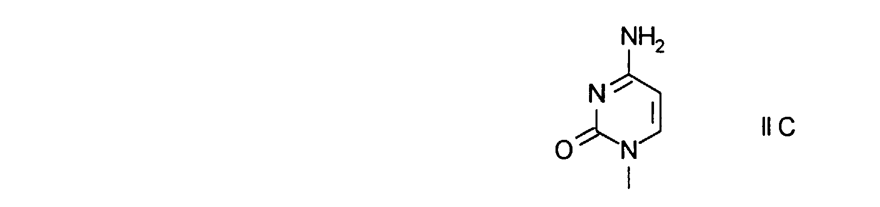

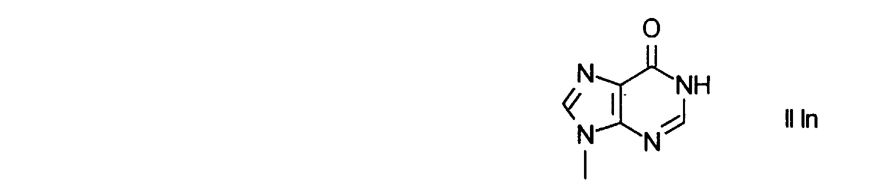

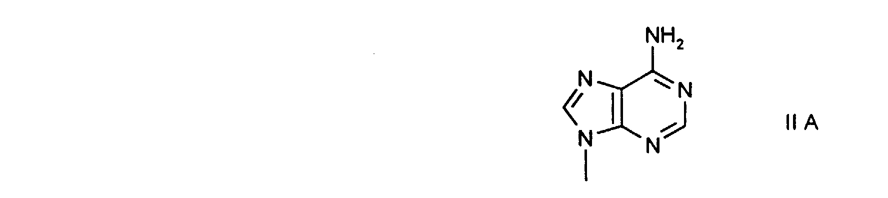

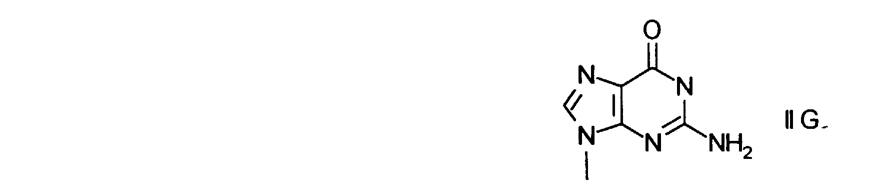

Zur Oligomerisierung wurden vorzugsweise nachfolgend aufgeführte Pyrrolidon-2-carboxyl-Bausteine eingesetzt. Alternativ zur Boc-Schutzgruppe kann ebenfalls die Fmoc-Schutzgruppe eingesetzt werden:

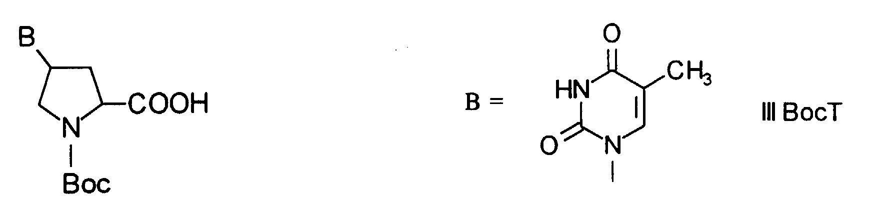

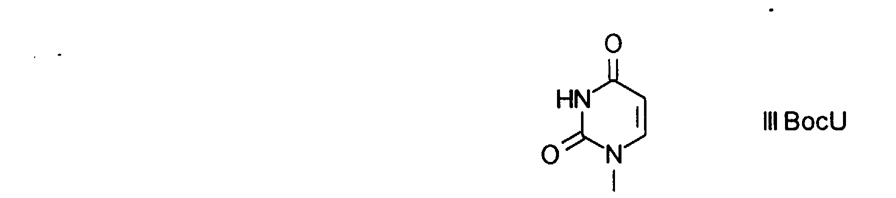

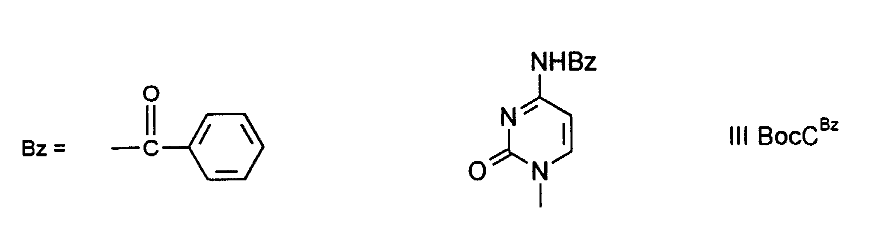

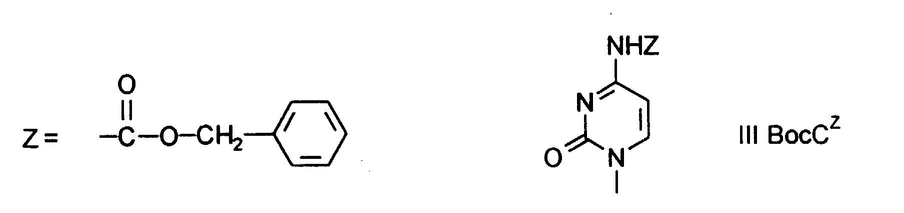

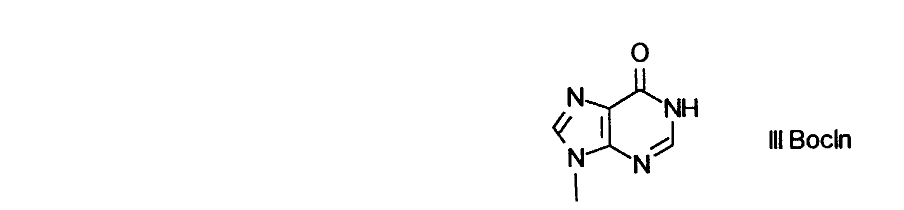

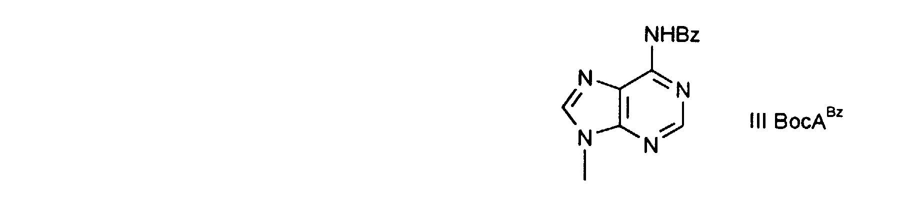

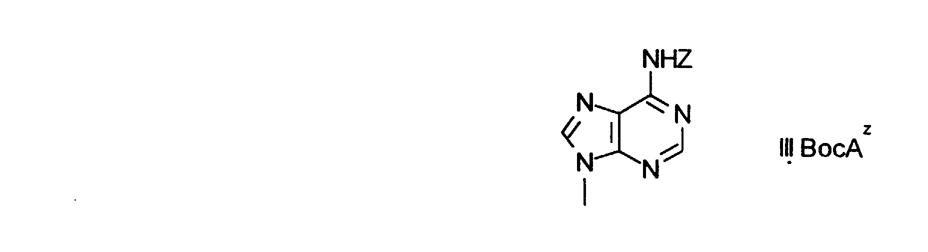

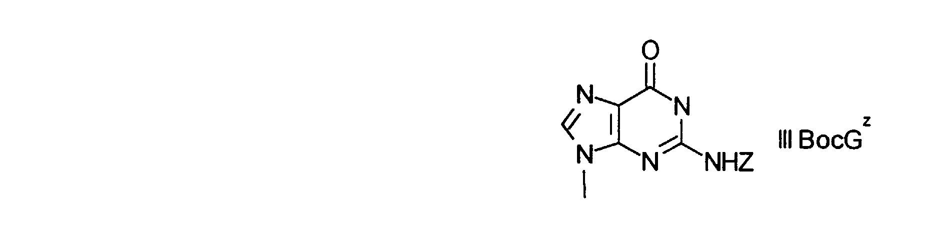

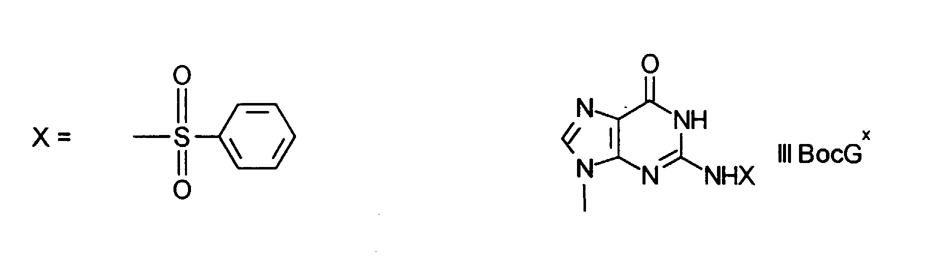

Dies ergibt folgende Syntheseäquivalente:

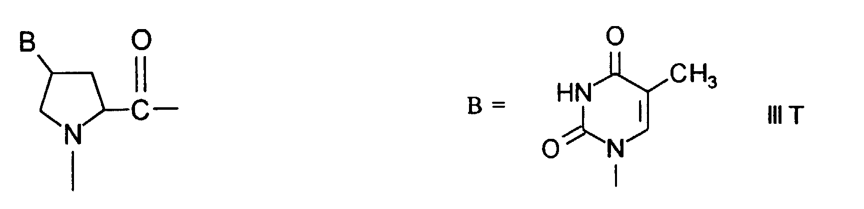

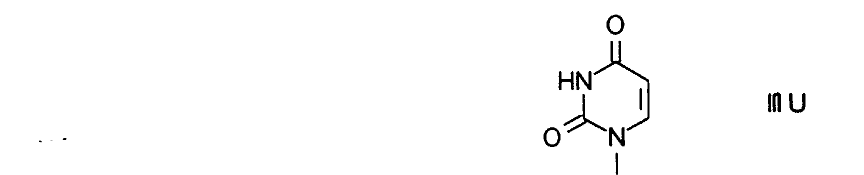

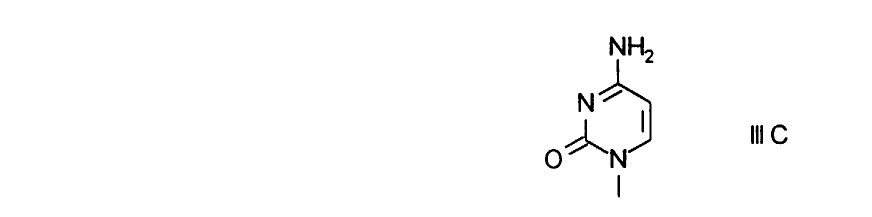

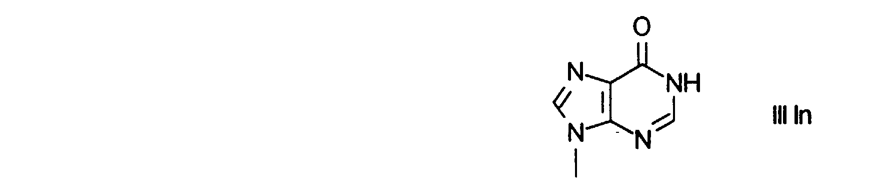

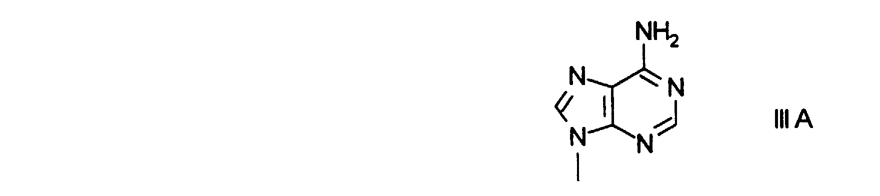

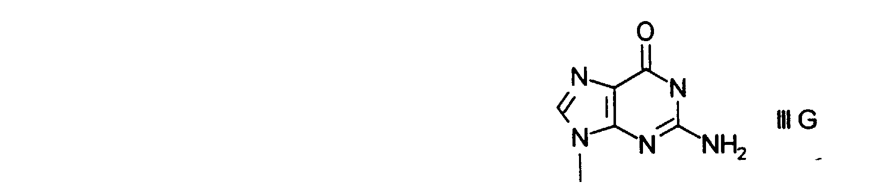

Zur Oligomerisierung wurden ebenfalls nachfolgend aufgeführte dimere Bausteine eingesetzt. Alternativ zur Boc-Schutzgruppe kann ebenfalls die Fmoc-Schutzgruppe eingesetzt werden:

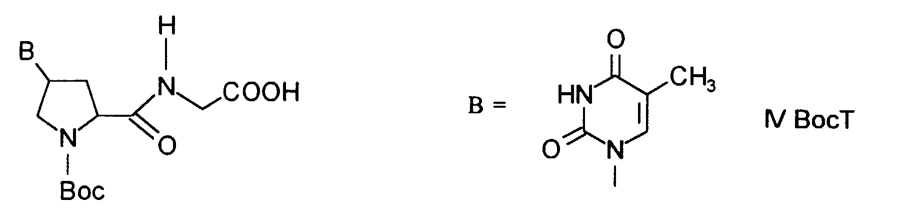

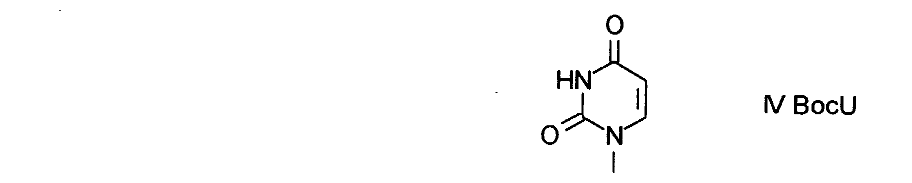

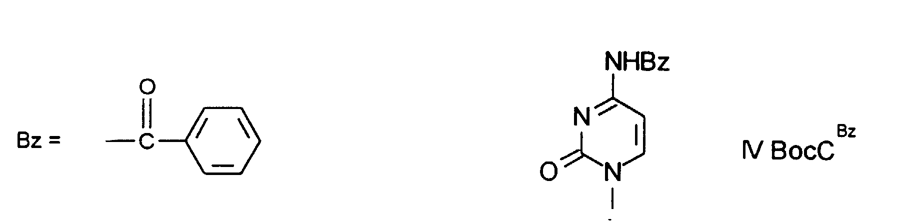

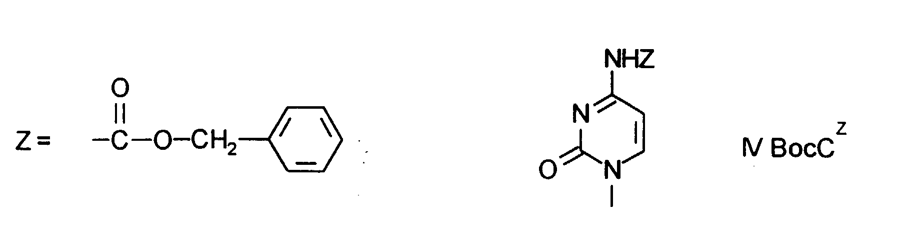

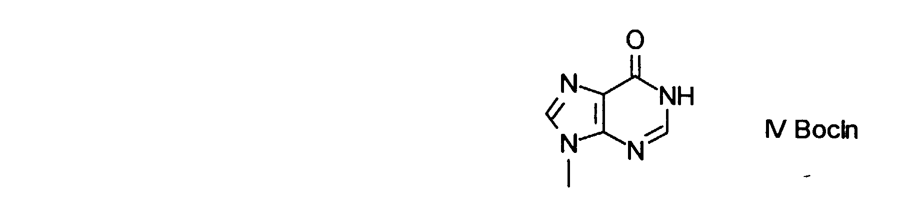

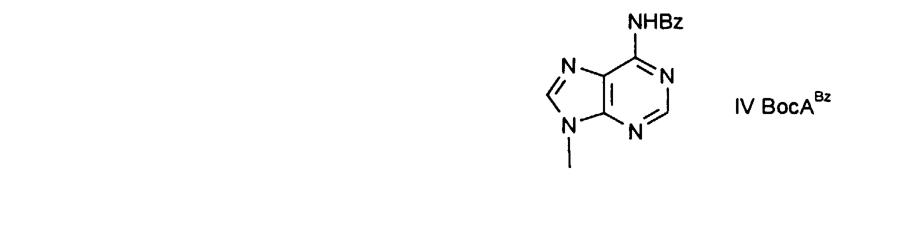

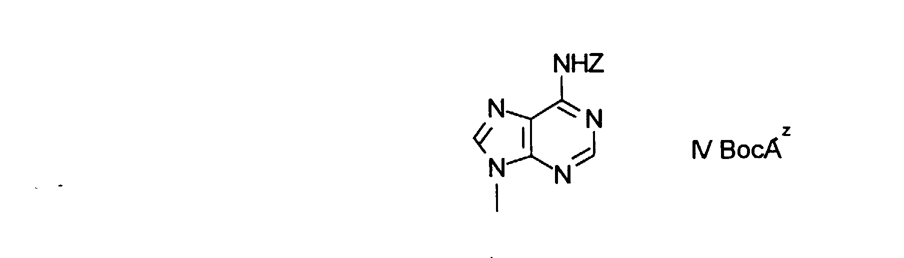

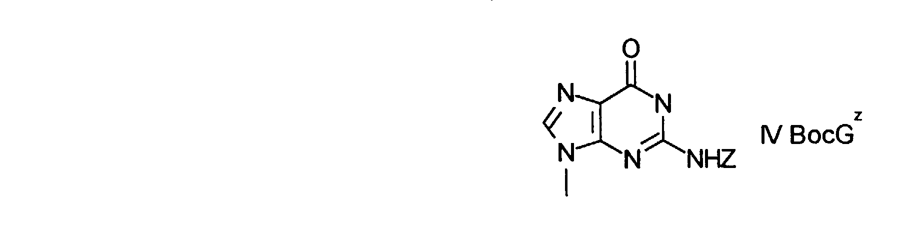

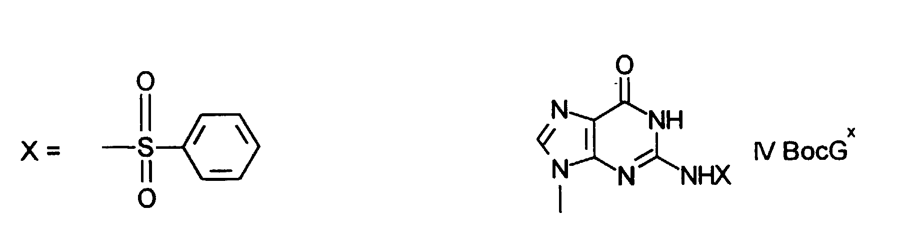

Dies ergibt folgende Syntheseäquivalente:

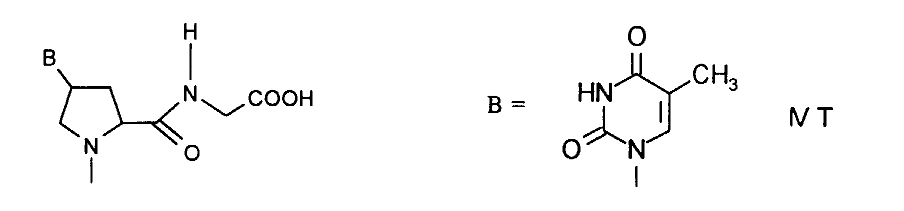

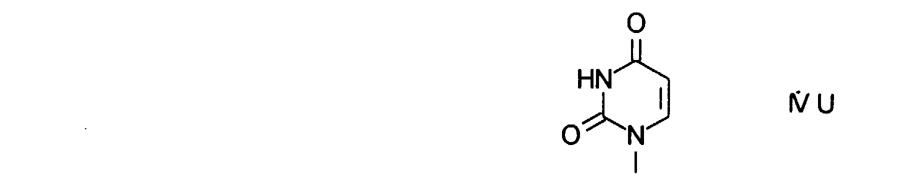

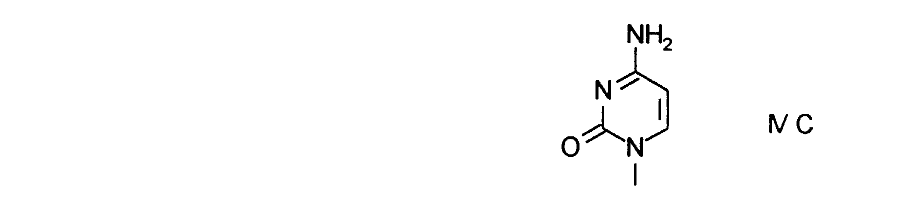

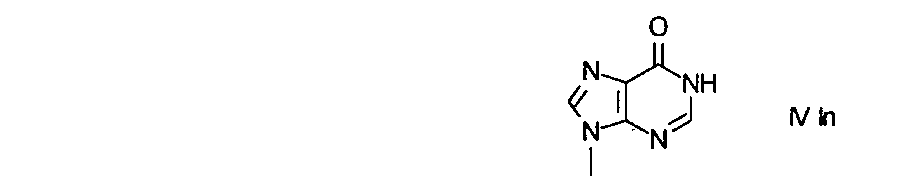

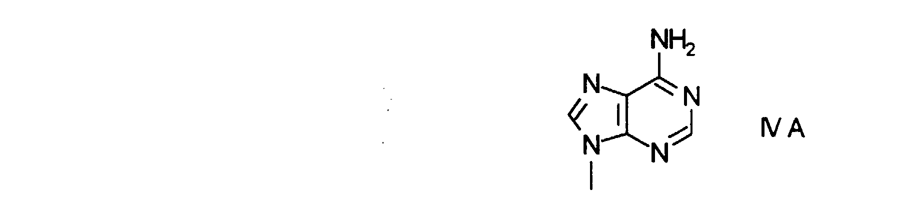

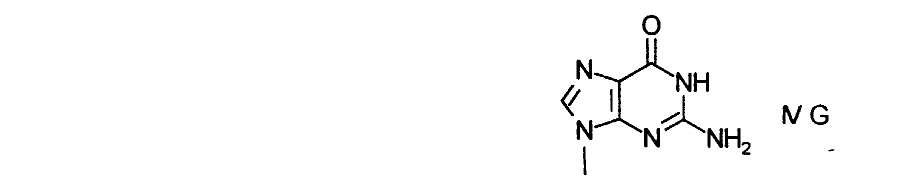

Im Fall 2-Aminobutyryl-Typs wurden ebenfalls dimere Bausteine zur Oligomerisierung eingesetzt:

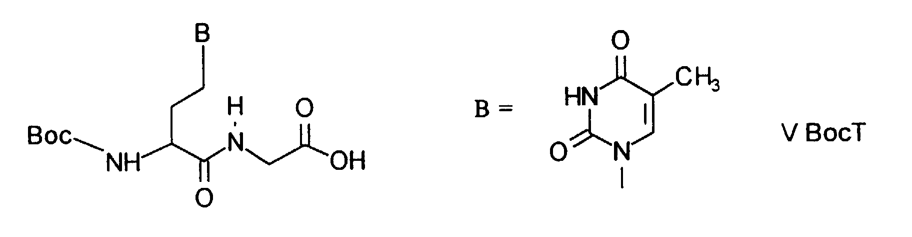

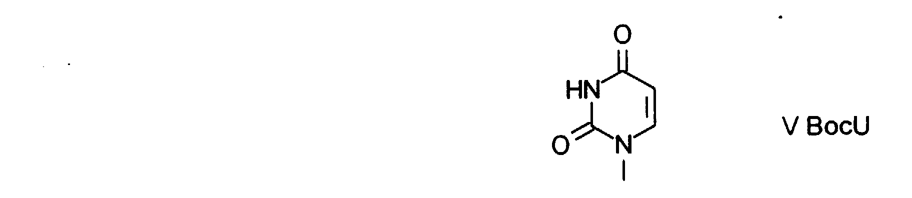

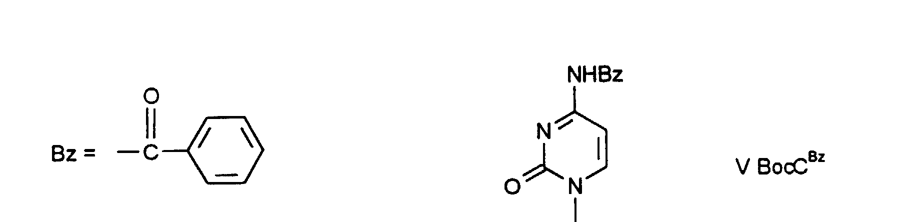

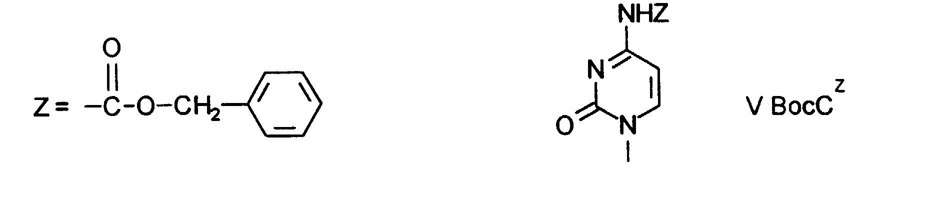

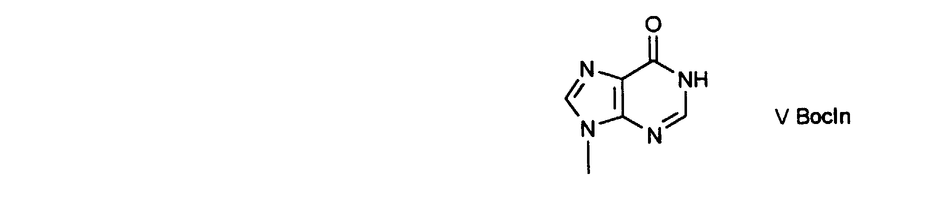

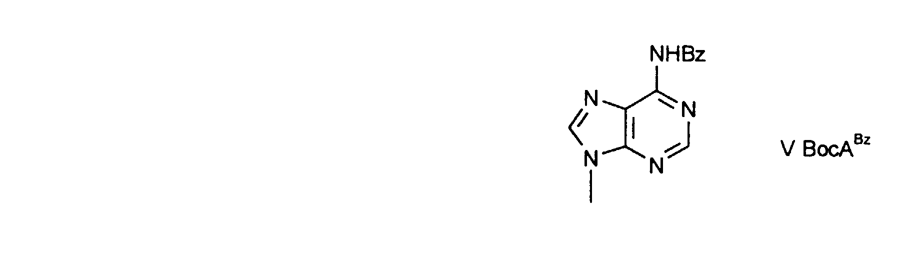

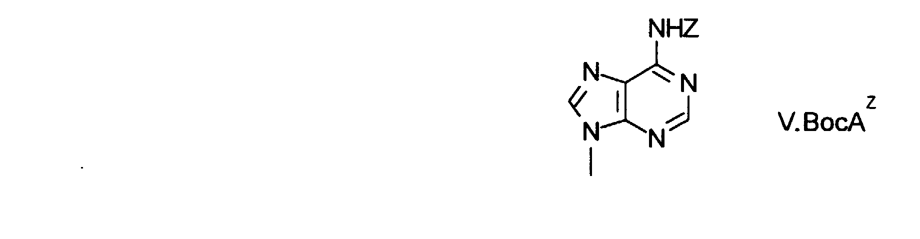

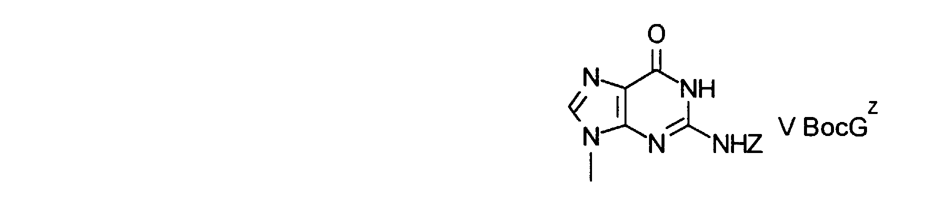

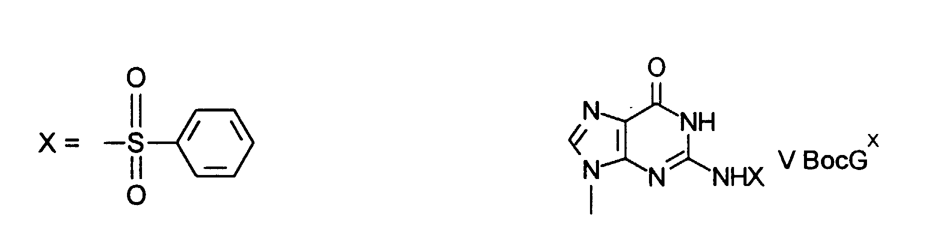

Das ergibt folgende 2-Aminobutyrylglycin-Syntheseäquivalente:

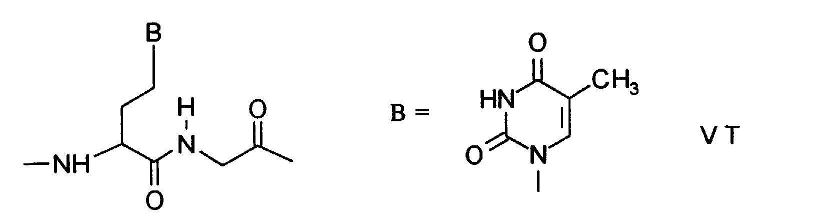

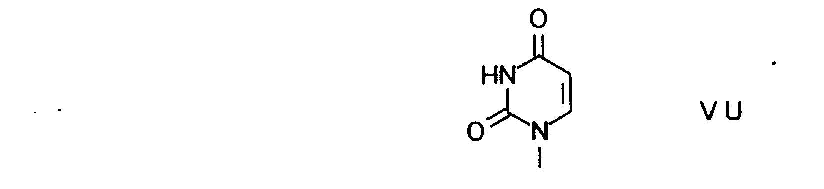

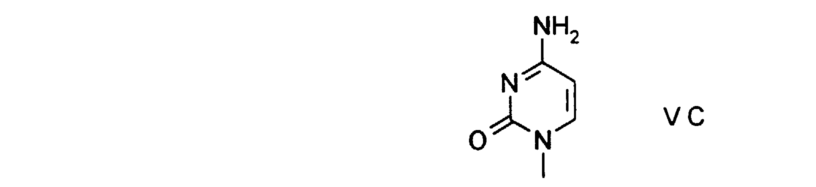

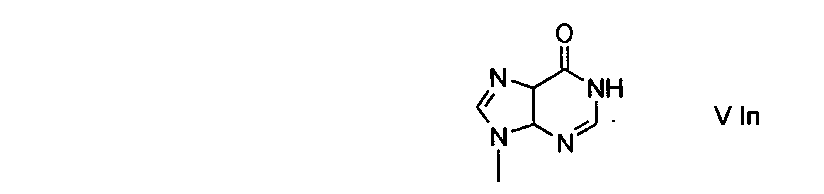

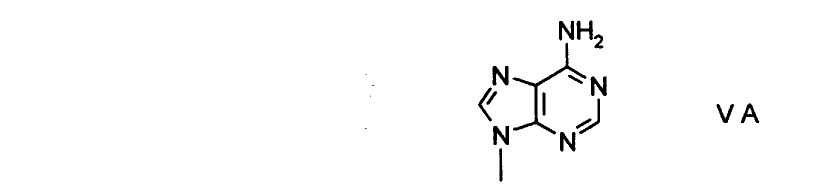

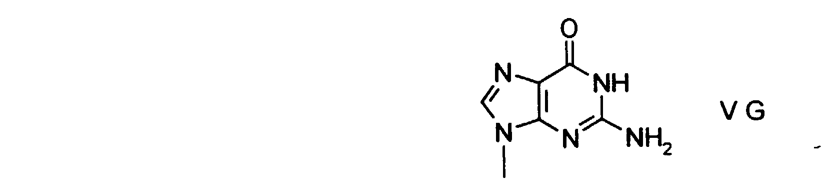

### Beispiele

### Monomere

### Beispiel 1

### α-N-Benzyloxycarbonyl-δ-N-tert.-butyloxycarbonyl-L-ornithinmethylester (2)

Eine Lösung von α-N-Benzyloxycarbonyl-δ-N-tert.-butyloxycarbonyl-L-ornithin (10,0 g; 27 mmol) in wasserfreiem Methanol (135 ml) wird mit Cäsiumcarbonat auf pH = 9,0 bis 9,5 eingestellt und 30 Minuten bei Raumtemperatur nachgerührt. Anschließend wird eingeengt und 30 Minuten am Hochvakuum getrocknet. Der Rückstand wird in wasserfreiem N,N-Dimethylformamid (135 ml) aufgenommen und mit Jodmethan (4,37 g; 30 mmol) versetzt. Man beläßt 30 Minuten bei Raumtemperatur, engt in vacuo ein und destilliert mehrfach mit Toluol nach. Das entstandene Öl wird in Chloroform (270 ml) aufgenommen und mit Wasser ausgeschüttelt. Die organische Phase wird getrocknet und eingeengt.
Ausbeute: 10,4 g (quantitativ).
Rf: 0,70 Laufmittel: Toluol/EtOH 1:3

### Beispiel 2

### δ-N-tert.-butyloxycarbonyl-L-ornithinmethylester (3)

Das Produkt aus Beispiel 1 (12,6 g; 33 mmol) wird in Methanol (330 ml) bei Raumtemperatur und Normaldruck 46 h über Palladium auf Bariumsulfat (5 %; 9,96 g) hydriert. Anschließend wird vom Katalysator abgesaugt (Celite) und eingeengt.
Ausbeute: 12,6 g (quantitativ).
Rf: 0,58 Laufmittel: Toluol/EtOH 4:1

### Beispiel 3

### δ-N-tert.-butyloxycarbonyl-α-N-(thymin-1-yl)acetyl-L-ornithin

Einer Lösung von 1-Carboxymethylthymin (4,22 g; 23 mmol) in wasserfreiem N,N-Dimethylformamid (60 ml) wird eine Lösung von Pentafluorphenol (4,22 g; 23 mmol) in wasserfreiem N,N-Dimethylformamid (20 ml) hinzugefügt. Anschließend wird bei 0°C N,N'-Dicyclohexylcarbodiimid (4,74 g; 23 mmol), gelöst in wasserfreiem N,N-Dimethylformamid (20 ml), langsam zugetropft. Die Reaktionslösung wird 3 h nachgerührt, wobei man auf Raumtemperatur aulwärmen laßt. Der ausgefallene Feststoff wird abfiltriert, mit N,N-Dimethylformamid nachgewaschen und zur Lösung δ-N-tert.-butyloxycarbonyl-L-ornithin (4,45 g; 19 mmol), gelöst in N,N-Dimethylformamid, bei 0°C zugetropft. Man rührt weitere 21 h bei Raumtemperatur, engt in vacuo ein, destilliert mehrfach mit Toluol nach und chromatographiert an Kieselgel mit Chloroform/Methanol (2:1) als Laufmittel.
Ausbeute: 7,70 g (quantitativ)
Rf: 0,78 Laufmittel: CHCl₃/MeOH 1:1

### Beispiel 4

### N⁴-Benzoyl-1-tert.-butyloxycarbonylmethylcytosin

Zu einer Suspension von N⁴-Benzoylcytosin (21,5 g; 0,1 mol) und Kaliumcarbonat (13,8 g; 0,1 mol) in wasserfreiem N,N-Dimethylformamid (2,15 1) wird bei Raumtempertur langsam Bromessigsäure-tert.-butylester (24 ml; 0,15 mol) zugetropft. Das heterogene Gemisch wird 20 h heftig bei Raumtemperatur verrührt, anschließend von unlöslichen Edukt abgesaugt, in vacuo eingeengt, mehrfach mit Toluol nachdestilliert, der Rückstand mit Chloroform (1,0 l) aufgenommen, einmal mit Wasser (0,3 l) ausgeschüttelt und zügig die Phasen getrennt. Die organische Phase wird nochmals filtriert und eingeengt.
Ausbeute: 15,23 g (46 %).
Rf: 0,33 Laufmittel Toluol/EtOH 10:1

### Beispiel 5

### N⁴-Benzoyl-1-carboxymethylcytosin (4)

Das Produkt aus Beispiel 4 wird in Trifluoressigsäure (170 ml) gelöst und 1 h 45 min bei Raumtemperatur belassen. Anschließend wird fünfmal mit Toluol codestilliert und das Produkt 24 h im Exsikkator über Phosphorpentoxid/Kaliumhydroxid getrocknet.
Ausbeute: 11,8 g (93 %).
Rf: 0,1 Laufmittel: Toluol/EtOH 1:1

### Beispiel 6

### α-N-(N⁴-Benzoylcytosin-1-yl)acetyl-δ-N-tert.-butyloxycarbonyl-L-ornithinmethylester (5)

N⁴-Benzoyl-1-carboxymethylcytosin (15,76 g; 58 mmol) und δ-tert.-Butyloxycarbonyl-L-ornithinmethylester (9,61 g; 39 mmol) werden in wasserfreiem N,N-Dimethylformamid (520 ml) suspendiert und mit N,N'-Dicyclohexylcarbodiimid (11,92 g; 58 mmol) versetzt. Das Gemisch wird 1 h bei Raumtemperatur gerührt, danach in vacuo eingeengt und mehrfach mit Toluol nachdestilliert. Das Rohprodukt wird chromatographisch an Kieselgel gereinigt (Laufmittel: Toluol/Ethanol 8:1).
Ausbeute: 5,21 g (27 %).
Rf: 0,47 Laufmittel: Toluol/EtOH 4:1

### Beispiel 7

### α-N-(N⁴-Benzoylcytosin-1-yl)acetyl-δ-N-tert.-butyloxycarbonyl-L-ornithin (6)

Eine Lösung des Produktes aus Beispiel 6 (4,80 g; 8,7 mmol) in Dioxan/Wasser (5:1; 70 ml) wird mit Lithiumhydroxid-Hydrat (440 mg; 10,5 mmol) versetzt und 1,5 h bei Raumtemperatur belassen. Nachfolgend wird mit 0,5 N Salzsäure neutralisiert und eingeengt. Das Produkt kristallisiert aus Methanol.
Ausbeute: 2,09 g (45 %).

### Beispiel 8

### N-Boc-L-Homoserin-tert.-butylester (8)

N-Boc-Asparaginsäure-tert.-butylester (15,4 g; 53,1 mmol) wird in abs. THF vorgelegt und auf 0°C gekühlt. Bei dieser Temperatur wird 1 eq. Triethylamin gefolgt von 1 eq. Chlorameisensäureethylester zugegeben. Das Reaktionsgemisch wird 15 min bei 0°C und 5 min bei Raumtemperatur gerührt. Anschließend wird vom ausgefallenen Triethylaminhydrochlorid abgesaugt und das Filtrat direkt weiter umgesetzt.

Das Filtrat wird zu einer Suspension von NaBH₄ in THF, die auf 0°C vorgekühlt ist, getropft und bei dieser Temperatur 15 bis 20 min gerührt. Danach wird das Eisbad entfernt und für weiter 30 bis 40 min bei Raumtemperatur gerührt. Nach beendeter Reaktion wird durch Zugabe an 1N HCl gequenched. Anschließend werden die Phasen getrennt und die organische Phase nacheinander je 3 mal mit 100 ml 1N HCl, ges. NaHCO₃- und ges. NaCl-Lösung ausgeschüttelt. Die organische Phase wird abgetrennt, über Na₂SO₄ getrocknet und am Rotationsverdampfer zur Trockne eingeengt. Zurück bleibt ein klares Öl.
Ausbeute: 12 g (82,2 %).
Rf: 0,66 Laufmittel: CH₂Cl₂/MeOH 9:1

### Beispiel 9

### N-Boc-γ-methansulfonyloxy-L-homoserin-tert.-butylester (9)

N-Boc-L-Homoserin-tert.-butylester (13 g; 47,2 mmol) wird in abs. Pyridin gelöst und die Lösung auf 0°C abgekühlt. Bei dieser Temperatur werden 1,1 eq. Methansulfonsäurechlorid zugetropft. Anschließend läßt man 5 h bei Raumtemperatur rühren. Nach beendeter Reaktion wird das Pyridin abdestilliert. Der Rückstand wird mit Essigsäureethylester überschichtet und nacheinander je 3 mal mit 100 ml 1N HCl, ges. NaHCO₃- und ges. NaCl-Lösung ausgeschüttelt. Die organische Phase wird abgetrennt, über Na₂SO₄ getrocknet und zur Trockne eingedampft. Der Rückstand wird in wenig Essigsäureethylester aufgenommen und das Produkt durch Zugabe an n-Hexan ausgefällt.
Ausbeute: 12,9 g (77 %).
Rf: 0,76 Laufmittel: Essigsäureethylester/n-Hexan 2:1
Smp: 93°C

### Beispiel 10a

### N-Boc-4-(N³-benzoyl-thymin-1-yl)-2-L-aminobuttersäure-tert.-butylester (10)

N³-Benzoyl-thymin (2 eq.) wird in abs. DMF vorgelegt und mit 2 eq. K₂CO₃ versetzt. Es wird 5 min bei Raumtemperatur gerührt. Danach wird das Mesylat (9) (1,18 g; 3,36 mmol) gelöst in DMF zugetropft. Nach beendeter Zugabe wird auf 60°C aufgeheizt und bei dieser Temperatur 4 h gerührt. Nach beendeter Reaktion wird das DMF abdestilliert, der Rückstand mit Essigsäureethylester überschichtet und nacheinander je 3 mal mit 20 ml 1N HCl, ges. NaHCO₃- und ges. NaCl-Lösung ausgeschüttelt. Die Essigsäureethylesterphase wird über Na₂SO₄ getrocknet und dann zur Trockne einrotiert. Das erhaltene Rohprodukt wird schließlich an Kieselgel mit Essigsäureethylester/n-Hexan 1:1 als Eluent chromatographiert.
Ausbeute: 897 mg (54,8 %) weißer Schaum.
Rf: 0,72 Laufmittel: Essigsäureethylester/n-Hexan 2:1

### Beispiel 10b

### N-Boc-4-(N³-benzoyl-thymin-1-yl)-2-L-aminobuttersäure-tert.-butylester (10)

2,5 eq. Triphenylphosphin und 2,5 eq. DEAD werden in abs. THF gelöst und 5 min bei Raumtemperatur gerührt. Nach dieser Zeit wird das N³-Benzoyl-thymin (2 eq.) zugegeben und erneut 5 min bei Raumtemperatur gerührt. Anschließend tropft man das in abs. DMF gelöste (8) (3,02 g; 10,98 mmol) zu und läßt über Nacht bei Raumtemperatur rühren. Nach beendeter Reaktion wird das DMF abdestilliert und das Rohprodukt an Kieselgel mit Essigsäureethylester/n-Hexan 1:1 als Laufmittel chromatographiert.
Ausbeute: 3,19 g (58,5 %) weißer Schaum.
Rf: 0,72 Laufmittel: Essigsäureethylester/n-Hexan 2:1

### Beispiel 11a

### N-Boc-4-(thymin-1-yl)-2-L-aminobuttersäure-tert.-butylester (11)

Verbindung (10) (3,19 g; 5,96 mmol) wird mit NH₃ in Methanol über Nacht bei Raumtemperatur gerührt. Danach wird das Lösungsmittel abdestilliert. Das erhaltene Rohprodukt wird ohne weitere Reinigung für die Abspaltung der Boc- und der OtBu-Schutzgruppe eingesetzt.

### Beispiel 11b

### N-Boc-4-(thymin-1-yl)-2-L-aminobuttersäure-tert.-butylester (11)

2 eq. Thymin und 2 eq. K₂CO₃ werden in abs. DMF bei Raumtemperatur 5 min gerührt. Anschließend wird Verbindung (9) (1,18 g; 3,36 mmol) gelöst in abs. DMF zugetropft und 4 h bei 60°C gerührt. Nach beendeter Reaktion wird das DMF abdestilliert und das Rohprodukt mit Essigsäureethylester überschichtet. Die organische Phase wird nacheinander je 3 mal mit 20 ml 1N HCl, ges. NaHCO₃- und ges. NaCl-Lösung ausgeschüttelt. Die organische Phase wird abgetrennt, mit Na₂SO₄ getrocknet und zur Trockne eingedampft. Schließlich wird an Kieselgel mit Essigsäureethylester/n-Hexan 2:1 als Eluent chromatographiert.
Ausbeute: 650 mg (50 %).
Rf: 0,53 Laufmittel: Essigsäureethylester/n-Hexan 2:1
Smp: 184 bis 187°C

### Beispiel 12

### 4-(Thymin-1-yl)-2-L-aminobuttersäure* TFA

Verbindung (11) wird in TFA (2 ml pro mmol) 2 h im Ultraschallbad behandelt. Die erhaltene Lösung wird anschließend in abs. Ether pipettiert, wobei das TFA-Salz ausfällt. Das erhaltene TFA-Salz wird abfiltriert und im Ölpumpenvakuum über KOH getrocknet.
Ausbeute: quantitativ.

### Beispiel 13

### 4-(Thymin-1-yl)-2-L-aminobuttersäure* HBr

Verbindung (11) wird in HBr/Essigsäure (5 ml pro mmol) gelöst und 30 min bei Raumtemperatur gerührt. Anschließend wird die Lösung in abs. Ether pipettiert, wobei das HBr-Salz ausfällt. Das erhaltene HBr-Salz wird abfiltriert und im Ölpumpenvakuum über KOH getrocknet.
Ausbeute quantitativ.
Smp: > 200°C Zersetzung

### Beispiel 14

### N-Boc-4-(thymin-1-yl)-2-L-aminobuttersäure (12)

Die Einführung der Boc-Schutzgruppe ausgehend von dem TFA-Salz aus Beispiel 12 erfolgt auf dem gleichen Weg wie für das HBr-Salz aus Beispiel 13. Exemplarisch wird die Schutzgruppeneinführung ausgehend von dem HBr-Salz beschrieben.

Das HBr-Salz (10,7 g; 34,7 mmol) wird in THF vorgelegt. Der pH der Lösung wird durch Zugabe an Triethylamin auf pH 8 eingestellt. Danach tropft man 1,1 eq. Di-tert.-butyl-dicarbonat gelöst in THF zu und läßt bei Raumtemperatur über Nacht rühren. Während der Reaktion wird darauf geachtet, daß der pH-Wert nicht unter den Wert 8 fällt. Nach beendeter Reaktion wird das THF abdestilliert, der Rückstand mit Essigsäureethylester überschichtet und der pH durch Zugabe an 1N HCl auf 1 bis 2 eingestellt. Danach werden die Phasen getrennt, die organische Phase über Na₂SO₄ getrocknet und zur Trockne eingedampft. Das erhaltene Rohprodukt wird an Kieselgel mit CH₂Cl₂/MeOH/HOAc 90:10:1 chromatographiert.
Ausbeute: 6 g (53 %).
Rf: 0,53 Laufmittel CH₂Cl₂/MeOH/HOAc 80:20:1
Smp: 124°C

### Beispiel 15

### N-Fmoc-4-(thymin-1-yl)-2-L-aminobuttersäure

Das TFA-Salz aus Beispiel 12 (3,4 g; 9,66 mmol) wird in einem Lösungsmittelgemisch aus Aceton/Wasser (30 ml : 30 ml) gelöst und mit 2 eq. NaHCO₃ versetzt. Dann gibt man 1 eq. 9-Fluorenylmethyl-succinimidylcarbonat zu und läßt bei Raumtemperatur über Nacht rühren. Nach beendeter Reaktion wird das Aceton abdestilliert, die wäßrige Phase mit Chloroform versetzt und anschließend mit 1N HCl auf pH 2 angesäuert. Danach werden die Phasen getrennt. Die Chloroform-Phase wird schließlich 3 mal mit je 50 ml 0,1N HCl, ges. NaHCO₃- und ges. NaCl-Lösung ausgeschüttelt. Die Phasen werden getrennt. Die organische Phase wird über Na₂SO₄ getrocknet und dann zur Trockne eingedampft. Das Produkt wird an Kieselgel mit CH₂Cl₂/MeOH/HOAc 90:10:1 als Laufmittel gesäult.
Ausbeute: 2,7 g (55,3 %).
Rf: 0,80 Laufmittel: CH₂Cl₂/MeOH/HOAc 80:20:1

### Beispiel 16

### N-Boc-4-(N⁴-benzoyl-cytosin-1-yl)-2-L-aminobuttersäure-tert.-butylester

2 eq. N⁴-Benzoyl-cytosin und 2 eq. K₂CO₃ werden in abs. DMF 5 min bei Raumtemperatur gerührt. Danach wird Verbindung (9) (13,35 g; 37,8 mmol) gelöst in DMF zugetropft. Die Lösung wird auf 60°C erhitzt und bei dieser Temperatur 4 h gerührt. Nach beendeter Reaktion wird das DMF abdestilliert, der Rückstand mit Essigsäureethylester überschichtet und nacheinander je 3 mal mit 100 ml 1N HCl, ges. NaHCO₃- und ges. NaCl-Lösung ausgeschüttelt. Die organische Phase wird schließlich über Na₂SO₄ getrocknet und zur Trockne eingedampft. Das erhaltene Rohprodukt wird an Kieselgel mit Essigsäureethylester/n-Hexan 1:1 chromatographiert.
Ausbeute: 6,1 g (35 %).
Rf: 0,55 Laufmittel: CH₂Cl₂/MeOH 9:1

### Beispiel 17

### 4-(N⁴-Benzoyl-Cytosin-1-yl)-2-L-aminobuttersäure* TFA

N-Boc-4-(N⁴-benzoyl-cytosin-1-yl)-2-L-aminobutyryl-tert.-butylester wird in TFA (2 ml pro mmol) 2 h im Ultraschallbad behandelt. Die erhaltene Lösung wird anschließend in abs. Ether pipettiert, wobei das TFA-Salz ausfällt. Das erhaltene TFA-Salz wird abfiltriert und im Ölpumpenvakuum über KOH getrocknet. Ausbeute: quantitativ.

### Beispiel 18

### N-Boc-4-(N⁴-benzoyl-cytosin-1-yl)-2-L-aminobuttersäure

Das TFA-Salz (5,5 g; 10,43 mmol) aus Beispiel 17 wird in einem Lösungsmittelgemisch aus Dioxan/Wasser (25 ml : 15 ml) gelöst und mit 3 eq. Na₂CO₃ versetzt. Die Lösung wird 5 min bei Raumtemperatur gerührt. Anschließend werden 1,3 eq. Di-tert.-butyl-dicarbonat gelöst in 15 ml Dioxan zugetropft und über Nacht bei Raumtemperatur gerührt. Nach beendeter Reaktion wird das Dioxan abdestilliert, die wäßrige Phase mit Essigsäureethylester überschichtet und mit 1N HCl auf pH 2 angesäuert. Danach werden die Phasen getrennt und die organische Phase nacheinander 3 mal mit je 100 ml 1N HCl, ges. NaHCO₃- und ges. NaCl-Lösung ausgeschüttelt. Die organische Phase wird über Na₂SO₄ getrocknet und dann zur Trockne eingedampft. Das Produkt wird aus Essigsäureethylester/n-Hexan gefällt.
Ausbeute: 3,65 g (83 %).
Rf: 0,50 Laufmittel: CH₂Cl₂/MeOH/HOAc 80:20:1

### Beispiel 19

### N-Boc-4-(N⁴-Z-cytosin-1-yl)-2-L-aminobuttersäure-tert.-butylester

N⁴-Z-Cytosin (2 eq.) wird in abs. DMF vorgelegt und mit 2 eq. K₂CO₃ versetzt. Es wird 5 min bei Raumtemperatur gerührt. Danach wird das Mesylat (9) (11 g; 31,1 mmol) gelöst in DMF zugetropft. Nach beendeter Zugabe wird auf 60°C aufgeheizt und bei dieser Temperatur 4 h gerührt. Nach beendeter Reaktion wird das DMF abdestilliert, der Rückstand mit Essigsäureethylester überschichtet und nacheinander 3 mal mit je 100 ml 1N HCl, ges. NaHCO₃- und ges. NaCl-Lösung ausgeschüttelt. Die organische Phase wird über Na₂SO₄ getrocknet und dann zur Trockne eingedampft. Das erhaltene Rohprodukt wird schließlich an Kieselgel mit Essigsäureethylester/n-Hexan 1:1 als Laufmittel chromatographiert.
Ausbeute: 9 g; (57,6 %).
Rf: 0,21 Laufmittel: Essigsäureethylester/n-Hexan 2:1

### Beispiel 20

### 4-(N⁴-Z-Cytosin-1-yl)-2-L-aminobuttersäure* TFA

N-Boc-4-(N⁴-Z-cytosin-1-yl)-2-L-aminobutyryl-tert.-butylester (9 g; 18,9 mmol) wird in TFA (2 ml pro mmol) 2 h im Ultraschallbad behandelt. Die erhaltene Lösung wird anschließend in abs. Ether pipettiert, wobei das TFA-Salz ausfällt. Das erhaltene TFA-Salz wird abfiltriert und im Ölpumpenvakuum über KOH getrocknet.
Ausbeute: quantitativ.

### Beispiel 21

### N-Boc-4-(N⁴-Z-cytosin-1-yl)-2-L-aminobuttersäure

Das TFA-Salz (6,74 g; 14,6 mmol) aus Beispiel 20 wird in einem Lösungsmittelgemisch aus Dioxan/Wasser (2:1) gelöst und mit 3 eq. Na₂CO₃ versetzt. Die Lösung wird 5 min bei Raumtemperatur gerührt. Anschließend werden 1,3 eq. Ditert.-butyl-dicarbonat gelöst in wenig Dioxan zugetropft und über Nacht bei Raumtemperatur gerührt. Nach beendeter Reaktion wird das Dioxan abdestilliert, die wäßrige Phase mit Essigsäureethylester überschichtet und mit 1N HCl auf pH 2 angesäuert. Danach werden die Phasen getrennt und die organische Phase nacheinander 3 mal mit je 100 ml 1N HCl, ges. NaHCO₃- und ges. NaCl-Lösung ausgeschüttelt. Die organische Phase wird über Na₂SO₄ getrocknet und dann zur Trockne eingedampft. Das Produkt wird aus Essigsäureethylester/n-Hexan gefällt.
Ausbeute: 4 g; (61,4 %).
Rf: 0,85 Laufmittel: CH₂Cl₂/MeOH/HOAc 80:20:1
Smp.: 190°C

### Beispiel 22

### 2S,4S-N-Boc-4-(N³-benzoyl-thymin-1-yl)-pyrrolidin-2-carbonsäure-methylester (16)

2,5 eq. Triphenylphosphin und 2,5 eq. DEAD werden in abs. THF gelöst und 5 min bei Raumtemperatur gerührt. Nach dieser Zeit wird das N³-Benzoyl-thymin (2 eq.) zugegeben und erneut 5 min bei Raumtemperatur gerührt. Anschließend tropft man den in abs. DMF gelösten 2S,4R-N-Boc-hydroxyprolin-methylester (1,23 g; 5 mmol) zu und läßt über Nacht bei Raumtemperatur rühren. Nach beendeter Reaktion wird das DMF abdestilliert und das Rohprodukt an Kieselgel mit Essigsäureethylester/n-Hexan 1:1 als Eluent chromatographiert.
Ausbeute: 1,2 g (52,5 %).
Rf: 0,40 Laufmittel: Essigsäureethylester/n-Hexan 2:1

### Beispiel 23

### 2S,4S-N-Boc-4-(N³-benzoyl-thymin-1-yl)-pyrrolidin-2-carbonsäure-benzylester (15)

2,5 eq. Triphenylphosphin und 2,5 eq. DEAD werden in abs. THF gelöst und 5 min bei Raumtemperatur gerührt. Nach dieser Zeit wird das N³-Benzoyl-thymin (2 eq.) zugegeben und erneut 5 min bei Raumtemperatur gerührt. Anschließend tropft man den in abs. DMF gelösten 2S,4R-N-Boc-hydroxyprolin-benzylester (11 g; 34,3 mmol) zu und läßt über Nacht bei Raumtemperatur rühren. Nach beendeter Reaktion wird das DMF abdestilliert und das Rohprodukt an Kieselgel mit Essigsäureethylester/n-Hexan 1:1 als Eluent chromatographiert.
Ausbeute: 10,86 g (58,3 %)
Rf: 0,68 Laufmittel Essigsäureethylester/n-Hexan 2:1

### Beispiel 24a

### 2S,4S-N-Boc-4-(N³-benzoyl-thymin-1-yl)-pyrrolidin-2-carbonsäure

2S,4S-N-Boc-4-(N³-benzoyl-thymin-1-yl)-pyrrolidin-2-carboxy-methylester (5,8 g; 12,7 mmol) wird in Isopropanol gelöst und mit 1,1 eq. 1N NaOH versetzt. Anschließend wird bei Raumtemperatur gerührt. Nach beendeter Reaktion (DC-Kontrolle, Laufmittel CH₂Cl₂/MeOH/HOAc 90:10:1) wird das Isopropanol abdestilliert, der Rückstand mit Essigsäureethylester überschichtet und mit 1N HCl auf pH 1 angesäuert. Danach werden die Phasen getrennt, die organische Phase wird über Na₂SO₄ getrocknet und dann zur Trockne eingedampft. Das auf diese Weise erhaltene Rohprodukt wird ohne weitere Aufreinigung weiter umgesetzt (siehe Beispiel 25).
Rf: 0,60 Laufmittel: CH₂Cl₂/MeOH 9:1

### Beispiel 24b

### 2S,4S-N-Boc-4-(N³-benzoyl-thymin-1-yl)-pyrrolidin-2-carbonsäure

2S,4S-N-Boc-4-(N³-benzoyl-thymin-1-yl)-pyrrolidin-2-carboxy-benzylester (10,86 g; 20,3 mmol) wird in MeOH gelöst und das Reaktionsgefäß 10 min mit N₂ gespült. Nach Zugabe von trockenem Katalysator (Pd auf Aktivkohle 10 %) wird in schwachem Wasserstoffstrom bei Normaldruck hydriert. Nach beendeter Reaktion (DC-Kontrolle, CH₂Cl₂/MeOH/HOAc 90:10:1) wird vom Katalysator abfiltriert und gründlich nachgewaschen. Das Filtrat wird zur Trockne eingedampft und an der Ölpumpe getrocknet.
Ausbeute: 9,03 g (quantitativ).
Rf: 0,60 Laufmittel: CH₂Cl₂/MeOH 9:1

### Beispiel 25

### 2S,4S-N-Boc-4-(thymin-1-yl)-pyrrolidin-2-carbonsäure (17)

2S,4S-N-Boc-4-(N³-benzoyl-thymin-1-yl)-pyrrolidin-2-carbonsäure (9,03 g; 20,3 mmol) wird mit NH₃, in Methanol über Nacht bei Raumtemperatur gerührt. Danach wird das Lösungsmittel abdestilliert. Das erhaltene Rohprodukt wird an Kieselgel mit CH₂Cl₂/MeOH/HOAc 90:10:1 als Laufmittel gesäult.
Ausbeute: 3,55 g (51,7 %).
Rf: 0,33 Laufmittel: CH₂Cl₂/MeOH/HOAc 80:20:1
Smp.: 228°C

### Beispiel 26

### 2S,4S-N-Boc-4-(N⁴-benzoyl-cytosin-1-yl)-pyrrolidin-2-carboxy-benzylester

2,5 eq. Triphenylphosphin und 2,5 eq. DEAD werden in abs. THF gelöst und 5 min bei Raumtemperatur gerührt. Nach dieser Zeit wird das N³-Benzoyl-thymin (2 eq.) zugegeben und erneut 5 min bei Raumtemperatur gerührt. Anschließend tropft man das in abs. DMF gelöste 2S,4R-N-Boc-hydroxyprolin-methylester (7,5 g; 23,36 mmol) zu und läßt über Nacht bei Raumtemperatur rühren. Nach beendeter Reaktion wird das DMF abdestilliert und das Rohprodukt an Kieselgel mit Essigsäureethylester/n-Hexan 1:1 als Eluent chromatographiert.
Ausbeute: 5,6 g (47,2 %).
Rf: 0,56 Laufmittel: Essigsäureethylester/n-Hexan 2:1

### Beispiel 27

### 2S,4S-N-Boc-4-(N⁴-benzoyl-cytosin-1-yl)-pyrrolidin-2-carbonsäure

2S,4S-N-Boc-4-(N⁴-benzoyl-cytosin-1-yl)-pyrrolidin-2-carboxy-benzylester (1,9 g; 3,75 mmol) wird in MeOH gelöst und das Reaktionsgefäß 10 min mit N₂ gespült. Nach Zugabe von trockenem Katalysator (Pd auf Aktivkohle 10 %) wird in schwachem Wasserstoffstrom bei Normaldruck hydriert. Nach beendeter Reaktion (DC-Kontrolle, CH₂Cl₂/MeOH/HOAc 90:10:1) wird vom Katalysator abfiltriert und gründlich nachgewaschen. Das Filtrat wird zur Trockne eingedampft und an der Ölpumpe getrocknet.
Ausbeute: 1,51 g (96,8 %).
Rf: 0,05 Laufmittel: CH₂Cl₂/MeOH 9:1

### Beispiel 28

### 2S,4S-N-Boc-4-(N⁴-benzoyl-cytosin-1-yl)-pyrrolidin-2-carboxyl-glycin-benzylester

2S,4S-N-Boc-4-(N⁴-benzoyl-cytosin-1-yl)-pyrrolidin-2-carbonsäure (5,9 g; 14,2 mmol) wird in DMF vorgelegt und die Lösung auf -30°C abgekühlt. Bei dieser Temperatur gibt man 1,3 eq. HOBt gefolgt von 1,3 eq. EDCI*HCl zu und läßt 10 bis 15 min bei max. -15°C rühren. In der Zwischenzeit wird das Hydrochlorid von Glycin-benzylester (1,2 eq.) in DMF aufgenommen und mit N-Ethylmorpholin neutralisiert. Diese Lösung wird nach der Voraktivierungszeit zu der auf -15°C gekühlten Reaktionslösung getropft. Man läßt 1 h bei max. -10°C und anschließend über Nacht bei Raumtemperatur rühren. Nach beendeter Reaktion wird das DMF abdestilliert, der Rückstand mit Essigsäureethylester überschichtet und 3 mal mit je 100 ml 1N HCl, ges. NaHCO₃- und ges. NaCl-Lösung ausgeschüttelt. Die organische Phase wird über Na₂SO₄ getrocknet und dann zur Trockne eingedampft. Das Rohprodukt wird schließlich an Kieselgel mit Essigsäureethylester/n-Hexan 2:1 chromatographiert.
Ausbeute: 4,2 g (51,2 %).
Rf: 0,25 Laufmittel: Essigsäureethylester/n-Hexan 2:1

### Beispiel 29

### 2S,4S-N-Boc-4-(N⁴-benzoyl-cytosin-1-yl)-pyrrolidin-2-carboxyl-glycin

2S,4S-N-Boc-4-(N⁴-benzoyl-cytosin-1-yl)-pyrrolidin-2-carboxyl-glycin-benzylester (3,67 g; 6,38 mmol) wird in MeOH gelöst und das Reaktionsgefäß 10 min. mit N₂ gespült. Nach Zugabe von trockenem Katalysator (Pd auf Aktivkohle 10 %) wird in schwachem Wasserstoffstrom bei Normaldruck hydriert. Nach beendeter Reaktion (DC-Kontrolle, CH₂Cl₂/MeOH/HOAc 90:10:1) wird vom Katalysator abfiltriert und gründlich nachgewaschen. Das Filtrat wird zur Trockne eingedampft und an der Ölpumpe getrocknet.
Ausbeute: 2,78 g (89,7 %).

### Beispiel 30

### 2S,4S-N-Boc-4-(N⁴-Z-cytosin-1-yl)-pyrrolidin-2-carboxyl-methylester

2,5 eq. Triphenylphosphin und 2,5 eq. DEAD werden in abs. THF gelöst und 5 min bei Raumtemperatur gerührt. Nach dieser Zeit wird das N⁴-Z-Cytosin (2 eq.) zugegeben und erneut 5 min bei Raumtemperatur gerührt. Anschließend tropft man das in abs. DMF gelöste 2S,4R-N-Boc-hydroxyprolin-methylester (10 g; 40,8 mmol) zu und läßt über Nacht bei Raumtemperatur rühren. Nach beendeter Reaktion wird das DMF abdestilliert und das Rohprodukt an Kieselgel mit Essigsäureethylester/n-Hexan 1:1 als Eluent chromatographiert.
Ausbeute: 15,6 g (81 %).
Rf: 0.60 Laufmittel: Essigsäureethylester/n-Hexan 2:1

### Beispiel 31

### 2S,4S-N-Boc-4-(N⁴-Z-cytosin-1-yl)-pyrrolidin-2-carbonsäure

2S,4S-N-Boc-4-(N⁴-Z-cytosin-1-yl)-pyrrolidin-2-carboxyl-methylester (15,6 g; 33 mmol) wird in Isopropanol gelöst und mit 1,1 eq. 1N NaOH versetzt. Anschließend wird bei Raumtemperatur gerührt. Nach beendeter Reaktion (DC-Kontrolle, Laufmittel CH₂Cl₂/MeOH/HOAc 90:10:1) wird das Isopropanol abdestilliert, der Rückstand mit Essigsäureethylester überschichtet und mit 1N HCl auf pH 1 angesäuert. Danach werden die Phasen getrennt, die organische Phase wird über Na₂SO₄ getrocknet und dann zur Trockne eingedampft. Das auf diese Weise erhaltene Rohprodukt wird an Kieselgel mit CH₂Cl₂/MeOH/HOAc 90:10:1 als Lauftmittel chromatographiert.
Ausbeute: 7,27 g (48 %)
Rf: 0,73 Laufmittel: CH₂Cl₂/MeOH/HOAc 80:20:1

### Beispiel 32

### 2S,4S-N-Boc-4-(4-Nitro-benzoyloxy)-pyrrolidin-2-carbonsäuremethylester (18)

2S,4R-N-Boc-hydroxyprolin-methylester (60,05 g; 269 mmol), 1,2 eq. Triphenylphosphin (85,93 g; 327 mmol) und 1,25 eq. para-Nitrobenzoesäure (56,05 g; 335 mmol) werden in abs. THF unter Schutzgas gelöst. Die erhaltene Lösung wird auf 0°C abgekühlt. Bei dieser Temperatur wird Azodicarbonsäurediethylester gelöst in abs. THF zugetropft. Anschließend läßt man auf Raumtemperatur auftauen und für weitere 70 h bei Raumtemperatur rühren. Danach wird das Lösungsmittel abdestilliert und der Rückstand mit Ether behandelt. Von den dabei ausfallenden Nebenprodukten wird abgesaugt, das Filtrat wird einrotiert und das erhaltene Rohprodukt wird an Kieselgel mit Essigsäureethylester/n-Hexan (im Verhältnis 2:1) aufgereinigt und schließlich durch Chromatographie an Kieselgel mit CH₂Cl₂/MeOH (im Verhältnis 60:1) endgereinigt.
Ausbeute: 100 g (94,15 %)
Rf: 0,85 Laufmittel: Essigsäureethylester/n-Hexan 2:1

### Beispiel 33

### 2S,4S-N-Boc-hydroxyprolin-methylester (19)

2S,4S-N-Boc-4-(4-Nitro-benzoyloxy)-pyrrolidin-2-carbonsäuremethylester (11,82 g; 30 mmol) wird in ca. 300 ml abs. MeOH gelöst. Zu dieser Lösung tropft man eine Lösung von Natriummethylat (1,62 g; 30 mmol) in Methanol und läßt 30 min. bei Raumtemperatur rühren. Anschließend wird mit 1N HCl ein pH von 5 eingestellt. Danach wird das Methanol abdestilliert. Die wäßrige Phase wird mehrmals mit Essigsäureethylester extrahiert und die vereinigten organischen Phasen werden schließlich mit ges. NaCl-Lösung gewaschen, über Natriumsulfat getrocknet und dann zur Trockne eingedampft. Das erhaltene Rohprodukt wird an Kieselgel mit Essigsäureethylester/n-Hexan (im Verhältnis 2:1) chromatographiert.
Ausbeute: 6,98 g (94,9 %)
Rf: 0,35 Laufmittel: Essigsäureethylester/n-Hexan 2:1

### Beispiel 34

### 2S,4R-N-Boc-4-(N³-benzoyl-thymin-1-yl)-pyrrolidin-2-carboxy-methylester (20)

2,5 eq. Triphenylphosphin und 2,5 eq. DEAD werden in abs. THF gelöst und 5 min. bei Raumtemperatur gerührt. Nach dieser Zeit wird das N³-Benzoyl-thymin (2 eq.) zugegeben und erneut 5 min. bei Raumtemperatur gerührt. Anschließend tropft man das in abs. DMF gelöste 2S,4S-N-Boc-hydroxyprolin-methylester (30 g; 122 mmol) zu und läßt über Nacht bei Raumtemperatur rühren. Nach beendeter Reaktion wird das DMF abdestilliert und das Rohprodukt an Kieselgel mit Essigsäureethylester/n-Hexan 1:1 als Eluent chromatographiert.
Ausbeute: 38,0 g (67,9 %)
Rf: 0,27 Laufmittel: Essigsäureethylester/n-Hexan 1:1

### Beispiel 35

### 2S,4R-N-Boc-4-(N³-benzoyl-thymin-1-yl)-pyrrolidin-2-carbonsäure

2S,4R-N-Boc-4-(N³-benzoyl-thymin-1-yl)-pyrrolidin-2-carboxy-methylester (38 g; 83 mmol) wird in Isopropanol gelöst und mit 1,1 eq. 1N NaOH versetzt. Anschließend wird bei Raumtemperatur gerührt. Nach beendeter Reaktion (DC-Kontrolle, Laufmittel CH₂Cl₂/MeOH/HOAc 90:10:1) wird das Isopropanol abdestilliert, der Rückstand mit Essigsäureethylester überschichtet und mit 1N HCl auf pH 1 angesäuert. Danach werden die Phasen getrennt, die organische Phase wird über Na₂SO₄ getrocknet und dann zur Trockne eingedampft. Das auf diese Weise erhaltene Rohprodukt wird ohne weitere Aufreinigung weiter umgesetzt (siehe Beispiel 36).

### Beispiel 36

### 2S,4R-N-Boc-4-(thymin-1-yl)-pyrrolidin-2-carbonsäure (21)

2S,4R-N-Boc-4-(N³-benzoyl-thymin-1-yl)-pyrrolidin-2-carbonsäure wird mit Ammoniak in Methanol über Nacht bei Raumtemperatur gerührt. Danach wird das Lösungsmittel abdestilliert. Das erhaltene Rohprodukt wird an Kieselgel mit CH₂Cl₂/MeOH/HOAc 90:10:1 als Laufmittel gesäult.
Ausbeute: 12,6 g (44,8 %)
Rf: 0,25 Laufmittel CH₂Cl₂/MeOH/HOAc 90:10:1

### Beispiel 37

### 2 S, 4R-N-Boc-N-(N⁴-benzyloxycarbonylcytosin-1-yl)-pyrrolidin-2-carbonsäuremethylester

3.28 g (12,5 mMol) Triphenylphosphin und 2,18 g (12,5 mMol) DEAD werden in 20 ml abs. THF gelöst und 5 min bei Raumtemperatur gerührt. Nach dieser Zeit werden 2,45 g (10 mMol) N⁴-Benzyloxycarbonylcytosin zugegeben und erneut 5 min verrührt. Anschließend tropft man 1,23 g (5 mMol) N-Boc-L-cis-hydroxyprolinmethylester in DMF gelöst zu und läßt bei Raumtemperatur über Nacht rühren. Das DMF wird abdestilliert und der Rückstand mit Essigsäureethylester versetzt. Nun wird einmal mit gesättigter NaCl-Lösung ausgeschüttelt, die organische Phase über Na₂SO₄ getrocknet, filtriert und eingeengt. Der Rückstand wird an SiO₂ mit Methylenchlorid/Methanol 30:1 als Eluent chromatographiert.
Ausbeute: 840 mg (35 % d.Th.)
RF: 0,7 Laufmittel CH₂Cl₂/CH₃OH 30:1

### Beispiel 38

### 2 S, 4R-N-Boc-4-(N⁴-benzyloxycarbonylcytosin-1-yl)-pyrrolidin-2-carbonsäure

580 mg (1,2 mmol) 2S,4R-N-Boc-4-(N⁴-Benzyloxycarbonylcytosin-1-yl)pyrrolidin-2-carbonsäuremethylester werden in 10 ml Dioxan und 2 ml H₂O gelöst und auf 10°C gekühlt. Nun werden 1,2 ml (1,2 mmol) 1N NaOH bei 10°C zugetropft und nach 5 h bei dieser Temperatur nochmals 0,6 ml (0,6 mmol) 1N NaOH. Es wird weitere 2 Stunden bei 10°C verrührt und über Nacht im Kühlschrank stehen lassen. Die Lösung wird eingeengt, mit Essigsäureethylester versetzt und einmal mit 1N HCl ausgeschüttelt. Danach wird die organische Phase über Na₂SO₄ getrocknet, filtriert und eingeengt. Der Rückstand wird an Kieselgel mit CH₂Cl₂/CH₃OH 10:1 als Eluent chromatographiert.
Ausbeute: 300 mg (53,0 % d.Th.)
RF: 0,17 Laufmittel CH₂Cl₂/CH₃OH 10:1

### Beispiel 39

### 2R, 4S-N-Boc-4-(N³-benzoyl-thymin-1-yl)-pyrrolidin-2-carbonsäuremethylester

69,5 (265 mMol) Triphenylphosphin und 46,1 g (265 mmol) DEAD werden in 400 ml abs. THF gelöst und 5 min bei Raumtemperatur verrührt. Nach dieser Zeit werden 48,8 g (212 mMol) N³-Benzoyl-thymin zugegeben und erneut 5 min bei Raumtemperatur verrührt. Anschließend tropft man 26,0 g (106 mmol). N-Boc-D-trans-hydroxyprolin-methylester in DMF gelöst zu und läßt über Nacht bei Raumtemperatur verrühren. Nach beendeter Reaktion wird das DMF abdestilliert, der Rückstand in Essigsäureethylester aufgenommen und einmal mit gesättigter NaCl-Lösung ausgeschüttelt. Die organische Phase wird über Na₂SO₄ getrocknet, abfiltriert und eingeengt. Das Rohprodukt wird an Kieselgel mit Essigsäureethylester/n-Hexan 1:1 als Eluent chromatographiert.
Ausbeute: 22,58 g (46,5 % d.Th.)
RF: 0,26 Laufmittel EE/n Hexan 1:1

### Beispiel 40

### 2R,4S-N-Boc-4-(thymin-1-yl)-pyrrolidin-2-carbonsäure

1,18 g (2,5 mMol) 2R, 4S-N-Boc-4-(N³-benzoyl-thymin-1-yl)pyrrolidin-2-carboxymethylester werden in 20 ml Isopropanol und 10 ml Dioxan gelöst und auf 3°C abgekühlt. Dann tropt man 5 ml (5 mMol) 1N NaOH bei 3°C zu und verrührt 7 Std. bei dieser Temperatur. Anschließend tropft man nochmal 2,5 ml (2,5 mmol) 1N NaOH zu und läßt über Nacht im Eisbad stehen. Die Lösung wird eingeengt und mit Essigsäureethylester und 1N HCl versetzt bis ein pH-Wertvon 1 erreicht ist. Nun schüttelt man einmal mit Essigsäureethylester aus, trocknet die organische Phase über Na₂SO₄ und engt zur Trockne ein. Das Rohprodukt wird an Kieselgel mit CH₂Cl₂/CH₃OH/HOAc 90:10:1 als Eluent chromatographiert.
Ausbeute: 430 mg (49,1 % d. Th)
RF: 0,23 Laufmittel CH₂Cl₂/CH₃OH/HOAC 90:10:1

### Beispiel 41

### N-Boc-4-(thymin-1-yl)-2-L-aminobutyryl-glycin-methylester

N-Boc-4-(thymin-1-yl)-2-L-aminobuttersäure (6,0 g; 18,35 mmol) wird in DMF vorgelegt und die Lösung auf -30°C abgekühlt. Bei dieser Temperatur gibt man 1,3 eq. HOBt gefolgt von 1,3 eq. EDCI*HCl zu und läßt 10 bis 15 min. bei maximal -15°C rühren. In der Zwischenzeit wird das Hydrochlorid von Glycinmethylester (1,2 eq.) in DMF aufgenommen und mit N-Ethylmorpholin neutralisiert. Diese Lösung wird nach der Voraktivierungszeit zu der auf -15°C gekühlten Reaktionslösung getropft. Man läßt 1 h bei maximal -10°C und anschließend über Nacht bei Raumtemperatur rühren. Nach beendeter Reaktion wird das DMF abdestilliert, der Rückstand mit Essigsäureethylester überschichtet und 3 mal mit je 100 ml 1N HCl, ges. NaHCO₃- und ges. NaCl-Lösung ausgeschüttelt. Die organische Phase wird über Na₂SO₄ getrocknet und dann zur Trockne eingedampft. Das Rohprodukt wird schließlich an Kieselgel mit Essigsäureethylester/n-Hexan 2:1 chromatographiert.
Ausbeute: 3.9 g (53 %)
Rf: 0,56 Laufmittel: CH₂Cl₂/MeOH 9:1

### Beispiel 42

N-Boc-4-(thymin-1-yl)-2-L-aminobutyryl-glycin (3,9 g; 10,1 mmol) wird in Isopropanol gelöst und mit 1,1 eq. 1N NaOH versetzt. Anschließend wird bei Raumtemperatur gerührt. Nach beendeter Reaktion (DC-Kontrolle, Laufmittel CH₂Cl₂/MeOH/HOAc 90:10:1) wird das Isopropanol abdestilliert, der Rückstand mit Essigsäureethylester überschichtet und mit 1N HCl auf pH 1 angesäuert. Danach werden die Phasen getrennt, die organische Phase wird über Na₂SO₄ getrocknet und dann zur Trockne eingedampft. Anschließend wird mit Essigsäureethylester überschichtet und mit 1N HCl angesäuert. Nach Phasentrennung wird die organische Phase über Na₂SO₄ getrocknet und dann zur Trockne eingedampft.
Ausbeute: 3,1 g (82 %)

### Beispiel 43

### 2S,4S-N-Boc-4-(thymin-1-yl)-pyrrolidin-2-carboxy-glycin-benzylester

2S,4S-N-Boc-4-(thymin-1-yl)-2-carbonsäure (5,0 g; 14,7 mmol) wird in DMF vorgelegt und die Lösung auf -30°C abgekühlt. Bei dieser Temperatur gibt man 1,3 eq. HOBt gefolgt von 1,3 eq. EDCI*HCl zu und läßt 10 bis 15 min. bei maximal -15°C rühren. In der Zwischenzeit wird das Hydrochlorid von Glycinbenzylester (1,2 eq.) in DMF aufgenommen und mit N-Ethylmorpholin neutralisiert. Diese Lösung wird nach der Voraktivierungszeit zu der auf -15°C gekühlten Reaktionslösung getropft. Man läßt 1 h bei maximal -10°C und anschließend über Nacht bei Raumtemperatur rühren. Nach beendeter Reaktion wird das DMF abdestilliert, der Rückstand mit Essigsäureethylester überschichtet und 3 mal mit je 100 ml 1N HCl, ges. NaHCO₃- und ges. NaCl-Lösung ausgeschüttelt. Die organische Phase wird über Na₂SO₄ getrocknet und dann zur Trockne eingedampft. Das Rohprodukt wird schließlich an Kieselgel mit CH₂Cl₂/MeOH (im Verhältnis 95:5) chromatographiert. Zur weiteren Reinigung wird das schon recht saubere Produkt aus einem Gemisch aus Essigsäureethylester, Ether und n-Hexan ausgefällt.
Ausbeute: 3,7 g (51,8 %)
Rf: 0,67 Laufmittel: CH₂Cl₂/MeOH 9:1
Smp: 178°C

### Beispiel 44

### 2S,4S-N-Boc-4-(thymin-1-yl)-pyrrolidin-2-carboxy-glycin

2S,4S-N-Boc-4-(thymin-1-yl)-pyrrolidin-2-carboxyl-glycin-benzylester (3,7 g; 7,61 mmol) wird in MeOH gelöst und das Reaktionsgefäß 10 min. mit N₂ gespült. Nach Zugabe von trockenem Katalysator (Pd auf Aktivkohle 10 %) wird in schwachem Wasserstoffstrom bei Normaldruck hydriert. Nach beendeter Reaktion (DC-Kontrolle, CH₂Cl₂/MeOH/HOAc 90:10:1) wird vom Katalysator abfiltriert und gründlich nachgewaschen. Das Filtrat wird zur Trockne eingedampft und an der Ölpumpe getrocknet.
Ausbeute: 2,7 g (89,5 %)

### Beispiel 45

### 2S,4R-N-Boc-4-(thymin-1-yl)-pyrrolidin-2-carboxyl-glycin-benzylester

2S,4R-N-Boc-4-(thymin-1-yl)-pyrrolidin-2-carbonsäure (4,65 g; 13,7 mmol) wird in DMF vorgelegt und die Lösung auf -30°C abgekühlt. Bei dieser Temperatur gibt man 1.3 eq. HOBt gefolgt von 1,3 eq. EDCI*HCl zu und läßt 10 bis 15 min. bei max. 15°C rühren. In der Zwischenzeit wird das Hydrochlorid von Glycinbenzylester (1,2 eq.) in DMF aufgenommen und mit N-Ethylmorpholin neutralisiert. Diese Lösung wird nach der Voraktivierungszeit zu der auf -15°C gekühlten Reaktionslösung getropft. Man läßt 1 h bei maximal -10°C und anschließend über Nacht bei Raumtemperatur rühren. Nach beendeter Reaktion wird das DMF abdestilliert, der Rückstand mit Essigsäureethylester überschichtet und 3 mal mit je 100 ml 1N HCl, ges. NaHCO₃- und ges. NaCl-Lösung ausgeschüttelt. Die organische Phase wird über Na₂SO₄ getrocknet und dann zur Trockne eingedampft.
Ausbeute: 6,7 g (97,6 %)
Rf: 0,56 Laufmittel: CH₂Cl₂/MeOH 9:1

### Beispiel 46

### 2S,4R-N-Boc-4-(thymin-1-yl)-pyrrolidin-2-carboxyl-glycin

2S,4R-N-Boc-4-(thymin-1-yl)-pyrrolidin-2-carboxyl-glycin-benzylester (6,0 g; 12,3 mmol) wird in MeOH gelöst und das Reaktionsgefäß 10 min. mit N₂ gespült. Nach Zugabe von trockenem Katalysator (Pd auf Aktivkohle 10 %) wird in schwachem Wasserstoffstrom bei Normaldruck hydriert. Nach beendeter Reaktion (DC-Kontrolle, CH₂Cl₂/MeOH/HOAc 90:10:1) wird vom Katalysator abfiltriert und gründlich nachgewaschen. Das Filtrat wird zur Trockne eingedampft und an der Ölpumpe getrocknet.
Ausbeute: 3,7 g (75,8 %)
Rf: 0,25 Laufmittel: CH₂Cl₂/MeOH/HOAc 90:10:1

### Oligomere

### Beispiel 47

### Festphasensynthese von H-(IT)₃-Gly-OH

120 mg (0,1 mmol) tert.-Butyloxycarbonyl-Glycin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 136 mg (0,34 mmol) IBocT erfolgt durch Umsetzung mit 365 mg (2,7 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 25-minütige Behandlung mit einer Lösung von 200 µl Trifluotmethansulfonsäure in 2 ml Trifluoressigsäure. Die Reindarstellung erfolgt durch RP-HPLC an C8 mit einem ansteigenden Gradienten von TFA in Acetonitril.
Ausbeute: 27 mg (29 %)

### Beispiel 48

### Festphasensynthese von H-(IT)₇-Gly-OH

120 mg (0,1 mmol) tert.-Butyloxycarbonyl-Glycin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 136 mg (0,34 mmol) IBocT erfolgt durch Umsetzung mit 365 mg (2,7 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 25-minütige Behandlung mit- einer Lösung von 200µl Trifluormethansulfonsäure in 2 ml Trifluoressigsäure. Die Reindarstellung erfolgt durch RP-HPLC an C8 mit einem ansteigenden Gradienten von TFA in Acetonitril.
Ausbeute: 72 mg (36 %)

### Beispiel 49

### Festphasensynthese von H-(IT)₁₅-Gly-OH

120 mg (0,1 mmol) tert.-Butyloxycarbonyl-Glycin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 136 mg (0,34 mmol) IBocT erfolgt durch Umsetzung mit 365 mg (2,7 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 25-minütige Behandlung mit einer Lösung von 200 µl Trifluormethansulfonsäure in 2 ml Trifluoressigsäure. Die Reindarstellung erfolgt durch RP-HPLC an C8 mit einem ansteigenden Gradienten von TFA in Acetonitril.
Ausbeute: 80 mg (19 %)
LDI-MS: 4282 g/mol gefunden, 4279,2 g/mol berechnet
LDI: Laser Desorptionsionisation

### Beispiel 50

### Festphasensynthese von H-(IC)₂-Gly-OH

120 mg (0,1 mmol) tert.-Butyloxycarbonyl-Glycin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 166 mg (0,34 mmol) IBocC^{Bz} erfolgt durch Umsetzung mit 365 mg (2,7 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 25-minütige Behandlung mit einer Lösung von 200 µl Trifluormethansulfonsäure in 2 ml Trifluoressigsäure. Die Abspaltung der Benzoyl-Schutzgruppe erfolgt durch Einwirkung von konzentrierter Ammoniak-Lösung bei 55°C. Die Reindarstellung erfolgt durch RP-HPLC an C8 mit einem ansteigenden Gradienten von TFA in Acetonitril.
Ausbeute: 7 mg (11 %)
FAB: Fast Atom Bombardment
FAB-MS: 605 g/mol gefunden 605 g/mol berechnet

### Beispiel 51

### Festphasensynthese von H-(IIT-Ala)₂-OH

125 mg (0,1 mmol) tert.-Butyloxycarbonyl-Alanin-PAM-Resin werden im Reaktionsgefäβ vorgelegt. Die tert.-Butyloxycarbonyl-Schutzgruppe wird vor jedem Kopplungsschritt durch Behandlung mit Trifluoressigsäure abgespalten. Die Aktivierung von jeweils 164 mg (0,5 mmol) IIBocT und 189 mg (1,0 mmol) tert.-Butyloxycarbonyl-Alanin erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-ButyloxycarbonylSchutzgruppe durch Behandlung mit Trifluoressigsäure entfernt. Die Abspaltung vom Träger erfolgt durch 25-minütige Behandlung mit einer Lösung von 200 µl Trifluormethansulfonsäure in 2 ml Trifluoressigsäure. Die Reindarstellung erfolgt durch RP-HPLC an C8 mit einem ansteigenden Gradienten von TFA in Acetonitril.
Ausbeute: 34 mg (59 %)
FAB-MS: 578 g/mol gefunden, 578,6 g/mol berechnet

### Beispiel 52

### Festphasensynthese von H-(IIT-Ala)₂-OH

192 mg (0,1 mmol) N-Fluorenylmethoxycarbonyl-Alanin-HMP-Resin werden im Reaktionsgefäß vorgelegt. Die N-Fluorenylmethoxycarbonyl-Schutzgruppe wird vor jedem Kopplungsschritt durch Behandlung mit Piperidin abgespalten. Die Aktivierung von jeweils 218 mg (0,5 mmol) IIFmocT und 311 mg (1,0 mmol) N-Fluorenylmethoxycarbonyl-Alanin erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die N-Fluorenylmethoxycarbonyl-Schutzgruppe durch Behandlung mit Piperidin entfernt. Die Abspaltung vom Träger erfolgt durch 60-minütige Behandlung mit Trifluoressigsäure. Die Reindarstellung erfolgt durch RP-HPLC an C8 mit einem ansteigenden Gradienten von TFA in Acetonitril.
Ausbeute: 27 mg (47 %)

### Beispiel 53

### Festphasensynthese von H-(IIC-Ala)₂-OH

125 mg (0,1 mmol) tert.-Butyloxycarbonyl-Alanin-PAM-Resin werden im Reaktionsgefäβ vorgelegt. Die tert.-Butyloxycarbonyl-Schutzgruppe wird vor jedem Kopplungsschritt durch Behandlung mit Trifluoressigsäure abgespalten. Die Aktivierung von jeweils 213 mg (0,5 mmol) IIBocC^{Bz} und 189 mg (1,0 mmol) tert.-Butyloxycarbonyl-Alanin erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 25-minütige Behandlung mit einer Lösung von 200 µl Trifluormethansulfonsäure in 2 ml Trifluoressigsäure. Die Abspaltung der Benzoyl-Schutzgruppe erfolgt durch Einwirkung von 0,4 M wässriger/methanolischer Natronlauge für 16 Stunden bei Raumtemperatur. Die Reindarstellung erfolgt durch RP-HPLC an C8 mit einem ansteigenden Gradienten von TFA in Acetonitril.
Ausbeute: 14 mg (26 %)
FAB-MS: 548 g/mol gefunden, 548,5 g/mol berechnet

### Beispiel 54

### Festphasensynthese von H-IIT-Ala-(IIT-Gly)₆-IIT-Ala-OH

125 mg (0,1 mmol) tert.-Butyloxycarbonyl-Alanin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 213 mg (0,5 mmol) IIBocT, 189 mg (1,0 mmol) tert.-Butyloxycarbonyl-Alanin und 175 mg (1,0 mmol) tert.-Butyloxycarbonyl-Glycin erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 25-minütige Behandlung mit einer Lösung von 200 µl Trifluormethansulfonsäure in 2 ml Trifluoressigsäure. Die Reindarstellung erfolgt durch RP-HPLC an C8 mit einem ansteigenden Gradienten von TFA in Acetonitril.
Ausbeute: 27 mg (12 %)
LDI-MS: 2178 g/mol gefunden, 2176,1 g/mol berechnet

### Beispiel 55

### Festphasensynthese von H-(IIT-Gly-IIT-Asp)₄-OH

135 mg (0,1 mmol) N-Fluorenylmethoxycarbonyl-Asparaginsäure-tert.-Butylester-HMP-Resin werden im Reaktionsgefäß vorgelegt. Die N-Fluorenylmethoxycarbonyl-Schutzgruppe wird vor jedem Kopplungsschritt durch Behandlung mit Piperidin abgespalten. Die Aktivierung von jeweils 100 mg (0,2 mmol) IIFmocT, 297 mg (1,0 mmol) N-Fluorenylmethoxycarbonyl-Glycin und 411 mg (1,0 mmol) N-Fluorenylmethoxycarbonyl-Asparaginsäure-tert.-butylester erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die N-Fluorenylmethoxycarbonyl-Schutzgruppe durch Behandlung mit Piperidin entfernt. Die Abspaltung vom Träger erfolgt durch 60-minütige Behandlung mit Trifluoressigsäure. Die Reindarstellung erfolgt durch RP-HPLC an C8 mit einem ansteigenden Gradienten von TFA in Acetonitril.
Ausbeute: 57 mg (24 %)
LDI-MS: 2379 g/mol gefunden, 2380,2 g/mol berechnet

### Beispiel 56

### Festphasensynthese von H-Gly-IIIT-Ala-OH

125 mg (0,1 mmol) tert.-Butyloxycarbonyl-Alanin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 169 mg (0,5 mmol) IIIBocT und 175 mg (1,0 mmol) tert.-Butyloxycarbonyl-Glycin erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 60-minütige Behandlung mit einer Lösung von 0,5 ml Anisol in 4,5 ml HF bei 0°C. Die Reindarstellung erfolgt durch RP-HPLC an C8 mit einem ansteigenden Gradienten von TFA in Acetonitril.
Ausbeute: 15 mg (41 %)
FAB-MS: 367 g/mol gefunden, 367,4 g/mol berechnet

### Beispiel 57

### Festphasensynthese von H-IIIT-Gly-IIIT-Ala-OH

125 mg (0,1 mmol) tert.-Butyloxycarbonyl-Alanin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 169 mg (0,5 mmol) IIIBocT und 175 mg (1,0 mmol) tert.-Butyloxycarbonyl-Glycin erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 60-minütige Behandlung mit einer Lösung von 0,5 ml Anisol in 4,5 ml HF bei 0°C. Die Reindarstellung erfolgt durch RP-HPLC an C8 mit einem ansteigenden Gradienten von TFA in Acetonitril.
Ausbeute: 25 mg (43 %)
FAB-MS: 588 g/mol gefunden, 588,6 g/mol berechnet

### Beispiel 58

### Festphasensynthese von H-(IVC)₂-Ala-OH

125 mg (0,1 mmol) tert.-Butyloxycarbonyl-Alanin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 243 mg (0,5 mmol) IVBocC^{Bz} erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 8-stündige Behandlung mit einer Lösung von 0,5 ml Anisol in 4,5 ml HF bei 0°C. Die Reindarstellung erfolgt durch RP-HPLC an C8 mit einem ansteigenden Gradienten von TFA in Acetonitril.
Ausbeute: 33 mg (53 %)
FAB-MS: 616 g/mol gefunden, 617 g/mol berechnet

### Beispiel 59

### Festphasensynthese von H-(IIC-Ala)₂-OH

125 mg (0,1 mmol) tert.-Butyloxycarbonyl-Alanin-PAM-Resin werden im Reaktionsgefäβ vorgelegt. Die tert.-Butyloxycarbonyl-Schutzgruppe wird vor jedem Kopplungsschritt durch Behandlung mit Trifluoressigsäure abgespalten. Die Aktivierung von jeweils 446 mg (1,0 mmol) IIBocC^{Bz} und 139 mg (1,0 mmol) tert.-Butyloxycarbonyl-Alanin erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-ButyloxycarbonylSchutzgruppe durch Behandlung mit Trifluoressigsäure entfernt. Die Abspaltung vom Träger erfolgt durch 60 minütige Behandlung mit einer Lösung von 0,5 ml Anisol in 4,5 ml HF bei 0°C. Die Reindarstellung erfolgt durch RP-HPLC an C8 mit einem ansteigenden Gradienten von TFA in Acetonitril.
Ausbeute: 42 mg (76 %)
FAB-MS: 548 g/mol gefunden, 549,0 g/mol berechnet

### Beispiel 60

### Festphasensynthese von H-IVT-IVC-Ala-OH

125 mg (0,1 mmol) tert-Butyloxycarbonyl-Alanin-PAM-Resin werden im Reaktionsgefäß vorgelegt. Die tert.-Butyloxycarbonyl-Schutsgruppe wird vor jedem Kopplungsschritt durch Behandlung mit Trifluoressigsäure abgespalten. Die Aktivierung von jeweils 198 mg (0,5 mmol) IVBocT und 243 mg (0,5 mmol) IVBocC^{Bz} erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 8-stündige Behandlung mit einer Lösung von 0,5 ml Anisol in 4,5 ml HF bei 0°C. Die Reindarstellung erfolgt durch RP-HPLC an C8 mit einem ansteigenden Gradienten von TFA in Acetonitril.
Ausbeute: 54 mg (86 %)
ESI-MS: 630 g/mol gefunden, 630,6 g/mol berechnet
ESI: Electron Spray Ionisation

### Beispiel 61

### Festphasensynthese von H-Lys-(IVT)₈-Ala-OH

125 mg (0,1 mmol) tert.-Butyloxycarbonyl-Alanin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 198 mg (0,5 mmol) IVBocT und 415mg (1,0 mmol) tert.-Butyloxycarbonyl-2-chlorbenzyloxycarbonyl-Lysin erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 60 minütige Behandlung mit einer Lösung von 0,5 ml Anisol in 4,5 ml HF bei 0°C. Die Reindarstellung erfolgt durch RP-HPLC an C8 mit einem ansteigenden Gradienten von TFA in Acetonitril.
Ausbeute: 180 mg (74 %)
ESI-MS: 2442 g/mol gefunden, 2443,3 g/mol berechnet

### Beispiel 62

### Festphasensynthese von H-Lys-IVT-IVC-IVT-IVC-IVC-IVT-IVC-IVT-Ala-OH

125 mg (0,1 mmol) tert.-Butyloxycarbonyl-Alanin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 198 mg (0,5 mmol) IVBocT, 243 mg (0,5 mmol) IVBocC^{Bz} und 415 mg (1,0 mmol) tert.-Butyloxycarbonyl-2-chlorbenzyloxycarbonyl-Lysin erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 8 stündige Behandlung mit einer Lösung von 0,5 ml Anisol in 4,5 ml HF bei 0°C. Die Reindarstellung erfolgt durch RP-HPLC an C8 mit einem ansteigenden Gradienten von TFA in Acetonitril.
Ausbeute: 83 mg (35 %)
LDI-MS: 2382 g/mol gefunden, 2383,3 g/mol berechnet

### Beispiel 63

### Festphasensynthese von H-Lys-(VT)₈-Ala-OH

125 mg (0,1 mmol) tert.-Butyloxycarbonyl-Alanin-PAM-Resin werden im Reaktionsgefäß vorgelegt. Die tert.-Butyloxycarbonyl-Schutzgruppe wird vor jedem Kopplungsschritt durch Behandlung mit Trifluoressigsäure abgespalten. Die Aktivierung von jeweils 115 mg (0,3 mmol) VBocT und 415 mg (1,0 mmol) tert.-Butyloxycarbonyl-2-chlorbenzyloxycarbonyl-Lysin erfolgt durch Umsetzung mit 135 mg (1,0 mmol) Hydroxybenzotriazol und 206 mg (1,0 mmol) Dicyclohexylcarbodiimid in N-Methyl-2-pyrrolidon. Im Anschluß erfolgt die schrittweise Ankopplung an den polymeren Träger. Nach der letzten Kopplung wird die tert.-Butyloxycarbonyl-Schutzgruppe durch Behandlung mit Trifluoressigsäure entfernt. Die Abspaltung vom Träger erfolgt durch 60 minütige Behandlung mit einer Lösung von 0,5 ml Anisol in 4,5 ml HF bei 0°C. Die Reindarstellung erfolgt durch RP-HPLC an C8 mit einem ansteigenden Gradienten von TFA in Acetonitril.
Ausbeute: 83 mg (35 %)
LDI-MS: 2347,4 g/mol gefunden, 2347,3 g/mol berechnet

### Nachweis von DNA-Einzelstrangbindung durch Gel-shift-Analysen

### Beispiel 64

1 µg Oligonukleotid von entsprechender Basensequenz wird mit Polynukleotidkinase und γ-ATP am 5'-Ende in bekannter Weise in einem Volumen von 10 µl markiert (Sambrook, Fritsch, Maniatis: Molecular Cloning, A Laboratory Manual, Cold Spring Harbor, 1989). Nach der Markierung wird die Probe zur Denaturierung des Enzyms bei 70°C für 10 min erhitzt und anschließend mit 9 µg nicht markiertem Oligomer vermischt. 1 µl dieses Ansatzes wird mit einer gewünschten Menge zu testendem Nukleinsäure-bindenden Oligomer versetzt (1-10 µg) und in einem Volumen von 20 µl 30 min bei 22°C (Raumtemperatur) inkubiert (Hybridisierung). Danach wird die Probe 30 min auf Eis gestellt. Ein nicht hybridisiertes markiertes Oligomer wird in gleicher Weise behandelt und dient als Kontrolle. Die Proben werden auf ein 15 % Polyacrylamidgel mit lx Tris-Borat-EDTA-Puffer aufgetragen. Das Gel und der Puffer wurden im Kühlschrank (8°C) vorgekühlt, die Elektrophorese wurde über Nacht mit 55 V im Kühlschrank laufen gelassen. Nach der Elektrophorese wurde ein Autoradiogramm auf AGFA-Film erstellt (Exponierzeiten 1 bis 16 Stunden).

### Nachweis der Strangverdrängung in doppelsträngiger Plasmid-DNA durch Nukleinsäurenbindende Oligomere

### Beispiel 65

Die Tests werden wie folgt durchgeführt:

(Die im Beispiel eingesetzte Plasmid-DNA ist nur ein Modell-Substrat im Test. Andere Plasmide, die Poly-Adenin Sequenzbereiche mit definierten Abständen zueinander enthalten, sind ebenfalls verwendbar.)

Doppelsträngige, zirkuläre Plasmid-DNA von 4880 Basenpaaren Länge, die zwei Poly-Adenin Sequenzbereiche mit mindestens neun aufeinander folgenden Adeninnukleotiden im Abstand von 1150 Basenpaaren enthält, wird in den hier beschriebenen Tests verwendet.

Sechs parallel angesetzte Proben (bezeichnet 1-6) enthielten je 1,0 µg ungeschnittene Plasmid-DNA in 14 µl H₂O. Den Proben 3 bis 6 wurden je 1 µl Lösung von 0,01 µg, 0,1 µg, 1,0 µg und 2,0 µg Nukleinsäuren-bindendes Oligomer zugesetzt und in geschlossenen Eppendorfreakfionsgefäßen 45 Minuten bei 37°C inkubiert. Anschließend wurden in alle Proben 4 µl Puffer (250 mM Na-Acetat, 1M NaCl, 2,5 % Glycerin, 5 mM ZnCl₂, pH 4,4), und in die Proben 2 bis 6 je 1 µl S1-Nuclease (Aspergillus oryzae, Fa. Boehringer-Mannheim) mit einer Aktivität von 10 U/µl gegeben.

Nach einer Inkubation von 15 Minuten bei 30°C wurden die Proben auf Eis gesetzt, 1µl 0,5M EDTA und 3 µl Auftragspuffer (50 % Glycerin, 0,25 % Bromphenolblau in 40 mM Tris-HCl, 20 mM Na-Acetat, 1 mM EDTA, pH: 7,2) zugegeben und ohne Zeitverzug die Proben über 1,2 %-Agarosegele elektrophoretisch aufgetrennt und nach Anfärbung mit Ethidiumbromid die Größe der entstandenen Plasmid-Fragmente im Gel im Vergleich zu einem Molekulargewichtsstandard (1-kb Leiter, Fa. Gibco-BRL, D-7514 Eggenstein) auf dem Transilluminator (264 nm UV-Licht) bestimmt.

Es zeigte sich, daß in den Proben mit einer Konzentration > 0,1 µg Oligomer (Proben 5 und 6) DNA-Fragmente von 4880 Basenpaaren (Plasmid-Linearisierung), und 3730 sowie 1150 Basenpaaren (sequenzselektive Fragmentierung) sichtbar waren.

Mit einem modifizierten Testansatz, bei dem in die Proben anstelle der zirkulären Plasmid-DNA eine Plasmid-DNA gegeben wurde, die durch Restriktionsendonukleaseverdau in unmittelbarer Nachbarschaft zu einer der beiden Poly-Adenin Sequenzbereiche linearisiert war, waren ebenfalls in den Proben 5 und 6 die DNA-Fragmente von 3730 und 1150 Basenpaaren Länge nachweisbar.

Mit diesen Testreihen war die konzentrationsabhängige und sequenzselektive Bindung von Nukleinsäuren-bindenden Oligomeren an doppelsträngige DNA und der Nachweis der dadurch entstehenden Einzelstrang-DNA durch S1-Nukleaseverdauung bei hohen Salzkonzentrationen (einzelstrangspezifische Aktivität von S1-Nuklease) nachweisbar.

### Proteinsynthesehemmung in in vitro Translationstesten durch Nukleinsäure-bindende Oligomere

### Beispiel 66

Zur in vitro Translation wurde ein Kaninchenretikulozytenlysat der Fa. Promega, Madison, Wisconsin verwendet, sowie in vitro transkribierte mRNA des tat-Gens aus HIV-I und der Delta-Untereinheit des Acetylcholinrezeptors aus Torpedo californica. Andere Gene können in gleicher Weise verwendet werden. Die cDNA-Konstrukte der Gene wurden mit SP6- bzw. T7-RNA-Polymerase in gängiger Weise transkribiert (Sambrook et al., dito), das DNA-Plasmid wurde anschließend durch DNase verdaut, die mRNA phenolisiert und dreimal mit Ethanol gefällt. 1 bis 2 µg der erhaltenen mRNA wurde in Gegenwart von ³⁵S-markiertem Cystein zur in vitro Translation eingesetzt. Die Analyse des gebildten radioaktiven Proteins erfolgte auf einem 6 bis 18 % bzw. 6 bis 10 % diskontinuierlichen SDS-PAGE nach Laemmli, U.K. (1970) Nature 227, 680-685.

Zur quantitativen Messung der Translationsinhibition durch Nukleinsäure-bindende Oligomere wurde zur mRNA eine gewünschte Menge Oligomer (0,01 bis 2 µg) hinzugegeben und dann wie oben beschrieben in Kaninchenretikulozytenlysat die in vitro Translation durchgeführt. Autoradiographien von SDS-Polyacrylamidelektrophoresegelen der Testansätze wurden mit einem Scanner quantitativ ausgewertet.

## Patentansprüche

1. Verbindungen der allgemeinen Formel (I) 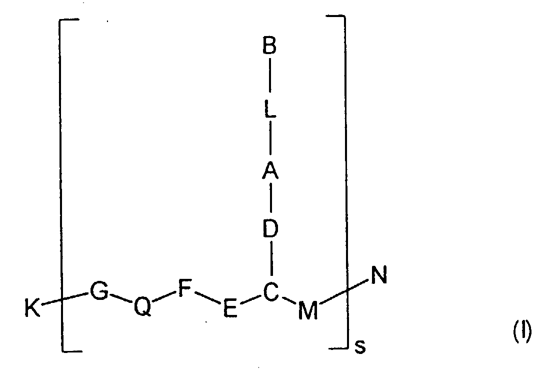 in der
A für -CO-, -CHR-, -CRR'- steht,
B unabhängig voneinander ausgewählt wird aus einer Gruppe, bestehend aus:
(C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin oder durch chemische Modifikation von diesen abgeleitete Derivate sowie halogenierten Vorstufen dieser Nukleobasen, sowie geschützte Derivate der Nukleobasen mit den in der Nukleotid- oder Peptidchemie üblichen Schutzgruppen,
C für -CH-, -CR- steht, wobei C einheitlich S oder R konfiguriert sein kann,
D für -NH-, -CH₂-, -CHR-, -CRR'- steht,
E für -NH-, -NR-, -CHR-, -CRR'-, -O-, -S- steht,
A und E miteinander über eine Alkylkette [-(CH₂)ₙ₋, mit n = 1, 2] verbunden sein können,
F für -CH₂-, -CO-, -SO₂, -SO-, -CS- steht,
Q für (-CR¹R²)ₘ mit m = 0, 1, 2 und R¹, R² unabhängig voneinander ausgewählt aus einer Gruppe bestehend aus Resten von natürlichen oder unnatürlichen Aminosäuren wie z.B. aus:
Glycin, Alanin, Valin, Leucin, Isoleucin, Serin, Threonin, Cystein, Methionin, Phenylalanin, Thyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, 4-Nitrophenylalanin, 3-Nitrophenylalanin, 2-Nitrophenylalanin, 2-, 3-oder 4-Aminophenylalanin, 3,4-Dichlorphenylalanin, 4-Jodphenylalanin, 4-Methoxyphenylalanin, 1-Triazolylalanin, 2-Pyridylalanin, 3-Pyridylalanin, 4-Pyridylalanin, 1-Naphthylalanin oder 2-Naphthylalanin, gegebenenfalls mit Schutzgruppen, stereochemisch einheitlich in der L-Form oder der D-Form steht,
G und Q miteinander über eine Alkylkette [(-CH₂)ₙ mit n = 1, 2] verbunden sein können
G für -NH-, -NR-, -O-, -S- steht,
M für -CH₂-, -CO-, -SO₂-, -SO-, -CS- steht,
L für (CH₂)ₚ mit p = 0, 1, 2, -CHR-, -CRR'- steht,
K für Carrier-System, Reporter-Ligand, H, löslichkeitsvermittelnde Gruppe steht,
N für Carrier-System, Reporter-Ligand, OH, löslichkeitsvermittelnde Gruppe steht,
s für einen Wert von 1 bis 30 steht und
R und R' für H, OH, Alkyl, Aralkyl oder Aryl stehen.

2. Verbindungen der allgemeinen Formel (I) gemäß Anspruch 1, in der
A für -CO-, -CHR-, -CRR'- steht,
B unabhängig voneinander ausgewählt wird aus einer Gruppe, bestehend aus:
(C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin. Inosin sowie halogenierten Vorstufen dieser Nukleobasen, sowie geschützte Derivate der Nukleobasen mit den in der Nukleotid- oder Peptidchemie üblichen Schutzgruppen,
C für -CH-, -CR- steht, wobei C einheitlich S oder R konfiguriert sein kann,
D für -NH-, -CH₂-, -CHR-, -CRR'- steht,
E für -NR-, -NH-, -CHR-, -CRR'-, -O- steht,
A und E miteinander über eine Alkylkette [-(CH₂)ₙ-, mit n = 1, 2] verbunden sein können,
F für -CH₂-, -CO-, SO₂-, -SO-, -CS- steht,
Q für (-CR¹R²)ₘ mit m = 0, 1, 2 und R¹, R² unabhängig voneinander ausgewählt aus einer Gruppe bestehend aus Resten von natürlichen oder unnatürlichen Aminosäuren wie z.B. aus:
Glycin, Alanin, Valin, Leucin, Isoleucin, Serin, Threonin, Cystein, Methionin, Phenylalanin, Thyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, 4-Nitrophenylalanin, 3-Nitrophenylalanin, 2-Nitrophenylalanin, 1-Naphthylalanin oder 2-Naphthylalanin, gegebenenfalls mit Schutzgruppen, stereochemisch einheitlich in der L-Form oder der D-Form steht,
G und Q miteinander über eine Alkylkette [(-CH₂)ₙ mit n = 1, 2] verbunden sein können
G für -NH-, -NR-, -O- steht,
M für -CH₂-, -CO-, -SO₂-, -CS- steht,
L für (CH₂)ₚ mit p = 0, 1, 2, -CHR-, -CRR'- steht,
K für Carrier-System, Reporter-Ligand, H, löslichkeitsvermittelnde Gruppe steht,
N für Carrier-System, Reporter-Ligand, OH, löslichkeitsvermittelnde Gruppe steht,
s für einen Wert von 1 bis 30 steht,
R und R' für H, OH, Alkyl, Aralkyl oder Aryl stehen.

3. Verbindungen der allgemeinen Formel (I) gemäß Anspruch 1, in der
A für -CO-, -CHR-, -CRR'- steht,
B unabhängig voneinander ausgewählt wird aus einer Gruppe, bestehend aus:
(C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin sowie halogenierten Vorstufen dieser Nukleobasen, sowie geschützte Derivate der Nukleobasen mit den in der Nukleotid- oder Peptidchemie üblichen Schutzgruppen,
C für -CH-, -CR- steht, wobei C einheitlich S oder R konfiguriert sein kann,
D für -NH-, -CH₂-, -CHR-, -CRR'- steht,
E für -NR-, -NH-, -CHR- steht,
A und E miteinander über eine Alkylkette [-(CH₂)ₙ-, mit n = 1, 2] verbunden sein können,
F für -CH₂-, -CO-, -CS- steht,
Q für (-CR¹R²)ₘ mit m = 0, 1, 2 und R¹, R² unabhängig voneinander ausgewählt aus einer Gruppe bestehend aus Resten von natürlichen oder unnatürlichen Aminosäuren wie z.B. aus:
Glycin, Alanin, Valin, Leucin, Isoleucin, Serin, Threonin, Cystein, Methionin, Phenylalanin, Thyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, gegebenenfalls mit Schutzgruppen, stereochemisch einheitlich in der L-Form oder der D-Form steht,
G und Q miteinander über eine Alkylkette [(-CH₂)ₙ mit n = 1, 2] verbunden sein können
G für -NH-, -NR-, -O- steht,
M für -CH₂-, -CO-, -CS- steht,
L für (CH₂)ₚ mit p = 0, 1, 2, -CHR-, -CRR'- steht,
K für Carrier-System, Reporter-Ligand, H, löslichkeitsvermittelnde Gruppe steht,
N für Carrier-System, Reporter-Ligand, OH, löslichkeitsvermittelnde Gruppe steht,
s für einen Wert von 1 bis 30 steht,
R und R' für H, OH, Alkyl, Aralkyl oder Aryl stehen.

4. Verbindungen der allgemeinen Formel (II) 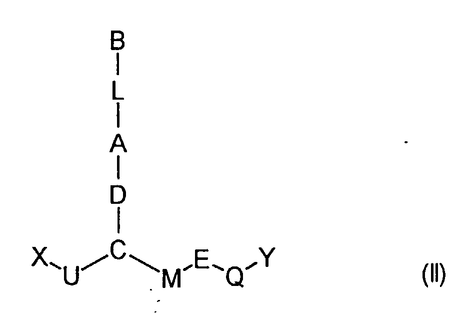 in der
A für -CO-, -CHR-, -CRR'- steht,
B unabhängig voneinander gewählt wird aus einer Gruppe, bestehend aus: (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin oder durch chemische Modifikation von diesen abgeleiteten Derivaten, sowie halogenierten Vorstufen dieser Nukleobasen, sowie geschützte Derivate dieser Nukleobasen mit den in der Nukleotid- oder Peptidchemie üblichen Schutzgruppen,
C für -CH-, -CR- steht, wobei C einheitlich S oder R konfiguriert sein kann,
D für -NH-, -CH₂-, -CHR-, -CRR'- steht,
E für -NH-, -NR-, -CHR-, -CRR'-, -O-, -S- steht,
Q für (-CR¹R²-)ₘ mit m = 0, 1, 2 und R¹, R² unabhängig voneinander ausgewählt aus einer Gruppe bestehend aus Resten von natürlichen oder unnatürlichen Aminosäuren wie z.B. aus: Glycin, Alanin, Valin, Leucin, Isoleucin, Serin, Threonin, Cystein, Methionin, Phenylalanin, Thyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, 4-Nitrophenylalanin, 3-Nitrophenylalanin, 2-Nitrophenylalanin, 2-, 3- oder 4-Aminophenylalanin, 3,4-Dichlorphenylalanin, 4-Jodphenylalanin, 4-Methoxyphenylalanin, 1-Triazolylalanin, 2-Pyridylalanin, 3-Pyridylalanin, 4-Pyridylalanin, 1-Naphthylalanin oder 2-Naphthylalanin, gegebenenfalls mit Schutzgruppen, stereochemisch einheitlich in der L-Form oder der D-Form steht,
E und Q miteinander über eine Alkylkette [(-CH₂-)ₙ mit n = 1, 2] verbunden sein können
M für -CH₂-, -CO-, -SO₂-, -SO-, -CS- steht,
L für (CH₂)ₚ mit p = 0, 1, 2, -CHR-, -CRR'- steht,
U für -NH-, -NR- steht,
X für eine beliebige aus der Peptidchemie bekannte Schutzgruppe, H oder eine beliebige natürliche oder unnatürliche Aminosäure in geschützter oder ungeschützter Form steht und
Y für COOH, CSOH, CH₂OH, COOR" mit R" = beliebige Schutzgruppe aus der Peptidchemie, Carrier, Reporter-Ligand, löslichkeitsvermittelnde Gruppe steht,
R und R' für H, OH, Alkyl, Aralkyl oder Aryl stehen.

5. Verbindungen der Formel (II) gemäß Anspruch 4, in der
A für -CO-, -CHR-, -CRR'- steht,
B unabhängig voneinander gewählt wird aus einer Gruppe, bestehend aus: (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin sowie halogenierten Vorstufen dieser Nukleobasen, sowie geschützte Derivate dieser Nukleobasen mit den in der Nukleotid- oder Peptidchemie üblichen Schutzgruppen,
C für -CH-, -CR- steht, wobei C einheitlich S oder R konfiguriert sein kann,
D für -NH-, -CH₂-, -CHR-, -CRR'- steht,
E für -NR-, -NH-, -CHR-, -CRR'-, -O- steht,
Q für (-CR¹R²-)ₘ mit m = 0, 1, 2 und R¹, R² unabhängig voneinander ausgewählt aus einer Gruppe bestehend aus Resten von natürlichen oder unnatürlichen Aminosäuren wie z.B. aus: Glycin, Alanin. Valin, Leucin, Isoleucin, Serin, Threonin, Cystein, Methionin. Phenylalanin, Thyrosin, Histidin, Tryptophan, Lysin, Ornithin. Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, 4-Nitrophenylalanin, 3-Nitrophenylalanin, 2-Nitrophenylalanin, 1-Naphthylalanin oder 2-Naphthylalanin, gegebenenfalls mit Schutzgruppen, stereochemisch einheitlich in der L-Form oder der D-Form steht,
E und Q miteinander über eine Alkylkette [(-CH₂-)ₙ mit n = 1, 2] verbunden sein können
M für -CH₂-, -CO-, -SO₂-, -SO-, -CS- steht,
L für (CH₂)ₚ mit p = 0, 1, 2, -CHR-, -CRR'- steht,
U für -NH-, -NR- steht,
X für eine beliebige aus der Peptidchemie bekannte Schutzgruppe, H oder eine beliebige natürliche oder unnatürliche Aminosäure in geschützter oder ungeschützter Form steht und
Y für COOH, CSOH, CH₂OH, COOR" mit R" = beliebige Schutzgruppe aus der Peptidchemie, Carrier, Reporter-Ligand, löslichkeitsvermittelnde Gruppe steht,
R und R' für H, OH, Alkyl, Aralkyl oder Aryl stehen.

6. Verbindungen der allgemeinen Formel (II) gemäß Anspruch 4, in der
A für -CO-, -CHR-, -CRR'- steht,
B unabhängig voneinander gewählt wird aus einer Gruppe, bestehend aus: (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin sowie halogenierten Vorstufen dieser Nukleobasen sowie geschützte Derivate dieser Nukleobasen mit den in der Nukleotid- oder Peptidchemie üblichen Schutzgruppen,
C für -CH-, -CR- steht, wobei C einheitlich S oder R konfiguriert sein kann,
D für -NH-, -CH₂-, -CHR-, -CRR'- steht,
E für -NR-, -NH-, -CHR-, -O- steht,
Q für (-CR¹R²-)ₘ mit m = 0, 1, 2 und R¹, R² unabhängig voneinander ausgewählt aus einer Gruppe bestehend aus Resten von natürlichen oder unnatürlichen Aminosäuren wie z.B. aus: Glycin, Alanin, Valin, Leucin, Isoleucin, Serin, Threonin, Cystein, Methionin, Phenylalanin, Thyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, gegebenenfalls mit Schutzgruppen, stereochemisch einheitlich in der L-Form oder der D-Form steht,
E und Q miteinander über eine Alkylkette [(-CH₂-)ₙ mit n = 1, 2] verbunden sein können
M für -CH₂-, -CO-, -CS- steht,
L für (CH₂)ₚ mit p = 0, 1, 2, -CHR-, -CRR'- steht,
U für -NH-, -NR- steht,
X für eine beliebige aus der Peptidchemie bekannte Schutzgruppe, H oder eine beliebige natürliche oder unnatürliche Aminosäure in geschützter oder ungeschützter Form steht
Y für COOH, CSOH, CH₂OH, COOR" mit R" = beliebige Schutzgruppe aus der Peptidchemie, Carrier, Reporter-Ligand, löslichkeitsvermittelnde Gruppe steht,
R und R' für H, OH, Alkyl, Aralkyl oder Aryl stehen.

7. Verbindungen der allgemeinen Formel (III) 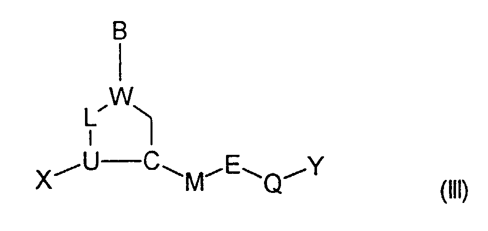 in der
B unabhängig voneinander gewählt wird aus einer Gruppe, bestehend aus: (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin, Inosin oder durch chemische Modifikation von diesen abgeleiteten Derivaten, sowie halogenierten Vorstufen dieser Nukleobasen sowie geschützte Derivate dieser Nukleobasen mit den in der Nukleotid- oder Peptidchemie üblichen Schutzgruppen,
C für -CH-, -CR- steht, wobei C einheitlich S oder R konfiguriert sein kann,
E für -NR-, -CHR-, -CRR'-, -O-, -S- steht,
Q für (-CR¹R²-)ₘ mit m = 0, 1, 2 und R¹, R² unabhängig voneinander ausgewählt aus einer Gruppe bestehend aus Resten von natürlichen oder unnatürlichen Aminosäuren wie z.B. aus: Glycin, Alanin, Valin, Leucin, Isoleucin, Serin, Threonin, Cystein, Methionin, Phenylalanin, Thyrosin. Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, 4-Nitrophenylalanin, 3-Nitrophenylalanin, 2-Nitrophenylalanin, 2-, 3-oder 4-Aminophenylalanin, 3,4-Dichlorphenylalanin, 4-Jodphenylalanin, 4-Methoxyphenylalanin, 1-Triazolylalanin, 2-Pyridylalanin, 3-Pyridylalanin, 4-Pyridylalanin, 1-Naphthylalanin oder 2-Naphthylalanin, gegebenenfalls mit Schutzgruppen, stereochemisch einheitlich in der L-Form oder der D-Form steht,
E und Q miteinander über eine Alkylkette [(-CH₂-)ₙ mit n = 1, 2] verbunden sein können
M für -CH₂-, -CO-, -SO₂-, -SO-. -CS- steht,
L für CH₂ steht,
U für -NH-, -NR- steht steht,
X für eine beliebige aus der Peptidchemie bekannte Schutzgruppe, H oder eine beliebige natürliche oder unnatürliche Aminosäure in geschützter oder ungeschützter Form steht,
Y für COOH, CSOH, CH₂OH, COOR" mit R" = beliebige Schutzgruppe aus der Peptidchemie. Carrier, Reporter-Ligand, löslichkeitsvermittelnde Gruppe steht,
W für ein chirales C-Atom steht, welches einheitlich S oder R konfiguriert sein kann,
R und R' für H, OH, Alkyl, Aralkyl oder Aryl stehen.

8. Verbindungen der Formel (III) gemäß Anspruch 7, in der
B unabhängig voneinander gewählt wird aus einer Gruppe, bestehend aus: -H, -OH, (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin. Inosin sowie halogenierten Vorstufen dieser Nukleobasen, sowie geschützte Derivate dieser Nukleobasen mit den in der Nukleotid- oder Peptidchemie üblichen Schutzgruppen,
C für -CH-, -CR- steht, wobei C einheitlich S oder R konfiguriert sein kann,
E für -NR-, -NH-, -CHR-, -CRR'-, -O- steht,
Q für (-CR¹R²-)ₘ mit m = 0, 1, 2 und R¹, R² unabhängig voneinander ausgewählt aus einer Gruppe bestehend aus Resten von natürlichen oder unnatürlichen Aminosäuren wie z.B. aus: Glycin, Alanin Valin, Leucin, Isoleucin, Serin, Threonin, Cystein, Methionin. Phenylalanin, Thyrosin, Histidin, Tryptophan, Lysin, Ornithin. Asparaginsäure, Glutaminsäure, Asparagin, Glutamin, Arginin. Prolin, Hydroxyprolin, Sarcosin, Aminoisobuttersäure, Dehydroaminosäuren wie z.B. Dehydroalanin, Dehydro-α-aminobuttersäure. andere unnatürliche Aminosäuren wie Phenylglycin, 4-Nitrophenylalanin, 3-Nitrophenylalanin, 2-Nitrophenylalanin, 1-Naphthylalanin oder 2-Naphthylalanin, gegebenenfalls mit Schutzgruppen, stereochemisch einheitlich in der L-Form oder der D-Form steht,
E und Q miteinander über eine Alkylkette [(-CH₂-)ₙ mit n = 1, 2] verbunden sein können
M für -CH₂-, -CO-, -SO₂-, -SO-, -CS- steht,
L für -CH₂- steht,
U für -NH-, -NR- steht.
X für eine beliebige aus der Peptidchemie bekannte Schutzgruppe, H oder eine beliebige natürliche oder unnatürliche Aminosäure in geschützter oder ungeschützter Form steht,
Y für COOH, CSOH, CH₂OH, COOR" mit R" = beliebige Schutzgruppe aus der Peptidchemie, Carrier, Reporter-Ligand, löslichkeitsvermittelnde Gruppe steht,
W für ein chirales C-Atom steht, welches einheitlich S oder R konfiguriert sein kann,
R und R' für H, OH, Alkyl. Aralkyl oder Aryl stehen.

9. Verbindungen der Formel (III) gemäß Anspruch 7, in der
B unabhängig voneinander gewählt wird aus einer Gruppe, bestehend aus: -H, -OH, (C₁-C₄)-Alkanoyl, DNA-Interkalatoren, aromatischen Resten, heterocyclischen Resten, natürlich vorkommenden Nukleobasen wie Thymin, Uracil, Cytosin, Adenin, Guanin, Hypoxanthin. Inosin sowie halogenierten Vorstufen dieser Nukleobasen sowie geschützte Derivate dieser Nukleobasen mit den in der Nukleotid-. oder Peptidchemie üblichen Schutzgruppen,
C für -CH-, -CR- steht, wobei C einheitlich S oder R konfiguriert sein kann,
E für -NR-, -NH-, -CHR-, -O- steht,
Q für (-CR¹R²-)ₘ mit m = 0, 1, 2 und R¹, R² unabhängig voneinander ausgewählt aus einer Gruppe bestehend aus Resten von natürlichen oder unnatürlichen Aminosäuren wie z.B. aus: Glycin, Alanin, Valin, Leucin, Isoleucin, Serin, Threonin, Cystein, Methionin, Phenylalanin, Thyrosin, Histidin, Tryptophan, Lysin, Ornithin, Asparaginsäure, Glutaminsäure, Asparagin, Glutamin, Arginin, Prolin, Hydroxyprolin, Sarcosin, Aminoisobuttersäure, Dehydroaminosäuren wie z.B. Dehydroalanin, Dehydro-α-aminobuttersäure, andere unnatürliche Aminosäuren wie Phenylglycin, gegebenenfalls mit Schutzgruppen, stereochemisch einheitlich in der L-Form oder der D-Form steht,
E und Q miteinander über eine Alkylkette [(-CH₂-)ₙ mit n = 1, 2] verbunden sein können
M für -CH₂-, -CO-, -CS- steht,
L für -CH₂- steht
U für -NH-, -NR-,
X für eine beliebige aus der Peptidchemie bekannte Schutzgruppe, H oder eine beliebige natürliche oder unnatürliche Aminosäure in geschützter oder ungeschützter Form steht,
Y für COOH, CSOH, CH₂OH, COOR" mit R" = beliebige Schutzgruppe aus der Peptidchemie, Carrier, Reporter-Ligand, löslichkeitsvermittelnde Gruppe steht,
W für ein chirales C-Atom steht, welches einheitlich S oder R konfiguriert sein kann,
R und R' für H, OH, Alkyl, Aralkyl oder Aryl stehen.

10. Arzneimittel enthaltend eine oder mehrere Verbindungen aus den Ansprüchen 1 bis 9.

## Claims

1. Compounds of the general formula (I) 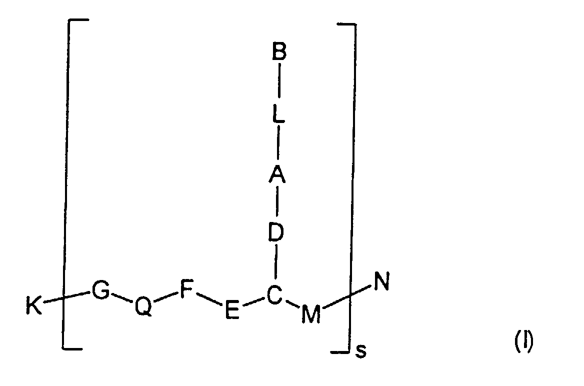 in which
A represents -CO-, -CHR- or -CRR'-,
B independently of one another is selected from a group consisting of:
(C₁-C₄)-alkanoyl, DNA intercalators, aromatic radicals, heterocyclic radicals, naturally occurring nucleic bases, such as thymine, uracil, cytosine, adenine, guanine, hypoxanthine, inosine or substances derived from these by chemical modification, and halogenated precursors of these nucleic bases, and protected derivatives of the nucleic bases having the protective groups which are customary in nucleotide or peptide chemistry,
C represents -CH- or -CR-, where C can uniformly exist in the S or R configuration,
D represents -NH-, -CH₂-, -CHR- or -CRR'-,
E represents -NH-, -NR-, -CHR-, -CRR'-, -O- or -S-,
A and E can be linked to each other via an alkyl chain [-(CH₂)ₙ- where n = 1, 2],
F represents -CH₂-, -CO-, -SO₂-, -SO- or -CS-,
Q represents (-CR¹R²)ₘ-, where m is 0, 1 or 2 and R¹ and R² independently of one another are selected from a group consisting of radicals of natural or unnatural amino acids, such as, for example:
glycine, alanine, valine, leucine, isoleucine, serine, threonine, cysteine, methionine, phenylalanine, tyrosine, histidine, tryptophan, lysine, ornithine, aspartic acid, glutamic acid, asparagine, glutamine, arginine, proline, hydroxyproline, sarcosine, aminoisobutyric acid, dehydroamino acids, such as, for example, dehydroalanine, dehydro-α-aminobutyric acid, other unnatural amino acids, such as phenylglycine, 4-nitrophenylalanine, 3-nitrophenylalanine, 2-nitrophenylalanine, 2-, 3- or 4-aminophenylalanine, 3,4-dichlorophenylalanine, 4-iodophenylalanine, 4-methoxyphenylalanine, 1-triazolylalanine, 2-pyridylalanine, 3-pyridylalanine, 4-pyridylalanine, 1-naphthylalanine or 2-naphthylalanine, if appropriate having protective groups, where Q stereochemically uniformly exists in the L form or the D form,
G and Q can be linked to each other via an alkyl chain [(-CH₂)ₙ- where n = 1, 2],
G represents -NH-, -NR-, -O- or -S-,
M represents -CH₂-, -CO-, -SO₂-, -SO- or -CS-,
L represents (CH₂)ₚ where p = 0, 1, 2, -CHR- or -CRR'-,
K represents carrier system, reporter ligand, H or solubilizing group,
N represents carrier system, reporter ligand, OH or solubilizing group,
s represents a value from 1 to 30 and
R and R' represent H, OH, alkyl, aralkyl or aryl.

2. Compounds of the general formula (I) according to Claim 1, in which
A represents -CO-, -CHR- or -CRR'-,
B independently of one another is selected from a group consisting of:
(C₁-C₄)-alkanoyl, DNA intercalators, aromatic radicals, heterocyclic radicals, naturally occurring nucleic bases, such as thymine, uracil, cytosine, adenine, guanine, hypoxanthine, inosine and halogenated precursors of these nucleic bases, and protected derivatives of the nucleic bases having the protective groups which are customary in nucleotide or peptide chemistry,
C represents -CH- or -CR-, where C can uniformly exist in the S or R configuration,
D represents -NH-, -CH₂-, -CHR- or -CRR'-,
E represents -NR-, -NH-, -CHR-, -CRR'- or -O-,
A and E can be linked to each other via an alkyl chain [(-CH₂)ₙ- where n = 1, 2],
F represents -CH₂-, -CO-, -SO₂-, -SO- or -CS-,
Q represents (-CR¹R²)ₘ-, where m is 0, 1 or 2 and R¹ and R² independently of one another are selected from a group consisting of radicals of natural or unnatural amino acids, such as, for example:
glycine, alanine, valine, leucine, isoleucine, serine, threonine, cysteine, methionine, phenylalanine, tyrosine, histidine, tryptophan, lysine, ornithine, aspartic acid, glutamic acid, asparagine, glutamine, arginine, proline, hydroxyproline, sarcosine, aminoisobutyric acid, dehydroamino acids, such as, for example, dehydroalanine, dehydro-a-aminobutyric acid, other unnatural amino acids, such as phenylglycine, 4-nitrophenylalanine, 3-nitrophenylalanine, 2-nitrophenylalanine, 1 -naphthylalanine or 2-naphthylalanine, if appropriate having protective groups, where Q stereochemically uniformly exists in the L form or the D form,
G and Q can be linked to each other via an alkyl chain [(-CH₂)ₙ- where n = 1, 2],
G represents -NH-, -NR- or -O-,
M represents -CH₂-, -CO-, -SO₂- or -CS-,
L represents (CH₂)ₚ where p = 0, 1, 2, -CHR- or -CRR'-,
K represents carrier system, reporter ligand, H or solubilizing group,
N represents carrier system, reporter ligand, OH or solubilizing group,
s represents a value from 1 to 30 and
R and R' represent H, OH, alkyl, aralkyl or aryl.

3. Compounds of the general formula (I) according to Claim 1, in which
A represents -CO-, -CHR- or -CRR'-,
B independently of one another is selected from a group consisting of:
(C₁-C₄)-alkanoyl, DNA intercalators, aromatic radicals, heterocyclic radicals, naturally occurring nucleic bases, such as thymine, uracil, cytosine, adenine, guanine, hypoxanthine, inosine and halogenated precursors of these nucleic bases, and protected derivatives of the nucleic bases having the protective groups which are customary in nucleotide or peptide chemistry,
C represents -CH- or -CR-, where C can uniformly exist in the S or R configuration,
D represents -NH-, -CH₂-, -CHR- or -CRR'-,
E represents -NR-, -NH- or -CHR-,
A and E can be linked to each other via an alkyl chain [(-CH₂)ₙ- where n = 1, 2],
F represents -CH₂-, -CO- or -CS-,
Q represents (-CR¹R²)ₘ-, where m is 0, 1 or 2 and R¹ and R² independently of one another are selected from a group consisting of radicals of natural or unnatural amino acids, such as, for example:
glycine, alanine, valine, leucine, isoleucine, serine, threonine, cysteine, methionine, phenylalanine, tyrosine, histidine, tryptophan, lysine, ornithine, aspartic acid, glutamic acid, asparagine, glutamine, arginine, proline, hydroxyproline, sarcosine, aminoisobutyric acid, dehydroamino acids, such as, for example, dehydroalanine, dehydro-a-aminobutyric acid, other unnatural amino acids, such as phenylglycine, if appropriate having protective groups, where L stereochemically uniformly exists in the L form or the D form,
G and Q can be linked to each other via an alkyl chain [(-CH₂)ₙ- where n = 1, 2],
G represents -NH-, -NR- or -O-,
M represents -CH₂-, -CO- or -CS-,
L represents (CH₂)ₚ where p = 0, 1, 2, -CHR- or -CRR'-,
K represents carrier system, reporter ligand, H or solubilizing group,
N represents carrier system, reporter ligand, OH or solubilizing group,
s represents a value from 1 to 30 and
R and R' represent H, OH, alkyl, aralkyl or aryl.

4. Compounds of the general formula (II) 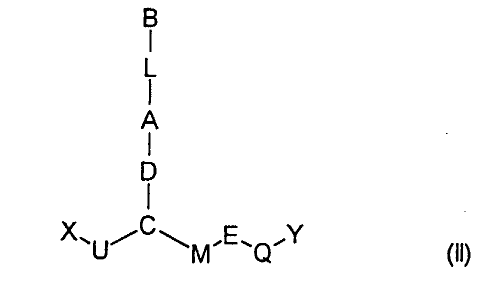 in which
A represents -CO-, -CHR- or -CRR'-,
B independently of one another is selected from a group consisting of: (C₁-C₄)-alkanoyl, DNA intercalators, aromatic radicals, heterocyclic radicals, naturally occurring nucleic bases, such as thymine, uracil, cytosine, adenine, guanine, hypoxanthine, inosine or derivatives obtained from these by chemical modification, and halogenated precursors of these nucleic bases, and protected derivatives of these nucleic bases having the protective groups which are customary in nucleotide or peptide chemistry,
C represents -CH- or -CR-, where C can uniformly exist in the S or R configuration,
D represents -NH-, -CH₂-, -CHR- or -CRR'-,
E represents -NH-, -NR-, -CHR-, -CRR'-, -O- or -S-,
Q represents (-CR¹R²)ₘ-, where m is 0, 1 or 2 and R¹ and R² independently of one another are selected from a group consisting of radicals of natural or unnatural amino acids, such as, for example: glycine, alanine, valine, leucine, isoleucine, serine, threonine, cysteine, methionine, phenylalanine, tyrosine, histidine, tryptophan, lysine, ornithine, aspartic acid, glutamic acid, asparagine, glutamine, arginine, proline, hydroxyproline, sarcosine, aminoisobutyric acid, dehydroamino acids, such as, for example, dehydroalanine, dehydro-α-aminobutyric acid, other unnatural amino acids, such as phenylglycine, 4-nitrophenylalanine, 3-nitrophenylalanine, 2-nitrophenylalanine, 2-, 3- or 4-aminophenylalanine, 3,4-dichlorophenylalanine, 4-iodophenylalanine, 4-methoxyphenylalanine, 1-triazolylalanine, 2-pyridylalanine, 3-pyridylalanine, 4-pyridylalanine, 1-naphthylalanine or 2-naphthylalanine, if appropriate having protective groups, where Q stereochemically uniformly exists in the L form or the D form,
E and Q can be linked to each other via an alkyl chain [(-CH₂)ₙ- where n = 1, 2],
M represents -CH₂-, -CO-, -SO₂-, -SO- or -CS-,
L represents (CH₂)ₚ where p = 0, 1, 2, -CHR- or -CRR'-,
U represents -NH- or -NR-,
X represents any protective group known from peptide chemistry, H or any natural or unnatural amino acid in protected or unprotected form,
Y represents COOH, CSOH, CH₂OH or COOR" where R" is any protective group from peptide chemistry, carrier, reporter ligand or solubilizing group, and
R and R' represent H, OH, alkyl, aralkyl or aryl.

5. Compounds of the formula (II) according to Claim 4, in which
A represents -CO-, -CHR- or -CRR'-,
B independently of one another is selected from a group consisting of: (C₁-C₄)-alkanoyl, DNA intercalators, aromatic radicals, heterocyclic radicals, naturally occurring nucleic bases, such as thymine, uracil, cytosine, adenine, guanine, hypoxanthine, inosine and halogenated precursors of these nucleic bases, and protected derivatives of these nucleic bases having the protective groups which are customary in nucleotide or peptide chemistry,
C represents -CH- or -CR-, where C can uniformly exist in the S or R configuration,
D represents -NH-, -CH₂-, -CHR- or -CRR'-,
E represents -NR-, -NH-, -CHR-, -CRR'- or -O-,
Q represents (-CR¹R²)ₘ-, where m is 0, 1 or 2 and R¹ and R² independently of one another are selected from a group consisting of radicals of natural or unnatural amino acids, such as, for example: glycine, alanine, valine, leucine, isoleucine, serine, threonine, cysteine, methionine, phenylalanine, tyrosine, histidine, tryptophan, lysine, ornithine, aspartic acid, glutamic acid, asparagine, glutamine, arginine, proline, hydroxyproline, sarcosine, aminoisobutyric acid, dehydroamino acids, such as, for example, dehydroalanine, dehydro-α-aminobutyric acid, other unnatural amino acids, such as phenylglycine, 4-nitrophenylalanine, 3-nitrophenylalanine, 2-nitrophenylalanine, 1-naphthylalanine or 2-naphthylalanine, if appropriate having protective groups, where Q stereochemically uniformly exists in the L form or the D form,
E and Q can be linked to each other via an alkyl chain [(-CH₂)ₙ- where n =1, 2],
M represents -CH₂-, -CO-, -SO₂-, -SO- or -CS-,
L represents (CH₂)ₚ where p = 0, 1, 2, -CHR- or -CRR'-,
U represents -NH- or -NR-,
X represents any protective group known from peptide chemistry, H or any natural or unnatural amino acid in protected or unprotected form,
Y represents COOH, CSOH, CH₂OH or COOR" where R" is any protective group from peptide chemistry, carrier, reporter ligand or solubilizing group, and
R and R' represent H, OH, alkyl, aralkyl or aryl.

6. Compounds of the general formula (II) according to Claim 4, in which
A represents -CO-, -CHR- or -CRR'-,
B independently of one another is selected from a group consisting of: (C₁-C₄)-alkanoyl, DNA intercalators, aromatic radicals, heterocyclic radicals, naturally occurring nucleic bases, such as thymine, uracil, cytosine, adenine, guanine, hypoxanthine, inosine and halogenated precursors of these nucleic bases, and protected derivatives of these nucleic bases having the protective groups which are customary in nucleotide or peptide chemistry,
C represents -CH- or -CR-, where C can uniformly exist in the S or R configuration,
D represents -NH-, -CH₂-, -CHR- or -CRR'-,
E represents -NR-, -NH-, -CHR- or -O-,
Q represents (-CR¹R²)ₘ-, where m is 0, 1 or 2 and R¹ and R² independently of one another are selected from a group consisting of radicals of natural or unnatural amino acids, such as, for example: glycine, alanine, valine, leucine, isoleucine, serine, threonine, cysteine, methionine, phenylalanine, tyrosine, histidine, tryptophan, lysine, ornithine, aspartic acid, glutamic acid, asparagine, glutamine, arginine, proline, hydroxyproline, sarcosine, aminoisobutyric acid, dehydroamino acids, such as, for example, dehydroalanine, dehydro-α-aminobutyric acid, other unnatural amino acids, such as phenylglycine, if appropriate having protective groups, where Q stereochemically uniformly exists in the L form or the D form,
E and Q can be linked to each other via an alkyl chain [(-CH₂)ₙ- where n = 1, 2],
M represents -CH₂-, -CO- or -CS-,
L represents (CH₂)ₚ where p = 0, 1, 2, -CHR- or -CRR'-,
U represents -NH- or -NR-,
X represents any protective group known from peptide chemistry, H or any natural or unnatural amino acid in protected or unprotected form
Y represents COOH, CSOH, CH₂OH or COOR" where R" is any protective group from peptide chemistry, carrier, reporter ligand or solubilizing group and
R and R' represent H, OH, alkyl, aralkyl or aryl.

7. Compounds of the general formula (III) 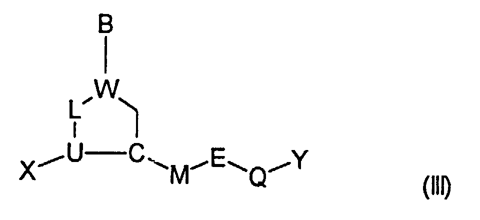 in which
B independently of one another is selected from a group consisting of: (C₁-C₄)-alkanoyl, DNA intercalators, aromatic radicals, heterocyclic radicals, naturally occurring nucleic bases, such as thymine, uracil, cytosine, adenine, guanine, hypoxanthine, inosine or derivatives obtained from these by chemical modification, and halogenated precursors of these nucleic bases, and protected derivatives of these nucleic bases having the protective groups which are customary in nucleotide or peptide chemistry,
C represents -CH- or -CR-, where C can uniformly exist in the S or R configuration,
E represents -NR-, -CHR-, -CRR'-, -O- or -S-,
Q represents (-CR¹R²-)ₘ, where m is 0, 1 or 2 and R¹ and R² independently of one another are selected from a group consisting of radicals of natural or unnatural amino acids, such as, for example: glycine, alanine, valine, leucine, isoleucine, serine, threonine, cysteine, methionine, phenylalanine, tyrosine, histidine, tryptophan, lysine, ornithine, aspartic acid, glutamic acid, asparagine, glutamine, arginine, proline, hydroxyproline, sarcosine, aminoisobutyric acid, dehydroamino acids, such as, for example, dehydroalanine, dehydro-α-aminobutyric acid, other unnatural amino acids, such as phenylglycine, 4-nitrophenylalanine, 3-nitrophenylalanine, 2-nitrophenylalanine, 2-, 3- or 4-aminophenylalanine, 3,4-dichlorophenylalanine, 4-iodophenylalanine, 4-methoxyphenylalanine, 1-triazolylalanine, 2-pyridylalanine, 3-pyridylalanine, 4-pyridylalanine, 1-naphthylalanine or 2-naphthylalanine, if appropriate having protective groups, where Q stereochemically uniformly exists in the L form or the D form,
E and Q can be linked to each other via an alkyl chain [(-CH₂)ₙ- where n = 1, 2],
M represents -CH₂-, -CO-, -SO₂-, -SO- or -CS-,
L represents CH₂,
U represents -NH- or -NR-,
X represents any protective group known from peptide chemistry, H or any natural or unnatural amino acid in protected or unprotected form,
Y represents COOH, CSOH, CH₂OH or COOR" where R" is any protective group from peptide chemistry, carrier, reporter ligand or solubilizing group,
W represents a chiral C atom which can uniformly exist in the S or R configuration and
R and R' represent H, OH, alkyl, aralkyl or aryl.

8. Compounds of the formula (III) according to Claim 7, in which
B independently of one another is selected from a group consisting of: -H, -OH, (C₁-C₄)-alkanoyl, DNA intercalators, aromatic radicals, heterocyclic radicals, naturally occurring nucleic bases, such as thymine, uracil, cytosine, adenine, guanine, hypoxanthine, inosine and halogenated precursors of these nucleic bases, and protected derivatives of these nucleic bases having the protective groups which are customary in nucleotide or peptide chemistry,
C represents -CH- or -CR-, where C can uniformly exist in the S or R configuration,
E represents -NR-, -NH-, -CHR-, -CRR'- or -O-,
Q represents (-CR¹R²)ₘ-, where m is 0, 1 or 2 and R¹ and R² independently of one another are selected from a group consisting of radicals of natural or unnatural amino acids, such as, for example: glycine, alanine, valine, leucine, isoleucine, serine, threonine, cysteine, methionine, phenylalanine, tyrosine, histidine, tryptophan, lysine, ornithine, aspartic acid, glutamic acid, asparagine, glutamine, arginine, proline, hydroxyproline, sarcosine, aminoisobutyric acid, dehydroamino acids, such as, for example, dehydroalanine, dehydro-α-aminobutyric acid, other unnatural amino acids, such as phenylglycine, 4-nitrophenylalanine, 3-nitrophenylalanine, 2-nitrophenylalanine, 1-naphthylalanine or 2-naphthylalanine, if appropriate having protective groups, where Q stereochemically uniformly exists in the L form or the D form,
E and Q can be linked to each other via an alkyl chain [(-CH₂)ₙ- where n = 1, 2],
M represents -CH₂-, -CO-, -SO₂-, -SO- or -CS-,
L represents -CH₂-,
U represents -NH- or -NR-,
X represents any protective group known from peptide chemistry, H or any natural or unnatural amino acid in protected or unprotected form,
Y represents COOH, CSOH, CH₂OH or COOR" where R" is any protective group from peptide chemistry, carrier, reporter ligand or solubilizing group,
W represents a chiral C atom which can uniformly exist in the S or R configuration, and
R and R' represent H, OH, alkyl, aralkyl or aryl.

9. Compounds of the formula (III) according to Claim 7, in which
B independently of one another is selected from a group consisting of: -H, -OH, (C₁-C₄)-alkanoyl, DNA intercalators, aromatic radicals, heterocyclic radicals, naturally occurring nucleic bases, such as thymine, uracil, cytosine, adenine, guanine, hypoxanthine, inosine and halogenated precursors of these nucleic bases, and protected derivatives of these nucleic bases having the protective groups which are customary in nucleotide or peptide chemistry,
C represents -CH- or -CR-, where C can uniformly exist in the S or R configuration,
E represents -NR-, -NHR-, -CHR- or -O-,
Q represents (-CR¹R²)ₘ-, where m is 0, 1 or 2 and R¹ and R² independently of one another are selected from a group consisting of radicals of natural or unnatural amino acids, such as, for example: glycine, alanine, valine, leucine, isoleucine, serine, threonine, cysteine, methionine, phenylalanine, tyrosine, histidine, tryptophan, lysine, ornithine, aspartic acid, glutamic acid, asparagine, glutamine, arginine, proline, hydroxyproline, sarcosine, aminoisobutyric acid, dehydroamino acids, such as, for example, dehydroalanine, dehydro-α-aminobutyric acid, other unnatural amino acids, such as phenylglycine, if appropriate having protective groups, where Q stereochemically uniformly exists in the L form or the D form, E and Q can be linked to each other via an alkyl chain [(-CH₂)ₙ- where n = 1, 2],
M represents -CH₂-, -CO- or -CS-,
L represents -CH₂-,
U represents -NH- or -NR-,
X represents any protective group known from peptide chemistry, H or any natural or unnatural amino acid in protected or unprotected form,
Y represents COOH, CSOH, CH₂OH or COOR" where R" is any protective group from peptide chemistry, carrier, reporter ligand or solubilizing group,
W represents a chiral C atom which can uniformly exist in the S or R configuration and
R and R' represent H, OH, alkyl, aralkyl or aryl.

10. Medicaments containing one or more compounds of Claims 1 to 9.

## Revendications

1. Composés de formule générale (I) : 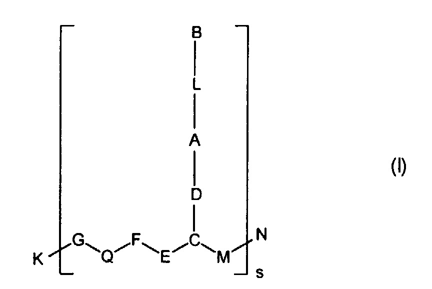 dans laquelle :
A représente -CO-, CHR-, -CRR'-;
B est choisi indépendamment l'un de l'autre, parmi un groupe constitué de :
alcanoyle en C₁-C₄, intercalants d'ADN, restes aromatiques, restes hétérocycliques, bases nucléiques d'origine naturelle comme thymine, uracile, cytosine, adénine, guanine, hypoxanthine, inosine ou par modification chimique des composés dérivés de ceux-ci ainsi que les géniteurs halogénés de ces bases nucléiques, ainsi que les dérivés protégés des bases nucléiques avec les groupes protecteurs classiques de la chimie nucléotidique ou peptidique;
C représente -CH-, -CR-, où C peut avoir la configuration S ou R, de manière homogène;
D représente -NH-, -CH₂-, -CHR-, -CRR'-;
E représente -NH-, -NR-, -CHR-, -CRR'-, -O-, -S-,
A et E peuvent être liés l'un à l'autre par une chaîne alcoyle [(-CH₂)ₙ-, avec n = 1, 2];
F représente -CH₂-, -CO-, -SO₂-, -SO-, -CS-;
Q représente (-CR¹R²)ₘ- avec m = 0, 1, 2 et R¹, R² sont choisis indépendamment l'un de l'autre, parmi un groupe constitué des restes d'acide aminé naturel ou non naturel, comme par exemple :
glycine, alanine, valine, leucine, isoleucine, sérine, thréonine, cystéine, méthionine, phénylalanine, tyrosine, histidine, tryptophane, lysine, ornithine, acide aspartique, acide glutamique, asparagine, glutamine, arginine, proline, hydroxyproline, sarcosine, acide aminoisobutyrique, les acides déshydroaminés comme par exemple, la déshydroalanine, l'acide déshydro-α-aminobutyrique, d'autres acides aminés non naturels comme la phénylglycine, la 4-nitrophénylalanine, la 3-nitrophénylalanine, la 2-nitrophénylalanine, la 2-, 3- ou 4-aminophénylalanine, la 3,4-dichlorophénylalanine, la 4-iodophénylalanine, la 4-méthoxyphénylalanine, la 1-triazolylalanine, la 2-pyridylalanine, la 3-pyridylalanine, la 4-pyridylalanine, la 1-naphtylalanine ou la 2-naphtylalanine, facultativement avec des groupes protecteurs, stéréochimiquement homogènes sous la forme L ou la forme D;
G et Q peuvent être reliés l'un à l'autre par une chaîne alcoyle [(-CH₂)ₙ- avec n = 1, 2];
G représente -NH-, -NR-, -O-, -S-;
M représente -CH₂-, -CO-, -SO₂-, -SO-, -CS-;
L représente (CH₂)ₚ avec p = 0, 1, 2, -CHR-, -CRR'-;
K représente un système porteur, un ligand reporter, H, un groupe agissant sur la solubilité;
N représente un système porteur, un ligand reporter, OH, un groupe agissant sur la solubilité, et
s représente une valeur allant de 1 à 30, et R et R' représentent H, OH, alcoyle, aralcoyle ou aryle.

2. Composés de formule générale (I) suivant la revendication 1, dans laquelle :
A représente -CO-, -CHR-, -CRR'-;
B est choisi indépendamment l'un de l'autre parmi un groupe constitué de :
alcanoyle en C₁-C₄, intercalants d'ADN, restes aromatiques, restes hétérocycliques, bases nucléiques d'origine naturelle comme thymine, uracile, cytosine, adénine, guanine, hypoxanthine, inosine ainsi que les géniteurs halogénés de ces bases nucléiques, ainsi que les dérivés protégés des bases nucléiques avec les groupes protecteurs classiques de la chimie nucléotidique ou peptidique;
C représente -CH-, -CR-, où C peut avoir la configuration S ou R, de manière homogène;
D représente -NH-, -CH₂-, -CHR-, -CRR'-;
E représente -NR-, -NH-, -CHR-, -CRR'-, -O-;
A et E peuvent être reliés l'un avec l'autre par une chaîne alcoyle [-(CH₂)ₙ- avec n = 1, 2];
F représente -CH₂-, -CO-, -SO₂-, -SO-, -CS-;
Q représente (-CR¹R²)ₘ- avec m = 0, 1, 2 et R¹, R² sont choisis indépendamment l'un de l'autre parmi un groupe consistant en des restes d'acides aminés naturels ou non naturels comme par exemple :
glycine, alanine, valine, leucine, isoleucine, sérine, thréonine, cystéine, méthionine, phénylalanine, tyrosine, histidine, tryptophane, lysine, ornithine, acide aspartique, acide glutamique, asparagine, glutamine, arginine, proline, hydroxyproline, sarcosine, acide aminoisobutyrique, les acides déshydroaminés comme par exemple, la déshydroalanine, l'acide déshydro-α-aminobutyrique, d'autres acides aminés non naturels comme la phénylglycine, la 4-nitrophénylalanine, la 3-nitrophénylalanine, la 2-nitrophénylalanine, la 1-naphtylalanine ou 2-naphtylalanine, facultativement avec des groupes protecteurs, stéréochimiquement homogènes sous la forme D ou la forme L;
G et Q peuvent être reliés l'un à l'autre par une chaîne alcoyle [(-CH₂)ₙ- avec n = 1, 2];
G représente -NH-, -NR-, -O-;
M représente -CH₂-, -CO-, -SO₂-, -CS-;
L représente (CH₂)ₚ avec p = 0, 1, 2, -CHR-, -CRR'-;
K représente un système porteur, un ligand reporter, H, un groupe agissant sur la solubilité;
N représente un système porteur, un ligand reporter, OH, un groupe agissant sur la solubilité, et
s représente une valeur allant de 1 à 30, et R et R' représentent H, OH, alcoyle, aralcoyle ou aryle.

3. Composés de formule générale (I) suivant la revendication 1, dans laquelle :
A représente -CO-, CHR-, -CRR'-;
B est choisi indépendamment l'un de l'autre, parmi un groupe constitué de :
alcanoyle en C₁-C₄, intercalants d'ADN, restes aromatiques, restes hétérocycliques, bases nucléiques d'origine naturelle comme thymine, uracile, cytosine, adénine, guanine, hypoxanthine, inosine ainsi que les géniteurs halogénés de ces bases nucléiques, ainsi que les dérivés protégés des bases nucléiques avec les groupes protecteurs classiques de la chimie nucléotidique ou peptidique;
C représente -CH-, -CR-, où C peut avoir la configuration S ou R, de manière homogène;
D représente -NH-, -CH₂-, -CHR-, -CRR'-;
E représente -NR-, -NH-, -CHR-;
A et E peuvent être liés l'un à l'autre par une chaîne alcoyle [(-CH₂)ₙ-, avec n = 1, 2];
F représente -CH₂-, -CO-, -CS-;
Q représente (-CR¹R²)ₘ- avec m = 0, 1, 2 et R¹, R² sont choisis indépendamment l'un de l'autre, parmi un groupe constitué des restes d'acide aminé naturel ou non naturel, comme par exemple :
glycine, alanine, valine, leucine, isoleucine, sérine, thréonine, cystéine, méthionine, phénylalanine, tyrosine, histidine, tryptophane, lysine, ornithine, acide aspartique, acide glutamique, asparagine, glutamine, arginine, proline, hydroxyproline, sarcosine, acide aminoisobutyrique, les acides déshydroaminés comme par exemple, la déshydroalanine, l'acide déshydro-α-aminobutyrique, d'autres acides aminés non naturels comme la phénylglycine, facultativement avec des groupes protecteurs, stéréochimiquement homogènes sous la forme L ou la forme D;
G et Q peuvent être reliés l'un à l'autre par une chaîne alcoyle [(-CH₂)ₙ- avec n = 1, 2];
G représente -NH-, -NR-, -O-;
M représente -CH₂-, -CO-, -CS-;
L représente (CH₂)ₚ avec p = 0, 1, 2, -CHR-, -CRR'-;
K représente un système porteur, un ligand reporter, H, un groupe agissant sur la solubilité;
N représente un système porteur, un ligand reporter, OH, un groupe agissant sur la solubilité, et
s représente une valeur allant de 1 à 30, et R et R' représentent H, OH, alcoyle, aralcoyle ou aryle.

4. Composés de formule générale (II) 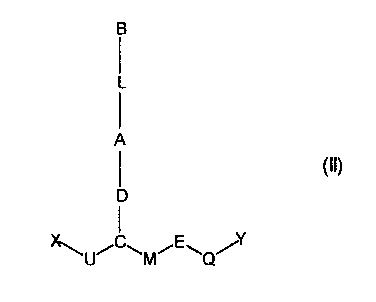 dans laquelle :
A représente -CO-, CHR-, -CRR'-;
B est choisi indépendamment l'un de l'autre, parmi un groupe constitué de :
alcanoyle en C₁-C₄, intercalants d'ADN, restes aromatiques, restes hétérocycliques, bases nucléiques d'origine naturelle comme thymine, uracile, cytosine, adénine, guanine, hypoxanthine, inosine ou par modification chimique des composés dérivés de ceux-ci ainsi que les géniteurs halogénés de ces bases nucléiques, ainsi que les dérivés protégés de ces bases nucléiques avec les groupes protecteurs classiques de la chimie nucléotidique ou peptidique;
C représente -CH-, -CR-, où C peut avoir la configuration S ou R, de manière homogène;
D représente -NH-, -CH₂-, -CHR-, -CRR'-;
E représente -NH-, -NR-, -CHR-, -CRR'-, -O-, -S-,
Q représente (-CR¹R²)ₘ- avec m = 0, 1, 2 et R¹, R² sont choisis indépendamment l'un de l'autre, parmi un groupe constitué des restes d'acide aminé naturel ou non naturel, comme par exemple :
glycine, alanine, valine, leucine, isoleucine, sérine, thréonine, cystéine, méthionine, phénylalanine, tyrosine, histidine, tryptophane, lysine, ornithine, acide aspartique, acide glutamique, asparagine, glutamine, arginine, proline, hydroxyproline, sarcosine, acide aminoisobutyrique, les acides déshydroaminés comme par exemple, la déshydroalanine, l'acide déshydro-α-aminobutyrique, d'autres acides aminés non naturels comme la phénylglycine, la 4-nitrophénylalanine, la 3-nitrophénylalanine, la 2-nitrophénylalanine, la 2-, 3-ou 4-aminophénylalanine, la 3,4-dichlorophénylalanine, la 4-iodophénylalanine, la 4-méhtoxyphénylalanine, la 1-triazolylalanine, la 2-pyridylalanine, la 3-pyridilalanine, la 4-pyridylalanine, la 1-naphtylalanine ou la 2-naphtylalanine, facultativement avec des groupes protecteurs, stéréochimiquement homogènes sous la forme L ou la forme D;
E et Q peuvent être reliés l'un à l'autre par une chaîne alcoyle [(-CH₂)ₙ- avec n = 1, 2];
M représente -CH₂-, -CO-, -SO₂-, -SO-, -CS-;
L représente (CH₂)ₚ avec p = 0, 1, 2, -CHR-, -CRR'-;
U représente -NH-, -NR-;
X représente un groupe protecteur quelconque connu dans la chimie peptidique, H ou un acide aminé quelconque naturel ou non naturel sous forme protégée ou non protégée, et
Y représente COOH, CSOH, CH₂OH, COOR" avec R" = un groupe protecteur quelconque de la chimie peptidique, un support, un ligand reporter, un groupe agissant sur la solubilité,
R et R' représentent H, OH, alcoyle, aralcoyle ou aryle.

5. Composés de formule générale (II) suivant la revendication 4, dans laquelle :
A représente -CO-, CHR-, -CRR'-;
B est choisi indépendamment l'un de l'autre, parmi un groupe constitué de :
alcanoyle en C₁-C₄, intercalants d'ADN, restes aromatiques, restes hétérocycliques, bases nucléiques d'origine naturelle comme thymine, uracile, cytosine, adénine, guanine, hypoxanthine, inosine ainsi que les géniteurs halogénés de ces bases nucléiques, ainsi que les dérivés protégés de ces bases nucléiques avec les groupes protecteurs classiques de la chimie nucléotidique ou peptidique;
C représente -CH-, -CR-, où C peut avoir la configuration S ou R, de manière homogène;
D représente -NH-, -CH₂-, -CHR-, -CRR'-;
E représente -NR-, -NH-, -CHR-, -CRR'-, -O-;
Q représente (-CR¹R²)ₘ- avec m = 0, 1, 2 et R¹, R² sont choisis indépendamment l'un de l'autre, parmi un groupe constitué des restes d'acide aminé naturel ou non naturel, comme par exemple :
glycine, alanine, valine, leucine, isoleucine, sérine, thréonine, cystéine, méthionine, phénylalanine, tyrosine, histidine, tryptophane, lysine, ornithine, acide aspartique, acide glutamique, asparagine, glutamine, arginine, proline, hydroxyproline, sarcosine, acide aminoisobutyrique, les acides déshydroaminés comme par exemple, la déshydroalanine, l'acide déshydro-α-aminobutyrique, d'autres acides aminés non naturels comme la phénylglycine, la 4-nitrophénylalanine, la 3-nitrophénylalanine, la 2-nitrophénylalanine, la 1-naphtylalanine ou la 2-naphtylalanine facultativement avec des groupes protecteurs, stéréochimiquement homogènes sous la forme L ou la forme D;
E et Q peuvent être reliés l'un à l'autre par une chaîne alcoyle [(-CH₂)ₙ- avec n = 1, 2];
M représente -CH₂-, -CO-, -SO₂-, -SO-, -CS-;
L représente (CH₂)ₚ avec p = 0, 1, 2, -CHR-, -CRR'-;
U représente -NH-, -NR-;
X représente un groupe protecteur quelconque connu dans la chimie peptidique, H ou un acide aminé quelconque naturel ou non naturel sous forme protégée ou non protégée, et
Y représente COOH, CSOH, CH₂OH, COOR" avec R" = un groupe protecteur quelconque de la chimie peptidique, un support, un ligand reporter, un groupe agissant sur la solubilité, et
R et R' représentent H, OH, alcoyle, aralcoyle ou aryle.

6. Composés de formule générale (II) suivant la revendication 4, dans laquelle :
A représente -CO-, CHR-, -CRR'-;
B est choisi indépendamment l'un de l'autre, parmi un groupe constitué de :
alcanoyle en C₁-C₄, intercalants d'ADN, restes aromatiques, restes hétérocycliques, bases nucléiques d'origine naturelle comme thymine, uracile, cytosine, adénine, guanine, hypoxanthine, inosine ainsi que les géniteurs halogénés de ces bases nucléiques, ainsi que les dérivés protégés de ces bases nucléiques avec les groupes protecteurs classiques de la chimie nucléotidique ou peptidique;
C représente -CH-, -CR-, où C peut avoir la configuration S ou R, de manière homogène;
D représente -NH-, -CH₂-, -CHR-, -CRR'-;
E représente -NR-, -NH-, -CHR-, -O-;
Q représente (-CR¹R²)ₘ- avec m = 0, 1, 2 et R¹, R² sont choisis indépendamment l'un de l'autre, parmi un groupe constitué des restes d'acide aminé naturel ou non naturel, comme par exemple :
glycine, alanine, valine, leucine, isoleucine, sérine, thréonine, cystéine, méthionine, phénylalanine, tyrosine, histidine, tryptophane, lysine, ornithine, acide aspartique, acide glutamique, asparagine, glutamine, arginine, proline, hydroxyproline, sarcosine, acide aminoisobutyrique, les acides déshydroaminés comme par exemple, la déshydroalanine, l'acide déshydro-α-aminobutyrique, d'autres acides aminés non naturels comme la phénylglycine, facultativement avec des groupes protecteurs, stéréochimiquement homogènes sous la forme L ou la forme D;
E et Q peuvent être reliés l'un à l'autre par une chaîne alcoyle [(-CH₂)ₙ- avec n = 1, 2];
M représente -CH₂-, -CO-, -CS-;
L représente (CH₂)ₚ avec p = 0, 1, 2, -CHR-, -CRR'-;
U représente -NH-, -NR-;
X représente un groupe protecteur quelconque connu dans la chimie peptidique, H ou un acide aminé quelconque naturel ou non naturel sous forme protégée ou non protégée,
Y représente COOH, CSOH, CH₂OH, COOR" avec R" = un groupe protecteur quelconque de la chimie peptidique, un support, un ligand reporter, un groupe agissant sur la solubilité, et
R et R' représentent H, OH, alcoyle, aralcoyle ou aryle.

7. Composés de formule générale (III) : 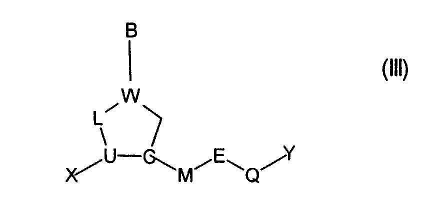 dans laquelle :
B est choisi indépendamment l'un de l'autre, parmi un groupe consistant en :
alcanoyle en C₁-C₄, intercalants d'ADN, restes aromatiques, restes hétérocycliques, bases nucléiques d'origine naturelle comme thymine, uracile, cytosine, adénine, guanine, hypoxanthine, inosine ou par modification chimique des composés dérivés de ceux-ci, ainsi que les géniteurs halogénés de ces bases nucléiques, ainsi que les dérivés protégés de ces bases nucléiques avec les groupes protecteurs classiques de la chimie nucléotidique ou peptidique;
C représente -CH-, -CR- où C peut avoir la configuration S ou R, de manière homogène;
E représente -NR-, -CHR-, -CRR'-, -O-, -S-;
Q représente (-CR¹R²-)ₘ avec m = 0, 1, 2 et R¹, R² sont choisis indépendamment l'un de l'autre, parmi un groupe consistant en les restes d'acides aminés naturels ou non naturels, comme par exemple : glycine, alanine, valine, leucine, isoleucine, sérine, thréonine, cystéine, méthionine, phénylalanine, tyrosine, histidine, tryptophane, lysine, ornithine, acide aspartique, acide glutamique, asparagine, glutamine, arginine, proline, hydroxyproline, sarcosine, acide aminoisobutyrique, les acides déshydroaminés comme par exemple, la déshydroalanine, l'acide déshydro-a-aminobutyrique, d'autres acides aminés non naturels comme la phénylglycine, la 4-nitrophénylalanine, la 3-nitrophénylalanine, la 2-nitrophénylalanine, la 2-, 3- ou 4-aminophénylalanine, la 3,4-dichlorophénylalanine, la 4-iodophénylalanine, la 4-méthoxyphénylalanine, la 1-triazolylalanine, la 2-pyridylalanine, la 3-pyridylalanine, la 4-pyridylalanine, la 1-naphtylalanine ou la 2-naphtylalanine, facultativement avec des groupes protecteurs, stéréochimiquement homogènes sous la forme L ou la forme D;
E et Q peuvent être reliés l'un avec l'autre par une chaîne alcoyle [(-CH₂-)ₙ avec n = 1, 2];
M représente -CH₂-, -CO-, -SO₂-, -SO-, -CS-;
L représente -CH₂-;
U représente -NH-, -NR-;
X représente un groupe protecteur quelconque, connu dans la chimie peptidique, H ou un acide aminé quelconque naturel ou non naturel, sous forme protégée ou non protégée;
Y représente COOH, CSOH, CH₂OH, COOR" avec R" = un groupe protecteur quelconque de la chimie peptidique, un support, un ligand reporter, un groupe agissant sur la solubilité;
W représente un atome C chiral, lequel peut avoir la configuration, de manière homogène, S ou R, et
R et R' représentent H, OH, alcoyle, aralcoyle ou aryle.

8. Composés de formule générale (III) suivant la revendication 7, dans laquelle :
B est choisi indépendamment l'un de l'autre, parmi un groupe consistant en :
H, OH, alcanoyle en C₁-C₄, intercalants d'ADN, restes aromatiques, restes hétérocycliques, bases nucléiques d'origine naturelle comme thymine, uracile, cytosine, adénine, guanine, hypoxanthine, inosine ainsi que les géniteurs halogénés de ces bases nucléiques, ainsi que les dérivés protégés de ces bases nucléiques avec les groupes protecteurs classiques de la chimie nucléotidique ou peptidique;
C représente -CH-, -CR- où C peut avoir la configuration S ou R, de manière homogène;
E représente -NR-, -NH-, -CHR-, -CRR'-, -O-;
Q représente (-CR¹R²-)ₘ avec m = 0, 1, 2 et R¹, R² sont choisis indépendamment l'un de l'autre, parmi un groupe consistant en les restes d'acide aminé naturel ou non naturel, comme par exemple : glycine, alanine, valine, leucine, isoleucine, sérine, thréonine, cystéine, méthionine, phénylalanine, tyrosine, histidine, tryptophane, lysine, ornithine, acide aspartique, acide glutamique, asparagine, glutamine, arginine, proline, hydroxyproline, sarcosine, acide aminoisobutyrique, les acides déshydroaminés comme par exemple, la déshydroalanine, l'acide déshydro-α-aminobutyrique, d'autres acides aminés non naturels comme la phénylglycine, la 4-nitrophénylalanine, la 3-nitrophénylalanine, la 2-nitrophénylalanine, la 1-naphtylalanine ou la 2-naphtylalanine, facultativement avec des groupes protecteurs, stéréochimiquement homogènes sous la forme L ou la forme D;
E et Q peuvent être reliés l'un avec l'autre par une chaîne alcoyle [(-CH₂-)ₙ avec n = 1, 2];
M représente -CH₂-, -CO-, -SO₂-, -SO-, -CS-;
L représente -CH₂-;
U représente -NH-, -NR-;
X représente un groupe protecteur quelconque, connu dans la chimie peptidique, H ou un acide aminé quelconque naturel ou non naturel, sous forme protégée ou non protégée;
Y représente COOH, CSOH, CH₂OH, COOR" avec R" = un groupe protecteur quelconque de la chimie peptidique, un support, un ligand reporter, un groupe agissant sur la solubilité;
W représente un atome C chiral, lequel peut être configuré de manière homogène, S ou R, et
R et R' représentent H, OH, alcoyle, aralcoyle ou aryle.

9. Composés de formule générale (III) suivant la revendication 7, dans laquelle :
B est choisi indépendamment l'un de l'autre, parmi un groupe consistant en :
H, OH, alcanoyle en C₁-C₄, intercalants d'ADN, restes aromatiques, restes hétérocycliques, bases nucléiques d'origine naturelle comme thymine, uracile, cytosine, adénine, guanine, hypoxanthine, inosine ainsi que les géniteurs halogénés de ces bases nucléiques, ainsi que les dérivés protégés de ces bases nucléiques avec les groupes protecteurs classiques de la chimie nucléotidique ou peptidique;
C représente -CH-, -CR- où C peut avoir la configuration S ou R, de manière homogène;
E représente -NR-, -NH-, -CHR-, -O-;
Q représente (-CR¹R²-)ₘ avec m = 0, 1, 2 et R¹, R² sont choisis indépendamment l'un de l'autre, parmi un groupe consistant en les restes d'acide aminé naturel ou non naturel, comme par exemple : glycine, alanine, valine, leucine, isoleucine, sérine, thréonine, cystéine, méthionine, phénylalanine, tyrosine, histidine, tryptophane, lysine, ornithine, acide aspartique, acide glutamique, asparagine, glutamine, arginine, proline, hydroxyproline, sarcosine, acide aminoisobutyrique, les acides déshydroaminés comme par exemple, la déshydroalanine, l'acide déshydro-a-aminobutyrique, d'autres acides aminés non naturels comme la phénylglycine, facultativement avec des groupes protecteurs, stéréochimiquement homogènes sous la forme L ou la forme D;
E et Q peuvent être reliés l'un avec l'autre par une chaîne alcoyle [(-CH₂-)ₙ avec n = 1, 2];
M représente -CH₂-, -CO-, -CS-;
L représente -CH₂-;
U représente -NH-, -NR-;
X représente un groupe protecteur quelconque, connu dans la chimie peptidique, H ou un acide aminé quelconque naturel ou non naturel, sous forme protégée ou non protégée;
Y représente COOH, CSOH, CH₂OH, COOR" avec R" = un groupe protecteur quelconque de la chimie peptidique, un support, un ligand reporter, un groupe agissant sur la solubilité;
W représente un atome C chiral, lequel peut avoir la configuration, de manière homogène, S ou R, et
R et R' représentent H, OH, alcoyle, aralcoyle ou aryle.

10. Produit pharmaceutique contenant un ou plusieurs composés suivant les revendications 1 à 9.
